# EUROPEAN PATENT SPECIFICATION

(11) **EP 4 165 032 B1**
(45) Date of publication and mention of the grant of the patent: **16.04.2025**
(21) Application number: 21739832.0
(22) Date of filing: 11.06.2021
(51) Int. Cl.: C07D 401/12, C07D 413/12, A61P 29/00, A61K 31/4704

(54) **HETEROALKYL DIHYDROQUINOLINE SULFONAMIDE COMPOUNDS**
HETEROALKYLDIHYDROCHINOLINSULFONAMIDVERBINDUNGEN
COMPOSÉS DE SULFONAMIDE DE DIHYDROQUINOLÉINE D'HÉTÉROALKYLE

(30) Priority: 10.06.2020 US 202063037000 P
(43) Date of publication of application: 19.04.2023
(73) Proprietor: Amgen Inc., Thousand Oaks, California 91320-1799 (US)
(72) Inventor: MILGRAM, Benjamin C., Thousand Oaks, California 91320-1799 (US); RESCOURIO, Gwenaella, Thousand Oaks, California 91320-1799 (US)
(74) Representative: Murgitroyd & Company
(86) International application number: PCT/US2021/036898
(87) International publication number: WO 2021/252822

(56) References cited:
- WO-A1-2014/201206
- WO-A1-2017/106871

## Description

### RELATED APPLICATION

This application claims priority from U.S. Provisional Application No. 63/037,000, having a filing date of June 10, 2020.

### FIELD OF THE INVENTION

The present invention provides heteroalkyl dihydroquinoline sulfonamide compounds that are inhibitors of voltage-gated sodium channels (Nav), in particular Nav 1.7, and are useful for the treatment of diseases treatable by inhibition of sodium channels such as pain disorders. Also provided are pharmaceutical compositions containing compounds of the present invention. The references to methods of treatment in the subsequent paragraphs are to be interpreted as references to the compounds, pharmaceutical compositions and medicaments of the present invention for use in a method for treatment of the human (or animal) body by therapy.

### BACKGROUND OF THE INVENTION

A 2011 report of the institute of medicine estimates that 100 million adults in the US, roughly 30 % of the population, suffer from chronic pain (C & E News, Bethany Halford, "Changing the Channel", published 3-24). Chronic pain by definition involves abnormal electrical spiking of neurons in the pain pathways: peripheral sensory neurons, spinal cord neurons, neurons in the pain matrix of the brain (e.g., somatosensory cortex, insular cortex, anterior cingular cortex), and/or neurons in brainstem. Although firing of these neurons is modulated and governed by many different receptors, enzymes, and growth factors, in most neurons the fast upstroke of the electrical spike is produced by entry of sodium ions through voltage-gated sodium channels (Hille B, Ion Channels of Excitable Membranes. Sinauer Associates, Inc.: Sunderland MA, 3rd Ed. 2001). There are nine different isoforms of voltage-gated sodium channel (Nav 1.1-Nav 1.9), and they have distinct expression patterns in tissues including neurons and cardiac and skeletal muscle (Goldin, A. L, "Resurgence of sodium channel research," Ann Rev Physiol 63:871-894, 2001; Wood, J. N. and, Boorman, J. "Voltage-gated sodium channel blockers; target validation and therapeutic potential" Curr. Top Med. Chem. 5:529-537, 2005).

Nav1.1 and Nav1.2 are highly expressed in the brain (Raymond, C.K., et al., J. Biol. Chem. (2004) 279 (44) :46234-41) and are vital to normal brain function. Some loss of function due to Nav 1.1 mutations in humans, have resulted in epilepsy, presumably as these channels are expressed in inhibitory neurons (Yu, F.H., et al., Nat. Neuroscience (2006), 9 (9) 1142-1149). Nav1.1 is also expressed in the peripheral nervous system and inhibition of Nav1.1 in the periphery may provide relief of pain. Hence, while inhibiting Nav1.1 may provide use for treating pain, it may also be undesirable possibly leading to anxiety and over excitability. Nav1.3 is expressed primarily in the fetal central nervous system, and expression was found to be upregulated after nerve injury in rats (Hains, B.D., et al., J. Neuroscience (2030) 23(26):8881-8892). Nav1.4 is expressed primarily in skeletal muscle. Mutations of the gene and its' product have significant impact on muscle function, including paralysis (Tamaoka A., Internal Medicine (2003), (9):769-770). Nav1.5 is expressed mainly in cardiac myocytes, including atria, ventricles, the sino-atrial node, atrioventricular node and cardiac Purkinje fibers. The rapid upstroke of the cardiac action potential and the rapid impulse conduction through cardiac tissue is due to the opening of the Nav1.5 channel. Mutations of the Nav1.5 channel have resulted in arrhythmic syndromes, including QTc prolongation, Brugada syndrome (BS), sudden unexpected nocturnal death syndrome (SUNDS) and sudden infant death syndrome (SIDS) (Liu, H., et al., Am. J. Pharmacogenomics (2003), 3(3):173-179). Nav1.6 is widely distributed voltage-gated sodium channel expressed throughout the central and peripheral nervous system. Nav1.8 is expressed primarily in sensory ganglia of the peripheral nervous system, such as the dorsal root ganglia. There are no identified Nav1.8 mutations that produce varied pain responses in humans. Nav1.8 differs from most neuronal Nav isotypes in that it is insensitive to inhibition by tetrodotoxin. Nav1.9, similar to Nav1.8, is also a tetrodotoxin insensitive sodium channels expressed primarily in dorsal root ganglia neurons (Dib-Hajj, S.D., et al., Proc. Natl. Acad. Sci. USA (1998), 95(15):8963-8968).

Recent evidence from several independent genetic studies has shown that the tetrodotoxin-sensitive voltage-gated sodium ion channel Nav 1.7 (SCN9A) is required to sense pain. Rare genetic forms of severe chronic pain, Primary Erythromelalgia and Paroxysmal Extreme Pain Disorder, result from mutations that increase the activity of Nav 1.7 (Fertleman C. R., Baker M. D., Parker K. A., Moffatt S., et al., "SCN9A mutations in paroxysmal extreme pain disorder: allelic variants underlie distinct channel defects and phenotypes," Neuron 52:767-774, 2006; Yang Y., Wang Y., Li S, et al., "Mutations in SCN9A, encoding a sodium channel alpha subunit, in patients with primary erythermalgia," J. Med. Genet. 41:171-174, 2004; Drenth J. P. H., te Morsche R. H. M., Guillet G., Taieb A., et al., "SCN9A mutations define primary erythermalgia as a neuropathic disorder of voltage gated sodium channels," J Invest Dermatol 124:1333-1338). Conversely, two separate clinical studies have determined that the root cause of the genetic disorder Congenital Indifference to Pain (CIP) is a loss of function of Nav 1.7 via mutations that truncate the protein and destroy function (Cox J.J., Reimann F, Nicholas A. K., et al. "An SCN9A channelopathy causes congenital inability to experience pain," Nature 444:894-898, 2006; Goldberg Y. P., MacFarlane J., MacDonald M. L., Thompson J., et al. "Loss-of-function mutations in the Nav1.7 gene underlie congenital indifference to pain in multiple human populations," Clin Genet 71:311-319, 2007). The disorder is inherited in Mendelian recessive manner with 100% penetrance. The phenotype associated with CIP is extreme: affected individuals are reported to have experienced painless burns, childbirth, appendicitis, and bone fractures, as well as to have insensitivity to clinical measures of pain such as pinprick or tendon pressure. Yet sensory, motor, autonomic, and other measured functions are normal, with the only reported abnormality being anosmia (inability to smell). These studies indicate that among the many possible targets in the pain pathway, Nav 1.7 governs one or more control points critical for pain perception.

Nonselective sodium channel inhibitors such as lidocaine, mexiletine, and carbamazepine show clinical efficacy in chronic pain, including neuropathic pain, but they are limited in dose and in use, likely due to effects on sodium channels outside the pain pathway. Lidocaine is a local anesthetic doctors use for minor surgery. Dentists use novocaine. However these compounds do not distinguish between the various sodium channel subtypes, making them unsuitable for use as systemic pain killers. "If you give a drug that blocks Nav1.7 but also blocks Nav1.5, the patient will die of heart failure," says Glenn F. King, a professor at Australia's University of Queensland who studies venoms that block ion channels. "It will be a completely painless death, but the patient will die nonetheless." Thus, selectivity for Nav 1.7 is desired, particularly over Nav1.5. Researchers have tailored their efforts to find a molecule that inhibitors or block the activity of only Nav1.7. To compound this problem, the identity, every location, every function and/or the tertiary structures of each subtype of voltage gated sodium channel proteins are not known or completely understood.

Consequently, a number of researchers are attempting to identify small molecule inhibitors of Nav1.7. For example, Chafeev et. al. disclose spiro-oxindole compound for the treatment and/or prevention of sodium channel-mediated diseases, such as pain, in U.S. Patent No. 8,101,647. International Publications WO 2013/134518 and WO 2014/201206 disclose sulfonamide derivatives which are different from the sulfonamide derivatives of the present invention. Thus, there is a need to identify Nav1.7 inhibitors selective over at least Nav1.5 to treat pain. The present invention provides compounds that are selective inhibitors of Nav1.7. over at least Nav1.5.

### SUMMARY OF THE INVENTION

The invention is defined by the claims and any other aspects, configurations or embodiments set forth herein not falling within the scope of the claims are for reference only.

In embodiment 1, the present invention provides a compound of Formula (I), or an enantiomer, diastereoisomer, atropisomer, or a mixture thereof, or a pharmaceutically acceptable salt thereof, 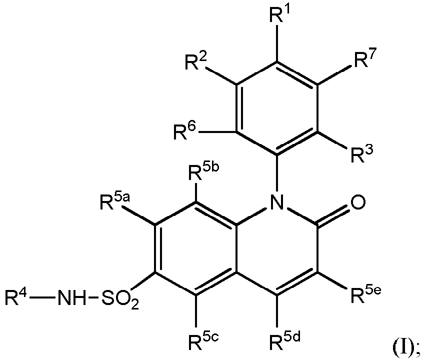 wherein:
R¹ is -C₀₋₄alk-O-C₁₋₈alk, -C₀₋₄alk-S-C₁₋₈alk, or -C₀₋₄alk-NH(C₁₋₈alk); wherein said C₁₋₈alk is substituted by 0, 1, 2, 3, 4, 5, or 6, halo;
R² is H, halo, -CN, C₁₋₆alk, or C₁₋₆haloalk;
R³ is C₁₋₆alk, C₁₋₆haloalk, -O-C₁₋₆alk, -O-cyclopropyl, or -O-cyclobutyl;
R⁴ is a 5- to 6-membered heteroaryl;
each of R⁶ and R⁷ is hydrogen; and
each of R^{5a}; R^{5b};R^{5c}; R^{5d;} and R^{5e} is independently hydrogen or halo.

In sub-embodiment 1a of embodiment 1, the compound of formula (I) has a sub-formula of (Ia), wherein R¹ is -C₀₋₂alk-O-C₁₋₃alk; and R⁴ is isoxazolyl or pyrimidyl.

In a more preferred sub embodiment 1a of embodiment 1, R¹ is -CH₂-O-CF₃ or -O-CF₃; R² is F or Cl; and R⁴ is isoxazolyl.

In a most preferred sub embodiment 1a of embodiment 1, R¹ is -CH₂-O-CF₃, R² is F; and R⁴ is isoxazolyl.

In sub-embodiment 1b of embodiment 1, the compound of formula (I) has a sub-formula of (Ib), wherein R¹ is -C₀₋₂alk-S-C₁₋₂alk; and R⁴ is isoxazolyl or pyrimidyl.

In a more preferred sub embodiment 1b of embodiment 1, R¹ is -S-CF₃ or -S-CH₂CF₃; R² is F, Cl, or methyl; and R⁴ is isoxazolyl or pyrimidyl.

In a most preferred sub embodiment 1b of embodiment 1, each R¹ is -S-CF₃; R² is F; and R⁴ is isoxazolyl.

In embodiment 2, the present invention provides compounds of Formula (I), or an enantiomer, diastereoisomer, atropisomer, or a mixture thereof, or a pharmaceutically acceptable salt thereof, wherein R¹ is -O-C₁₋₈alk, -CH₂-O-C₁₋₈alk, -S-C₁₋₈alk, -CH₂-S-C₁₋₈alk, or -CH(CH₃)-S-C₁₋₈alk, wherein said C₁₋₈alk is substituted by 1, 2, 3, or 4 halo.

In embodiment 3, the present invention provides compounds of Formula (I), or an enantiomer, diastereoisomer, atropisomer, or a mixture thereof, or a pharmaceutically acceptable salt thereof, wherein R¹ is selected from -O-CF₃, -O-CH₂-CF₃, -O-CH₂-CH₂-CF₃, -O-CH(CH₃)-CF₃, -CH₂-O-CF₃, -S-CF₃, or -S-CH₂-CF₃.

In a sub embodiment 3a of embodiment 3, R¹ is -CH₂-O-CF₃ or -S-CF₃.

In a sub embodiment 3b of embodiment 3, R¹ is -CH₂-O-CF₃.

In a sub embodiment 3c of embodiment 3, R¹ is -S-CF₃.

In embodiment 4, the present invention provides compounds of Formula (I), or an enantiomer, diastereoisomer, atropisomer, or a mixture thereof, or a pharmaceutically acceptable salt thereof, wherein R² is H, fluoro, chloro, methyl, CN, CF₃, CHF₂, or CH₂F.

In embodiment 5, the present invention provides compounds of Formula (I), or an enantiomer, diastereoisomer, atropisomer, or a mixture thereof, or a pharmaceutically acceptable salt thereof, wherein R³ is methoxy.

In embodiment 6, the present invention provides compounds of Formula (I), or an enantiomer, diastereoisomer, atropisomer, or a mixture thereof, or a pharmaceutically acceptable salt thereof, R⁴ is isoxazolyl, pyridazinyl, thiazolyl, thiadiazolyl, oxazolyl, or pyrimidinyl.

In embodiment 7, the present invention provides compounds of Formula (I), or an enantiomer, diastereoisomer, atropisomer, or a mixture thereof, or a pharmaceutically acceptable salt thereof, wherein each of R^{5a} , R^{5b};R^{5c}; R^{5d}; and R^{5e} is hydrogen; R^{5a} is F and each of R^{5b};R^{5c}; R^{5d;} and R^{5e} is hydrogen; or R^{5c} is F; and each of R^{5a}; R^{5b};R^{5d}; and R^{5e} is hydrogen.

In embodiment 8, the present invention provides compounds of Formula (I), or a pharmaceutically acceptable salt thereof, wherein the compound is selected from:
(P)-1-(5-chloro-2-methoxy-4-((trifluoromethoxy)methyl)phenyl)-N-(isoxazol-3-yl)-2-oxo-1,2-dihydroquinoline-6-sulfonamide;
(P)-1-(5-chloro-2-methoxy-4-((trifluoromethyl)thio)phenyl)-N-(isoxazol-3-yl)-2-oxo-1,2-dihydroquinoline-6-sulfonamide;
(P)-1-(5-chloro-2-methoxy-4-(trifluoromethoxy)phenyl)-N-(isoxazol-3-yl)-2-oxo-1,2-dihydroquinoline-6-sulfonamide;
(P)-1-(5-fluoro-2-methoxy-4-((trifluoromethoxy)methyl)phenyl)-N-(isoxazol-3-yl)-2-oxo-1,2-dihydroquinoline-6-sulfonamide;
(P)-1-(5-fluoro-2-methoxy-4-((2,2,2-trifluoroethyl)thio)phenyl)-N-(isoxazol-3-yl)-2-oxo-1,2-dihydroquinoline-6-sulfonamide;
(P)- 7-fluoro-1-(5-fluoro-2-methoxy-4-((trifluoromethoxy)methyl)phenyl)-N-(isoxazol-3-yl)-2-oxo-1,2-dihydroquinoline-6-sulfonamide;
(P)-7-fluoro-1-(5-fluoro-2-methoxy-4-((trifluoromethyl)thio)phenyl)-N-(isoxazol-3-yl)-2-oxo-1,2-dihydroquinoline-6-sulfonamide;
(P)-4-fluoro-1-(5-fluoro-2-methoxy-4-((trifluoromethyl)thio)phenyl)-N-(isoxazol-3-yl)-2-oxo-1,2-dihydroquinoline-6-sulfonamide; or
(P)-N-(isoxazol-3-yl)-1-(2-methoxy-5-methyl-4-((trifluoromethyl)thio)phenyl)-2-oxo-1,2-dihydroquinoline-6-sulfonamide.

In embodiment 9, the present invention provides a compound of Formula (I), or a pharmaceutically acceptable salt thereof, wherein the compound is selected from:
(P)-1-(5-chloro-2-methoxy-4-((trifluoromethoxy)methyl)phenyl)-N-(isoxazol-3-yl)-2-oxo-1,2-dihydroquinoline-6-sulfonamide;
(P)-1-(5-chloro-2-methoxy-4-(trifluoromethoxy)phenyl)-N-(isoxazol-3-yl)-2-oxo-1,2-dihydroquinoline-6-sulfonamide;
(P)-1-(5-fluoro-2-methoxy-4-((trifluoromethoxy)methyl)phenyl)-N-(isoxazol-3-yl)-2-oxo-1,2-dihydroquinoline-6-sulfonamide; or
(P)- 7-fluoro-1-(5-fluoro-2-methoxy-4-((trifluoromethoxy)methyl)phenyl)-N-(isoxazol-3-yl)-2-oxo-1,2-dihydroquinoline-6-sulfonamide.

In embodiment 10, the present invention provides a compound of Formula (I), or a pharmaceutically acceptable salt thereof, wherein the compound is selected from:
(P)-1-(5-chloro-2-methoxy-4-((trifluoromethyl)thio)phenyl)-N-(isoxazol-3-yl)-2-oxo-1,2-dihydroquinoline-6-sulfonamide;
(P)-1-(5-fluoro-2-methoxy-4-((2,2,2-trifluoroethyl)thio)phenyl)-N-(isoxazol-3-yl)-2-oxo-1,2-dihydroquinoline-6-sulfonamide;
(P)-7-fluoro-1-(5-fluoro-2-methoxy-4-((trifluoromethyl)thio)phenyl)-N-(isoxazol-3-yl)-2-oxo-1,2-dihydroquinoline-6-sulfonamide;
(P)-4-fluoro-1-(5-fluoro-2-methoxy-4-((trifluoromethyl)thio)phenyl)-N-(isoxazol-3-yl)-2-oxo-1,2-dihydroquinoline-6-sulfonamide; or
(P)-N-(isoxazol-3-yl)-1-(2-methoxy-5-methyl-4-((trifluoromethyl)thio)phenyl)-2-oxo-1,2-dihydroquinoline-6-sulfonamide.

In embodiment 11, the present invention provides a P atropisomer of each individual compound, or pharmaceutically acceptable salts thereof, recited in embodiments 1 to 10, including any one of each sub-embodiment thereof.

In embodiment 12, the present invention provides pharmaceutical compositions comprising a compound, or pharmaceutically acceptable salts thereof, in accordance with any one of embodiments 1, 2, 3, 4, 5, 6, 7, 8, 9, 10, or 11, , including any one of each sub-embodiment thereof, and a pharmaceutically acceptable excipient.

In embodiment 14, the present invention provides methods of treating pain, cough, or itch, the methods comprising administering to a patient in need thereof a therapeutically effective amount of a compound, or an enantiomer, diastereoisomer, atropisomer, or a mixture thereof, or pharmaceutically acceptable salts thereof, in accordance with any one of embodiments 1, 2, 3, 4, 5, 6, 7, 8, 9, 10,or 11, including any one of each sub-embodiment thereof.

In embodiment 15, the present invention provides methods of embodiment 14, wherein the pain is selected from chronic pain, acute pain, neuropathic pain, pain associated with rheumatoid arthritis, pain associated with osteoarthritis, pain associated with cancer, peripheral diabetic neuropathy, and neuropathic low back pain.

In embodiment 16, the present invention provides methods of embodiment 14 wherein the cough is selected from post viral cough, viral cough, or acute viral cough. See Dib-Hajj. et. al., "The NaV1.7 sodium channel: from molecule to man", Nature Reviews Neuroscience (2013), 14, 49-62.

### DETAILED DESCRIPTION OF THE INVENTION

The present invention provides compounds of Formula (I), as defined above, or an enantiomer, diastereoisomer, atropisomer, or a mixture thereof, or pharmaceutically acceptable salts thereof. The present invention also provides pharmaceutical compositions comprising a compound of Formula (I), compound, or an enantiomer, diastereoisomer, atropisomer, or a mixture thereof, or pharmaceutically acceptable salts thereof, and methods of treating diseases and/or conditions, such as pain, using compounds of Formula (I), compound, or an enantiomer, diastereoisomer, atropisomer, or a mixture thereof, or pharmaceutically acceptable salts thereof.

The term "C_{α-β}alk" means an alkyl group comprising a minimum of α and a maximum of β carbon atoms in a branched or linear relationship or any combination of the two, wherein α and β represent integers. A designation of C₀alk indicates a direct bond. Examples of C₁₋₆alk include, but are not limited to the following:

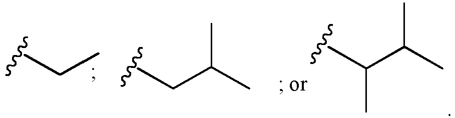

The term "halo" or "halogen" means a halogen atoms selected from F, Cl, Br or I.

The term "C_{α-β}haloalk" means an alk group, as defined herein, in which at least one of the hydrogen atoms has been replaced with a halo atom, as defined herein. Common C_{α-β}haloalk groups are C₁-₃fluoroalk. An example of a common C₁-₃fluoroalk group is -CF₃.

The term "heteroatom" as used herein means an oxygen, nitrogen or sulfur atom.

The term "monocyclic ring" as used herein means a group that features one single ring. A monocyclic ring can be carbocyclic (all of the ring atoms are carbons), or heterocyclic (the rings atoms consist, for example, 1, 2 or 3 heteroatoms, such as N, O, or S, in addition to carbon atoms). Examples of monocyclic compound include, but are not limited to: cyclobutyl, cyclopentyl, or cyclohexyl.

The term "bicyclic ring" as used herein means a group that features two joined rings. A bicyclic ring can be carbocyclic (all of the ring atoms are carbons), or heterocyclic (the rings atoms consist, for example, 1, 2 or 3 heteroatoms, such as N, O, or S, in addition to carbon atoms). The two rings can both be aliphatic (*e.g.* decalin and norbornane), or can be aromatic (*e.g.*naphthalene), or a combination of aliphatic and aromatic (*e.g.* tetralin). Bicyclic rings include (a) spirocyclic compounds, wherein the two rings share only one single atom, the spiro atom, which is usually a quaternary carbon. Examples of spirocyclic compound include, but are not limited to: 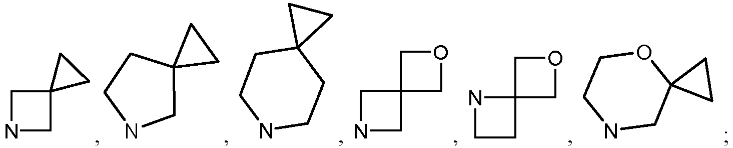
(b) fused bicyclic compounds, wherein two rings share two adjacent atoms. In other words, the rings share one covalent bond, *i.e.* the bridgehead atoms are directly connected (*e.g*. α-thujene and decalin). Examples of fused bicyclic rings include, but are not limited to: 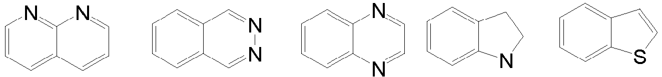 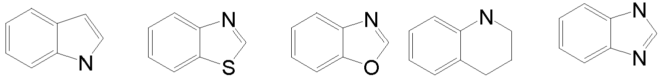 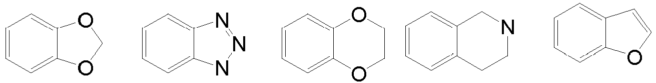 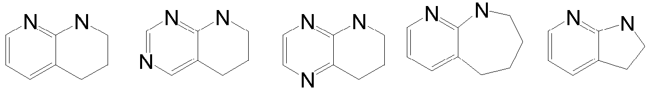 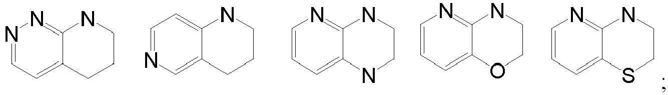 and
(c) bridged bicyclic compounds, wherein the two rings share three or more atoms, separating the two bridgehead atoms by a bridge containing at least one atom. For example, norbornane, also known as bicyclo[2.2.1]heptane, can be thought of as a pair of cyclopentane rings each sharing three of their five carbon atoms. Examples of bridged bicyclic rings include, but are not limited to:

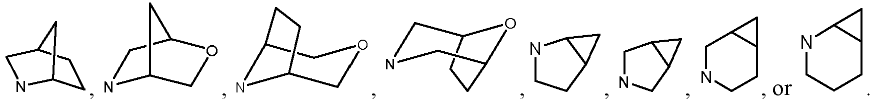

The term "aryl" means a cyclic, aromatic hydrocarbon. Examples of aryl groups include phenyl and naphthyl. Common aryl groups are six to thirteen membered rings.

The term "heteroaryl" means a cyclic, aromatic hydrocarbon in which one or more carbon atoms of an aryl group have been replaced with a heteroatom. If the heteroaryl group contains more than one heteroatom, the heteroatoms may be the same or different. Examples of heteroaryl groups include pyridyl, pyrimidinyl, imidazolyl, thienyl, furyl, pyrazinyl, pyrrolyl, indolyl, triazolyl, pyridazinyl, indazolyl, purinyl, quinolizinyl, isoquinolyl, quinolyl, naphthyridinyl, quinoxalinyl, isothiazolyl and benzo[b]thienyl. Common heteroaryl groups are five to thirteen membered rings that contain from 1 to 4 heteroatoms. Heteroaryl groups that are five and six membered rings that contain 1 to 3 heterotaoms are particularly common.

The term "saturated, partially-saturated or unsaturated" includes substituents saturated with hydrogens, substituents completely unsaturated with hydrogens and substituents partially saturated with hydrogens.

The term "pharmaceutically acceptable salt" means a salt prepared by conventional means, and are well known by those skilled in the art. The "pharmacologically acceptable salts" include basic salts of inorganic and organic acids, including but not limited to hydrochloric acid, hydrobromic acid, sulfuric acid, phosphoric acid, methanesulfonic acid, ethanesulfonic acid, malic acid, acetic acid, oxalic acid, tartaric acid, citric acid, lactic acid, fumaric acid, succinic acid, maleic acid, salicylic acid, benzoic acid, phenylacetic acid, mandelic acid and the like. For additional examples of "pharmacologically acceptable salts," and Berge et al., J. Pharm. Sci. 66:1 (1977).

The term "substituted" means that a hydrogen atom on a molecule or group is replaced with a group or atom. Typical substituents include: halogen, C₁-₈alkyl, hydroxyl, C₁-₈alkoxy, -NR^{x}R^{x}, nitro, cyano, halo or perhaloC₁-₈alkyl, C₂-₈alkenyl, C₂-₈alkynyl, -SR^{x}, - S(=O)₂R^{x}, -C(=O)OR^{x}, -C(=O)R^{x}, wherein each R^{x} is independently hydrogen or C₁-C₈ alkyl. It is noted that when the substituent is -NR^{x}R^{x}, the R^{x} groups may be joined together with the nitrogen atom to form a ring.

A group or atom that replaces a hydrogen atom is also called a substituent.

Any particular molecule or group can have one or more substituent depending on the number of hydrogen atoms that can be replaced.

The term "unsubstituted" means a hydrogen atom on a molecule or group. The term "substituted" means that a hydrogen atom on a molecule or group is replaced with a group or atom. Typical substituents include: halogen, C₁-₈alkyl, hydroxyl, C₁-₈alkoxy, -NR^{x}R^{x}, nitro, cyano, halo or perhaloC₁-₈alkyl, C₂-₈alkenyl, C₂-₈alkynyl, -SR^{x}, -S(=O)₂R^{x}, -C(=O)OR^{x}, -C(=O)R^{x}, wherein each R^{x} is independently hydrogen or C₁-C₈ alkyl. It is noted that when the substituent is -NR^{x}R^{x}, the R^{x} groups may be joined together with the nitrogen atom to form a ring.

The symbol "-" represents a covalent bond and can also be used in a radical group to indicate the point of attachment to another group. In chemical structures, the symbol is commonly used to represent a methyl group in a molecule.

The term "leaving group" generally refers to groups readily displaceable by a nucleophile, such as an amine, a thiol or an alcohol nucleophile, or by metallic agent such as boronic acids or boronates under transition metal catalyzed coupling conditions. Such leaving groups are well known in the art. Examples of such leaving groups include, but are not limited to, N-hydroxysuccinimide, N-hydroxybenzotriazole, halides, triflates, tosylates and the like. Preferred leaving groups are indicated herein where appropriate.

The term "protecting group" generally refers to groups well known in the art which are used to prevent selected reactive groups, such as carboxy, amino, hydroxy, mercapto and the like, from undergoing undesired reactions, such as nucleophilic, electrophilic, oxidation, reduction and the like. Preferred protecting groups are indicated herein where appropriate. Examples of amino protecting groups include, but are not limited to, aralkyl, substituted aralkyl, cycloalkenylalkyl and substituted cycloalkenyl alkyl, allyl, substituted allyl, acyl, alkoxycarbonyl, aralkoxycarbonyl, silyl and the like. Examples of aralkyl include, but are not limited to, benzyl, ortho-methylbenzyl, trityl and benzhydryl, which can be optionally substituted with halogen, alkyl, alkoxy, hydroxy, nitro, acylamino, acyl and the like, and salts, such as phosphonium and ammonium salts. Examples of aryl groups include phenyl, naphthyl, indanyl, anthracenyl, 9-(9-phenylfluorenyl), phenanthrenyl, durenyl and the like. Examples of cycloalkenylalkyl or substituted cycloalkylenylalkyl radicals, preferably have 6-10 carbon atoms, include, but are not limited to, cyclohexenyl methyl and the like. Suitable acyl, alkoxycarbonyl and aralkoxycarbonyl groups include benzyloxycarbonyl, t-butoxycarbonyl, iso-butoxycarbonyl, benzoyl, substituted benzoyl, butyryl, acetyl, trifluoroacetyl, trichloro acetyl, phthaloyl and the like. A mixture of protecting groups can be used to protect the same amino group, such as a primary amino group can be protected by both an aralkyl group and an aralkoxycarbonyl group. Amino protecting groups can also form a heterocyclic ring with the nitrogen to which they are attached, for example, 1,2-bis(methylene)benzene, phthalimidyl, succinimidyl, maleimidyl and the like and where these heterocyclic groups can further include adjoining aryl and cycloalkyl rings. In addition, the heterocyclic groups can be mono-, di- or tri-substituted, such as nitrophthalimidyl. Amino groups may also be protected against undesired reactions, such as oxidation, through the formation of an addition salt, such as hydrochloride, toluenesulfonic acid, trifluoroacetic acid and the like. Many of the amino protecting groups are also suitable for protecting carboxy, hydroxy and mercapto groups. For example, aralkyl groups. Alkyl groups are also suitable groups for protecting hydroxy and mercapto groups, such as tert-butyl.

Protecting groups are removed under conditions which will not affect the remaining portion of the molecule. These methods are well known in the art and include acid hydrolysis, hydrogenolysis and the like. A preferred method involves removal of a protecting group, such as removal of a benzyloxycarbonyl group by hydrogenolysis utilizing palladium on carbon in a suitable solvent system such as an alcohol, acetic acid, and the like or mixtures thereof. A *tert*-butoxycarbonyl protecting group can be removed utilizing an inorganic or organic acid, such as HCl or trifluoroacetic acid, in a suitable solvent system, such as dioxane or methylene chloride. The resulting amino salt can readily be neutralized to yield the free amine. Carboxy protecting group, such as methyl, ethyl, benzyl, *tert*-butyl, 4-methoxyphenylmethyl and the like, can be removed under hydrolysis and hydrogenolysis conditions well known to those skilled in the art.

Prodrugs of the compounds of this invention are also contemplated by this invention. A prodrug is an active or inactive compound that is modified chemically through in vivo physiological action, such as hydrolysis, metabolism and the like, into a compound of this invention following administration of the prodrug to a patient. The suitability and techniques involved in making and using prodrugs are well known by those skilled in the art. For a general discussion of prodrugs involving esters see Svensson and Tunek Drug Metabolism Reviews 165 (1988) and Bundgaard Design of Prodrugs, Elsevier (1985). Examples of a masked carboxylate anion include a variety of esters, such as alkyl (for example, methyl, ethyl), cycloalkyl (for example, cyclohexyl), aralkyl (for example, benzyl, p-methoxybenzyl), and alkylcarbonyloxyalkyl (for example, pivaloyloxymethyl). Amines have been masked as arylcarbonyloxymethyl substituted derivatives which are cleaved by esterases in vivo releasing the free drug and formaldehyde (Bundgaard J. Med. Chem. 2503 (1989)). Also, drugs containing an acidic NH group, such as imidazole, imide, indole and the like, have been masked with N-acyloxymethyl groups (Bundgaard Design of Prodrugs, Elsevier (1985)). Hydroxy groups have been masked as esters and ethers. EP 039,051 (Sloan and Little, 4/11/81) discloses Mannich-base hydroxamic acid prodrugs, their preparation and use.

The term "therapeutically effective amount" means an amount of a compound that ameliorates, attenuates or eliminates one or more symptom of a particular disease or condition, or prevents or delays the onset of one of more symptom of a particular disease or condition.

The term "patient" means animals, such as dogs, cats, cows, horses, sheep and humans. Particular patients are mammals. The term patient includes males and females.

The term "pharmaceutically acceptable" means that the referenced substance, such as a compound of Formula I, or a salt of a compound of Formula I, or a formulation containing a compound of Formula I, or a particular excipient, are suitable for administration to a patient.

The terms "treating", "treat" or "treatment" and the like include preventative (e.g., prophylactic) and palliative treatment.

The term "excipient" means any pharmaceutically acceptable additive, carrier, diluent, adjuvant, or other ingredient, other than the active pharmaceutical ingredient (API), which is typically included for formulation and/or administration to a patient.

The compounds of the present invention are administered to a patient in a therapeutically effective amount. The compounds can be administered alone or as part of a pharmaceutically acceptable composition or formulation. In addition, the compounds or compositions can be administered all at once, as for example, by a bolus injection, multiple times, such as by a series of tablets, or delivered substantially uniformly over a period of time, as for example, using transdermal delivery. It is also noted that the dose of the compound can be varied over time.

In addition, the compounds of the present invention can be administered alone, in combination with other compounds of the present invention, or with other pharmaceutically active compounds. The other pharmaceutically active compounds can be intended to treat the same disease or condition as the compounds of the present invention or a different disease or condition. If the patient is to receive or is receiving multiple pharmaceutically active compounds, the compounds can be administered simultaneously, or sequentially. For example, in the case of tablets, the active compounds may be found in one tablet or in separate tablets, which can be administered at once or sequentially in any order. In addition, it should be recognized that the compositions may be different forms. For example, one or more compound may be delivered by a tablet, while another is administered by injection or orally as syrup. All combinations, delivery methods and administration sequences are contemplated.

The compounds of the present invention may be used in the manufacture of a medicament for the treatment of a disease and/or condition mediated by Nav 1.7, such as pain, chronic cough or itch.

Pain is typically divided into primary types: chronic and acute pain based on the duration of the pain. Typically, chronic pain lasts for longer than 3 months. Examples of chronic pain include pain associated with rheumatoid arthritis, osteoarthritis, lumbosacral radiculopathy or cancer. Chronic pain also includes idiopathic pain, which is pain that has no identified cause. An example of idiopathic pain is fibromyalgia.

Another type of pain is nociceptive pain. Nociceptive pain is caused by stimulation of peripheral nerve fibers that respond to highly noxious events such as thermal, mechanical or chemical stimuli.

Still another type of pain is neuropathic pain. Neuropathic pain is pain that is caused by damage or disease affecting a part of the nervous system. Phantom limb pain is a type of neuropathic pain. In phantom limb pain, the body detects pain from a part of a body that no longer exists. For example, a person who has had a leg amputated may feel leg pain even though the leg no longer exists.

In one embodiment of the methods of treatment provided by the present invention using the compounds of Formula (I), or pharmaceutically acceptable salts thereof, the disease is chronic pain. In another aspect, the chronic pain is associated with, but are not limited to, post-herpetic neuralgia (shingles), rheumatoid arthritis, osteoarthritis, diabetic neuropathy, complex regional pain syndrome (CRPS), cancer or chemotherapy-induced pain, chronic back pain, phantom limb pain, trigeminal neuralgia, HIV-induced neuropathy, cluster headache disorders, and migraine, primary erythromelalgia, and paroxysmal extreme pain disorder. Other indications for Nav 1.7 inhibitors include, but are not limited to, depression (Morinville et al., J Comp Neurol., 504:680-689 (2007)), bipolar and other CNS disorders (Ettinger and Argoff, Neurotherapeutics, 4:75-83 (2007)), epilepsy: ibid., and Gonzalez, Termin, Wilson, Methods and Principles in Medicinal Chemistry, 29:168-192 (2006)), multiple sclerosis (Waxman, Nature Neurosci. 7 :932-941 (2006)), Parkinson's (Do and Bean, Neuron 39 :109-120 (2003); Puopolo et al., J. Neurosci. 27 :645-656 (2007)), restless legs syndrome, ataxia, tremor, muscle weakness, dystonia, tetanus (Hamann M., et. al., Exp. Neurol. 184(2):830-838, 2003), anxiety, depression: McKinney B. C, et. al., Genes Brain Behav. 7(6):629-638, 2008), learning and memory, cognition (Woodruff-Pak D. S., et. al., Behav. Neurosci. 120(2):229-240, 2006), cardiac arrhythmia and fibrillation, contractility, congestive heart failure, sick sinus syndrome (Haufe V., et. al., .J Mol. Cell Cardiol. 42(3):469-477, 2007), schizophrenia, neuroprotection after stroke, drug and alcohol abuse (Johannessen L. C., CNS Drugs 22(1)27-47, 2008), Alzheimer's (Kim D. Y., et. al., Nat. Cell. Biol. 9(7):755-764, 2007), and cancer (Gillet L., et. al., J Biol Chem 2009, Jan 28 (epub)).

Another aspect of the invention relates to a method of treating acute and/or chronic inflammatory and neuropathic pain, dental pain, general headache, migraine, cluster headache, mixed-vascular and non-vascular syndromes, tension headache, general inflammation, arthritis, rheumatic diseases, rheumatoid arthritis, osteoarthritis, inflammatory bowel disorders, inflammatory eye disorders, inflammatory or unstable bladder disorders, psoriasis, skin complaints with inflammatory components, chronic inflammatory conditions, inflammatory pain and associated hyperalgesia and allodynia, neuropathic pain and associated hyperalgesia and allodynia, diabetic neuropathy pain, causalgia, sympathetically maintained pain, deafferentation pain syndromes, asthma, epithelial tissue damage or dysfunction, herpes simplex, disturbances of visceral motility at respiratory, genitourinary, gastrointestinal or vascular regions, wounds, burns, allergic skin reactions, pruritus, vitiligo, general gastrointestinal disorders, gastric ulceration, duodenal ulcers, diarrhea, gastric lesions induced by necrotising agents, hair growth, vasomotor or allergic rhinitis, bronchial disorders or bladder disorders, comprising the step of administering a compound according to the present invention. A preferred type of pain to be treated is chronic neuropathic pain. Another preferred type of pain to be treated is chronic inflammatory pain.

In another aspect of the invention, the compounds of the present invention can be used in combination with other compounds that are used to treat pain. Examples of such other compounds include, but are not limited to aspirin, celecoxib, hydrocodone, oxycodone, codeine, fentanyl, ibuprofen, ketoprofen, naproxen, acetaminophen, gabapentin and pregabalin. Examples of classes of medicines that contain compounds that can be used in combination with the compounds of the present invention include non-steroidal anti-inflammatory compounds (NSAIDS), steroidal compounds, cyclooxygenase inhibitors and opioid analgesics.

The compounds of the present invention may also be used to treat diabetes, obesity and/or to facilitate weight loss.

The compounds of the present invention may be used in combination with other pharmaceutically active compounds. It is noted that the term "pharmaceutically active compounds" can include biologics, such as proteins, antibodies and peptides.

Since one aspect of the present invention contemplates the treatment of the disease/conditions with a combination of pharmaceutically active compounds that may be administered separately, the invention further relates to combining separate pharmaceutical compositions in kit form. The kit comprises two separate pharmaceutical compositions: a compound of the present invention, and a second pharmaceutical compound. The kit comprises a container for containing the separate compositions such as a divided bottle or a divided foil packet. Additional examples of containers include syringes, boxes and bags. Typically, the kit comprises directions for the use of the separate components. The kit form is particularly advantageous when the separate components are preferably administered in different dosage forms (e.g., oral and parenteral), are administered at different dosage intervals, or when titration of the individual components of the combination is desired by the prescribing physician or veterinarian.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed by said opening.

It may be desirable to provide a memory aid on the kit, e.g., in the form of numbers next to the tablets or capsules whereby the numbers correspond with the days of the regimen which the tablets or capsules so specified should be ingested. Another example of such a memory aid is a calendar printed on the card, e.g., as follows "First Week, Monday, Tuesday, ... etc... Second Week, Monday, Tuesday, ... " etc. Other variations of memory aids will be readily apparent. A "daily dose" can be a single tablet or capsule or several pills or capsules to be taken on a given day. Also, a daily dose of a compound of the present invention can consist of one tablet or capsule, while a daily dose of the second compound can consist of several tablets or capsules and vice versa. The memory aid should reflect this and aid in correct administration of the active agents.

In another specific embodiment of the invention, a dispenser designed to dispense the daily doses one at a time in the order of their intended use is provided. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

The compounds of the present invention and other pharmaceutically active compounds, if desired, can be administered to a patient either orally, rectally, parenterally, (for example, intravenously, intramuscularly, or subcutaneously) intracisternally, intravaginally, intraperitoneally, intravesically, locally (for example, powders, ointments or drops), or as a buccal or nasal spray. All methods that are used by those skilled in the art to administer a pharmaceutically active agent are contemplated.

Compositions suitable for parenteral injection may comprise physiologically acceptable sterile aqueous or nonaqueous solutions, dispersions, suspensions, or emulsions, and sterile powders for reconstitution into sterile injectable solutions or dispersions. Examples of suitable aqueous and nonaqueous carriers, diluents, solvents, or vehicles include water, ethanol, polyols (propylene glycol, polyethylene glycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants.

These compositions may also contain adjuvants such as preserving, wetting, emulsifying, and dispersing agents. Microorganism contamination can be prevented by adding various antibacterial and antifungal agents, for example, parabens, chlorobutanol, phenol, sorbic acid, and the like. It may also be desirable to include isotonic agents, for example, sugars, sodium chloride, and the like. Prolonged absorption of injectable pharmaceutical compositions can be brought about by the use of agents delaying absorption, for example, aluminum monostearate and gelatin.

Solid dosage forms for oral administration include capsules, tablets, powders, and granules. In such solid dosage forms, the active compound is admixed with at least one inert customary excipient (or carrier) such as sodium citrate or dicalcium phosphate or (a) fillers or extenders, as for example, starches, lactose, sucrose, mannitol, and silicic acid; (b) binders, as for example, carboxymethylcellulose, alginates, gelatin, polyvinylpyrrolidone, sucrose, and acacia; (c) humectants, as for example, glycerol; (d) disintegrating agents, as for example, agar-agar, calcium carbonate, potato or tapioca starch, alginic acid, certain complex silicates, and sodium carbonate; (a) solution retarders, as for example, paraffin; (f) absorption accelerators, as for example, quaternary ammonium compounds; wetting agents, as for example, cetyl alcohol and glycerol monostearate; (h) adsorbents, as for example, kaolin and bentonite; and (i) lubricants, as for example, talc, calcium stearate, magnesium stearate, solid polyethylene glycols, sodium lauryl sulfate, or mixtures thereof. In the case of capsules, and tablets, the dosage forms may also comprise buffering agents.

Solid compositions of a similar type may also be used as fillers in soft and hard filled gelatin capsules using such excipients as lactose or milk sugar, as well as high molecular weight polyethylene glycols, and the like.

Solid dosage forms such as tablets, dragées, capsules, pills, and granules can be prepared with coatings and shells, such as enteric coatings and others well known in the art. They may also contain opacifying agents, and can also be of such composition that they release the active compound or compounds in a certain part of the intestinal tract in a delayed manner. Examples of embedding compositions that can be used are polymeric substances and waxes. The active compounds can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to the active compounds, the liquid dosage form may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular, cottonseed oil, groundnut oil, corn germ oil, olive oil, castor oil, and sesame seed oil, glycerol, tetrahydrofurfuryl alcohol, polyethylene glycols and fatty acid esters of sorbitan, or mixtures of these substances, and the like.

Besides such inert diluents, the composition can also include adjuvants, such as wetting agents, emulsifying and suspending agents, sweetening, flavoring, and perfuming agents. Suspensions, in addition to the active compound, may contain suspending agents, as for example, ethoxylated isostearyl alcohols, polyoxyethylene sorbitol and sorbitan esters, microcrystalline cellulose, aluminum metahydroxide, bentonite, agar-agar, and tragacanth, or mixtures of these substances, and the like.

Compositions for rectal administration are preferable suppositories, which can be prepared by mixing the compounds of the present invention with suitable non-irritating excipients or carriers such as cocoa butter, polyethylene glycol or a suppository wax, which are solid at ordinary room temperature, but liquid at body temperature, and therefore, melt in the rectum or vaginal cavity and release the active component.

Dosage forms for topical administration of a compound of the present invention include ointments, powders, sprays and inhalants. The active compound or fit compounds are admixed under sterile condition with a physiologically acceptable carrier, and any preservatives, buffers, or propellants that may be required. Ophthalmic formulations, eye ointments, powders, and solutions are also contemplated as being within the scope of this invention.

The compounds of the present invention can be administered to a patient at therapeutically effective dosage levels. The specific dosage and dosage range that can be used depends on a number of factors, including the requirements of the patient, the severity of the condition or disease being treated, and the pharmacological activity of the compound being administered.

The compounds of the present invention can be administered as pharmaceutically acceptable salts, co-crystals, esters, amides or prodrugs. The term "salts" refers to inorganic and organic salts of compounds of the present invention. The salts can be prepared in situ during the final isolation and purification of a compound, or by separately reacting a purified compound in its free base or acid form with a suitable organic or inorganic base or acid and isolating the salt thus formed. Representative salts include the hydrobromide, hydrochloride, sulfate, bisulfate, nitrate, acetate, oxalate, palmitiate, stearate, laurate, borate, benzoate, lactate, phosphate, tosylate, citrate, maleate, fumarate, succinate, tartrate, naphthylate, mesylate, glucoheptonate, lactobionate, and laurylsulphonate salts, and the like. The salts may include cations based on the alkali and alkaline earth metals, such as sodium, lithium, potassium, calcium, magnesium, and the like, as well as non-toxic ammonium, quaternary ammonium, and amine cations including, but not limited to, ammonium, tetramethylammonium, tetraethylammonium, methylamine, dimethylamine, trimethylamine, triethylamine, ethylamine, and the like. See, for example, S. M. Berge, et al., "Pharmaceutical Salts," J Pharm Sci, 66: 1-19 (1977).

Examples of pharmaceutically acceptable esters of the compounds of the present invention include C₁-C₈ alkyl esters. Acceptable esters also include C₅-C₇ cycloalkyl esters, as well as arylalkyl esters such as benzyl. C₁-C₄ alkyl esters are commonly used. Esters of compounds of the present invention may be prepared according to methods that are well known in the art.

Examples of pharmaceutically acceptable amides of the compounds of the present invention include amides derived from ammonia, primary C₁-C₈ alkyl amines, and secondary C₁-C₈ dialkyl amines. In the case of secondary amines, the amine may also be in the form of a 5 or 6 membered heterocycloalkyl group containing at least one nitrogen atom. Amides derived from ammonia, C₁-C₃ primary alkyl amines and C₁-C₂ dialkyl secondary amines are commonly used. Amides of the compounds of the present invention may be prepared according to methods well known to those skilled in the art.

The term "prodrug" means compounds that are transformed in vivo to yield a compound of the present invention. The transformation may occur by various mechanisms, such as through hydrolysis in blood. A discussion of the use of prodrugs is provided by T. Higuchi and W. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

To illustrate, if the compound of the invention contains a carboxylic acid functional group, a prodrug can comprise an ester formed by the replacement of the hydrogen atom of the acid group with a group such as (C₁-C₈ alkyl, (C₂-C1₂)alkanoyloxymethyl, 1-(alkanoyloxy)ethyl having from 4 to 9 carbon atoms, 1-methyl-1-(alkanoyloxy)ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)aminomethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as β-dimethylaminoethyl), carbamoyl-(C₁-C₂)alkyl, N,N-di(C₁-C₂)alkylcarbamoyl-(C₁-C₂)alkyl and piperidino-, pyrrolidino- or morpholino(C₂-₃)alkyl.

Similarly, if a compound of the present invention comprises an alcohol functional group, a prodrug can be formed by the replacement of the hydrogen atom of the alcohol group with a group such as (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, α-amino(C₁-C₄)alkanoyl, arylacyl and α-aminoacyl, or α-aminoacyl-α-aminoacyl, where each α-aminoacyl group is independently selected from the naturally occurring L-amino acids, -P(O)(OH)₂, -P(O)(O(C₁-C₆)alkyl)₂ or glycosyl (the radical resulting from the removal of a hydroxyl group of the hemiacetal form of a carbohydrate).

In addition, if a compound of the present invention comprises a sulfonamide moiety, a prodrug can be formed by replacement of the sulfonamide N(H) with a group such as -CH₂P(O)(O(C₁-C₆)alkyl)₂ or -CH₂OC(O)(C₁-C₆)alkyl.

The compounds of the present invention also include tautomeric forms of prodrugs.

The compounds of the present invention may contain asymmetric or chiral centers, and therefore, exist in different stereoisomeric forms. It is contemplated that all stereoisomeric forms of the compounds as well as mixtures thereof, including racemic mixtures, form part of the present invention. In addition, the present invention contemplates all geometric and positional isomers. For example, if the compound contains a double bond, both the cis and trans forms (designated as S and E, respectively), as well as mixtures, are contemplated.

Mixture of stereoisomers, such as diastereomeric mixtures, can be separated into their individual stereochemical components on the basis of their physical chemical differences by known methods such as chromatography and/or fractional crystallization. Enantiomers can also be separated by converting the enantiomeric mixture into a diasteromeric mixture by reaction with an appropriate optically active compound (e.g., an alcohol), separating the diastereomers and converting (e.g., hydrolyzing) the individual diastereomers to the corresponding pure enantiomers.

The compounds of general formula (I) may also exist in the form of atropisomers. Atropisomers are compounds with identical structural formulae, but which have a particular spatial configuration resulting from a restricted rotation around a single bond, due to a major steric hindrance on either side of this single bond. Atropisomerism is independent of the presence of stereogenic elements, such as an asymmetric carbon. The terms "P atropisomer" or "M atropisomer" are used herein in order to be able to clearly name two atropisomers of the same pair. For example, the following compound of Intermediate B1, Step 1, having the structure below can be separated into the pair of atropisomers P and M via a chiral column:

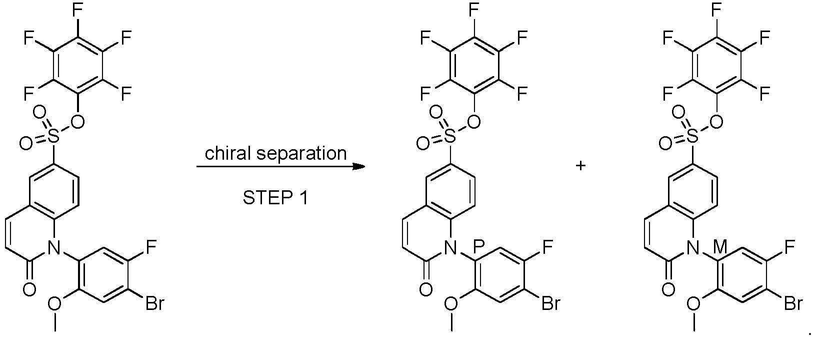

The compounds of the present invention may exist in unsolvated as well as solvated forms with pharmaceutically acceptable solvents such as water (hydrate), ethanol, and the like. The present invention contemplates and encompasses both the solvated and unsolvated forms.

It is also possible that compounds of the present invention may exist in different tautomeric forms. All tautomers of compounds of the present invention are contemplated. For example, all of the tautomeric forms of the tetrazole moiety are included in this invention. Also, for example, all keto-enol or imine-enamine forms of the compounds are included in this invention. Other examples of tautomerism are as follows:

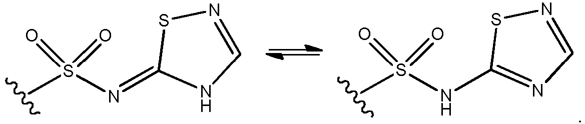

Those skilled in the art will recognize that the compound names and structures contained herein may be based on a particular tautomer of a compound. While the name or structure for only a particular tautomer may be used, it is intended that all tautomers are encompassed by the present invention, unless stated otherwise.

It is also intended that the present invention encompass compounds that are synthesized in vitro using laboratory techniques, such as those well known to synthetic chemists; or synthesized using in vivo techniques, such as through metabolism, fermentation, digestion, and the like. It is also contemplated that the compounds of the present invention may be synthesized using a combination of in vitro and in vivo techniques.

The present invention also includes isotopically-labelled compounds, which are identical to those recited herein, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine and chlorine, such as ²H, ³H, ¹³C, ¹⁴C, ¹⁵N, ¹⁶O, ¹⁷O, ³¹P, ³²P, ³⁵S, ¹⁸F, and ³⁶Cl. In another aspect, the compounds of the present invention contain one or more deuterium atoms (2H) in place of one or more hydrogen atoms.

Compounds of the present invention that contain the aforementioned isotopes and/or other isotopes of other atoms are within the scope of this invention. Certain isotopically-labelled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detection. Further, substitution with heavier isotopes such as deuterium, i.e., ²H, can afford certain therapeutic advantages resulting from greater metabolic stability, for example increased in vivo half-life or reduced dosage requirements and, hence, may be preferred in some circumstances. Isotopically labelled compounds of this invention can generally be prepared by substituting a readily available isotopically labelled reagent for a non-isotopically labelled reagent.

The compounds of the present invention may exist in various solid states including crystalline states and as an amorphous state. The different crystalline states, also called polymorphs, and the amorphous states of the present compounds are contemplated as part of this invention.

All patents and other publications recited herein are hereby incorporated by reference in their entirety.

The examples presented below illustrate specific embodiments of the present invention. These examples are meant to be representative and are not intended to limit the scope of the claims in any manner.

It is noted that when a percent (%) is used with regard to a liquid, it is a percent by volume with respect to the solution. When used with a solid, it is the percent with regard to the solid composition. Materials obtained from commercial suppliers were typically used without further purification. Reactions involving air or moisture sensitive reagents were typically performed under a nitrogen or argon atmosphere. Purity was measured using high performance liquid chromatography (HPLC) system with UV detection at 254 nm and 215 nm (System A: HALO C8, 3.0 x 50 mm, 2.7 µm, 5 to 95% CH₃CN in H₂O with 0.1% TFA for 2.0 min at 2.0 mL/min) (Agilent Technologies, Santa Clara, CA). Silica gel chromatography was generally performed with prepacked silica gel cartridges (Biotage, Uppsala, Sweden or Teledyne-Isco, Lincoln, NE). ¹H NMR spectra were recorded on a Bruker AV-400 (400 MHz) spectrometer (Bruker Corporation, Madison, WI) or a Varian (Agilent Technologies, Santa Clara, CA) 400 MHz spectrometer at ambient temperature. All observed protons are reported as parts per million (ppm) downfield from tetramethylsilane (TMS) or other internal reference in the appropriate solvent indicated. Data are reported as follows: chemical shift, multiplicity (s = singlet, d = doublet, t = triplet, q = quartet, br = broad, m = multiplet), coupling constants, and number of protons. Low-resolution mass spectral (MS) data were determined on an Agilent 1100 Series (Agilent Technologies, Santa Clara, CA) LC/MS with UV detection at 254 nm and 215 nm and a low resonance electrospray mode (ESI).

The following abbreviations may be used herein:
- 2-PrOH: Isopropanol
- AcOH: acetic acid
- AgOTf: silver(I) trifluoromethanesulfonate
- AIBN: Azobisisobutyronitrile
- aq.: Aqueous
- Bu: Butyl
- ca.: Circa
- Cm: centimeter(s)
- CPhos: 2-dicyclohexylphosphino-2',6'-dimethylamino-1,1'-biphenyl
- DAST: diethylaminosulfur trifluoride
- Dba: Dibenzylideneacetone
- DCM: Dichloromethane
- Deoxy-Fluor: bis(2-methoxyethyl)aminosulfur trifluoride
- DIPEA: N,N-diisopropylethylamine
- DMF: N,N-dimethylformamide
- DMSO: Dimethylsulfoxide
- ESI or ES: electrospray ionization
- Et: Ethyl
- Et₂O: diethyl ether
- EtOAc: ethyl acetate
- EtOH: Ethanol
- G: gram(s)
- H: hour(s)
- HOAc: acetic acid
- HPLC: high pressure liquid chromatography
- IPA: 2-propanol
- Kg: kilogram(s)
- L: liter(s)
- LCMS: liquid chromatography mass spectroscopy
- LHMDS: lithium hexamethyldisilazide
- M: Molar
- m/z: mass divided by charge
- Me: Methyl
- MeOH: Methanol
- Mg: milligram(s)
- MHz: Megahertz
- Min: minute(s)
- mL or ml: milliliter(s)
- Mmol: millimole(s)
- Mol: mole(s)
- MTBE: methyl tert-butyl ether
- N: Normal
- NaOMe: sodium methoxide
- n-Bu: n-butyl
- NEt₃: Triethylamine
- NMR: nuclear magnetic resonance
- OAc: Acetate
- OTf: Trifluoromethanesulfonate
- PFP-OH: Perfluorophenol
- Ph: Phenyl
- PhMe: Toluene
- PMB: 4-methoxylbenzyl
- Ppm: parts per million
- Pr: Propyl
- RT or rt: room temperature
- sat.: Saturated
- SFC: supercritical fluid chromatography
- TBAF: tetra-n-butylammonium fluoride
- TFA: trifluoroacetic acid
- THF: Tetrahydrofuran
- Ti(O*i*Pr)₄: titanium(IV) isopropoxide
- TLC: thin-layer chromatography
- TMS-CF₃: (trifluoromethyl)trimethylsilane
- wt%: percentage by weight
- XantPhos: 4,5-bis(diphenylphosphino)-9,9-dimethylxanthene
- XtalFluor-M: difluoro(morpholino)sulfonium tetrafluoroborate

The following compounds presented herein, as examples of the present invention, and intermediates thereof as building blocks to prepare compounds provided by the invention, may be made by the various methods and synthetic strategies taught herein below. These compounds, and others provided by the invention, may also be prepared using methods described in International Publication No. WO2014/201206, filed June 12, 2014, and WO2017/106871, filed December 19, 2016, which specifications are incorporated herein by reference in their entirety.

### INTERMEDIATE A1: (P)-1-(4-BROMO-5-FLUORO-2-METHOXYPHENYL)-N-(ISOXAZOL-3-YL)-N-(4-METHOXYBENZYL)-2-OXO-1,2-DIHYDROQUINOLINE-6-SULFONAMIDE

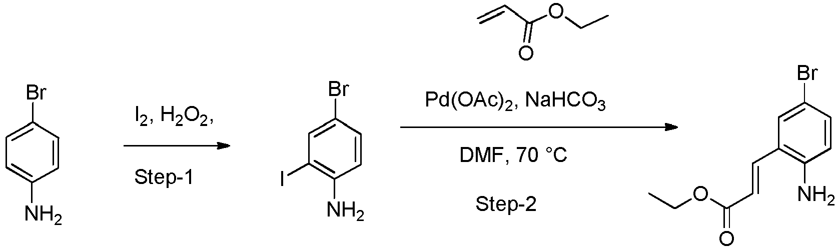

### STEP-1: 4-BROMO-2-IODOANILINE

To a solution of 4-bromo-aniline (500 g, 2.90 mol, 2.0 equiv, Saibain Chem) in cyclohexane (2.5 L) was added iodine (368 g, 1.45 mol, 1.0 equiv, Qualigens) and the mixture was heated at 50 °C. After 30 min, the reaction mixture became homogenous. 30% aqueous hydrogen peroxide solution (250 mL, Spectrochem) was added to the reaction mixture. The reaction was heated for 4 h at 50 °C. The reaction was cooled to room temperature, diluted with ethyl acetate (5.0 L) and washed with aqueous sodium-sulphite (2.5 Kg in 4.0 L) solution. The organic layer was washed with water (3.0 L) and brine (3.0 L) dried over magnesium sulfate, filtered and concentrated under reduced pressure to obtain the crude material which was purified by column chromatography (silica gel; mesh size 60-120, elution 0-20% ethyl acetate and hexanes) to get 4-bromo-2-iodoaniline (650 g, 75.0 %), as off white solid. TLC solvent system: 100 % hexanes. Product's R_{f} : 0.6. MS (ESI, positive ion) m/z: 297.0 (M+1). ¹H NMR (400 MHz, CDCl₃) δ 7.72 (d, *J =* 2.5 Hz, 1H), 7.23 (dd, *J =* 8.4, 2.1 Hz, 1H), 6.62 (d, *J =* 8.3 Hz, 1H), 4.09 (s, 2H).

### STEP-2: ETHYL (E)-3-(2-AMINO-5-BROMOPHENYL)ACRYLATE

To a solution of 4-bromo-2-iodoaniline (750 g, 2.51 mol, 1.0 equiv) in DMF (5.0 L) was added ethyl acrylate (277 g, 2.76 mol, 1.1 equiv, Avra) and sodium bicarbonate (680 g, 6.29 mol, 2.5 equiv). The reaction mixture was degassed with nitrogen for 20 min followed by the addition of palladium acetate (28.8 g, 128.27 mmol, 0.05 equiv, Hindustan Platinum). The reaction mixture was heated at 70 °C for 3h. The reaction was filtered through CELITE^{®} and the CELITE^{®} bed was washed with ethyl acetate (2 x 500 mL). The filtrate was concentrated under reduced pressure to obtain the crude residue which was purified by column chromatography (silica gel; mesh size 60-120, elution 0-20% ethyl acetate in hexanes) to obtain (E)-ethyl 3-(2-amino-5-bromophenyl)acrylate (620 g, 77.0 %), as yellow solid. TLC solvent system: 20% ethyl acetate in hexanes. Product's R_{f} : 0.4. MS (ESI, positive ion) m/z; 270.2 (M+1). ¹H NMR (400 MHz, DMSO) δ 7.75 (d, *J =* 16.1 Hz, 1H), 7.57 (d, *J =* 2.0 Hz, 1H), 7.16 (dd, *J =* 9.1, 2.4 Hz, 1H), 6.66 (d, *J =* 8.6 Hz, 1H), 6.43 (d, *J =* 8.6 Hz, 1H), 5.81 (s, 2H), 4.20 (q, *J =* 7.2 Hz, 2H), 1.27 (t, *J =* 7.2 Hz, 3H).

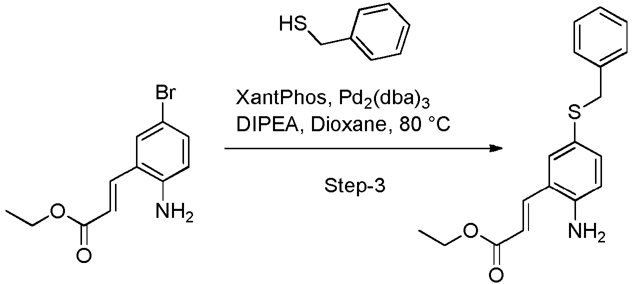

### STEP-3: ETHYL (E)-3-(2-AMINO-5-(BENZYLTHIO)PHENYL)ACRYLATE

To a solution of (E)-ethyl 3-(2-amino-5-bromophenyl)acrylate (620 g, 2.29 mol, 1.0 equiv) in 1,4-dioxane (4.0 L) was added DIPEA (1.26 L, 8.88 mol, 3.9 equiv, GLR) and degassed with nitrogen for 20 mins. XantPhos (92.9 g, 106 mmol, 0.05 equiv, GLR), and tris(dibenzylideneacetone)dipalladium (84 g, 91.0 mmol, 0.04 equiv, Hindustan Platinum) was added to the reaction mixture. The mixture was purged with nitrogen and heated to 80 °C for 30 mins. The reaction was cooled to RT and benzyl mercaptan (455.5 g, 3.67 mol, 1.6 equiv, Alfa Aesar) was added and the reaction was heated at 80 °C for an additional 4 h. The reaction was cooled to room temperature and diluted with ethyl acetate (4.0 L). The mixture was filtered through CELITE^{®} and the CELITE^{®} bed was washed with ethyl acetate (2 x 1.0 L). The filtrate was concentrated under reduced pressure to obtain the crude material which was purified by chromatography (silica gel; mesh size 60-120, elution 0-40% ethyl acetate and petroleum ether) to obtain (E)-ethyl 3-(2-amino-5-(benzylthio)phenyl)acrylate (520 g, 72.0%), as yellow solid. TLC solvent system: 30 % ethyl acetate in hexanes. Product's R_{f} : 0.4. MS (ESI, positive ion) m/z; 314.1 (M+1). ¹H NMR (400 MHz, DMSO) δ 7.79 (d, *J =* 16.1 Hz, 1H), 7.37 (d, *J* = 2.0 Hz, 1H), 7.25 - 7.17 (m, 5H) 7.10 (dd, *J =* 8.4, 2.1 Hz, 1H), 6.61 (d, *J =* 8.3 Hz, 1H), 6.32 (d, *J =* 15.2 Hz, 1H), 5.75 (s, 2H), 4.20 (q, *J =* 7.2 Hz, 2H), 4.01 (s, 2H), 1.27 (t, *J=* 7.2 Hz, 3H).

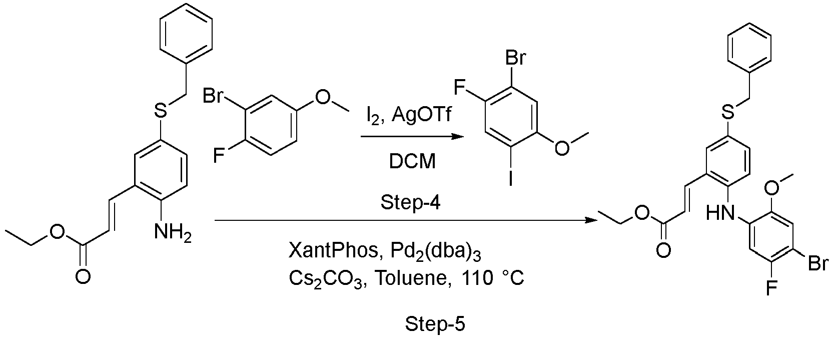

### STEP-4: 1-BROMO-2-FLUORO-4-IODO-5-METHOXYBENZENE

To a solution of 2-bromo-1-fluoro-4-methoxybenzene (500.0 g, 2.44 mol, 1.0 equiv) in DCM (5.0 L) was added silver trifluoromethane sulfonate (686.0 g, 2.68 mol, 1.1 equiv, Angene) and the reaction mixture was stirred for 20 mins. Iodine (678.0 g, 2.68 mol, 1.1 equiv) was added to the reaction and the mixture was stirred at room temperature for 16h. The mixture was diluted with DCM (3.0 L) and filtered through CELITE^{®}. The CELITE bed was washed with DCM (2 x 1.0 L) and the filtrate was washed with 20% aqueous sodium thiosulfate (3.0 L) and saturated aqueous sodium bicarbonate solution (3.0 L). The organic layer was dried over sodium sulfate, filtered and concentrated under reduced pressure to obtain the crude material which was purified by chromatography (silica gel; mesh size 60-120, elution 0-5% ethyl acetate and petroleum ether) to get 1-bromo-2-fluoro-4-iodo-5-methoxybenzene (720 g, 87 %), as off-white solid. TLC solvent system: 100 % hexanes. Product's R_{f}: 0.6. MS (ESI, positive ion) m/z: 331.0 (M+1). ¹H NMR (400 MHz, CDCl₃) δ 7.55 (d, *J =* 7.2 Hz, 1H), 6.95 (d, *J =* 5.6 Hz, 1H), 3.89 (s, 3H).

### STEP-5: ETHYL (E)-3-(5-(BENZYLTHIO)-2-((4-BROMO-5-FLUORO-2-METHOXYPHENYL)AMINO)PHENYL) ACRYLATE

To a solution of (E)-ethyl 3-(2-amino-5-(benzylthio)phenyl)acrylate (300 g, 958.1 mmol, 1.0 equiv) and 1-bromo-2-fluoro-4-iodo-5-methoxybenzene (348.0 g, 1051.6 mmol, 1.1 equiv) in toluene (2.5 L) was added Cs₂CO₃ (468 g, 1436.3 mmol, 1.5 equiv, Spectrochem) and the mixture was degassed with nitrogen for 20 mins. Pd₂(dba)₃ (35 g, 38.2 mmol, 0.04 equiv, Hindustan Platinum) and XantPhos (44.6 g, 76.4 mmol, 0.08 equiv, GLR) were added to the reaction mixture and the mixture was heated at 110 °C for 5h. The reaction mixture was allowed to cool to room temperature, diluted with dichloromethane (2.0 L) and filtered through CELITE^{®} The filtrate was concentrated under reduced pressure to obtain the crude material which was purified by stirring with 5% ethyl acetate in hexanes (3.0 L) for 30 min and filtered to obtain (E)-ethyl 3-(5-(benzylthio)-2-((4-bromo-5-fluoro-2-methoxyphenyl)amino)phenyl)acrylate (350 g, 71 %) as yellow solid. TLC solvent system: 30% ethyl acetate in hexanes. Product's R_{f} : 0.5. MS (ESI, positive ion) m/z; 516.2 (M+1). ¹H NMR (400 MHz, DMSO) δ 7.73 - 7.61 (m, 3H), 7.34 - 7.15 (m, 6H), 7.02 (d, *J =* 11.4 Hz, 1H), 6.60 (d, *J =* 21.2 Hz, 1H), 6.33 (d, *J =* 14.1 Hz, 1H), 4.26 (s, 2H), 4.16 - 4.09 (m, 2H), 3.81 (s, 3H), 1.22 (t, *J =* 7.2 Hz, 3H). Note: NH proton not observed.

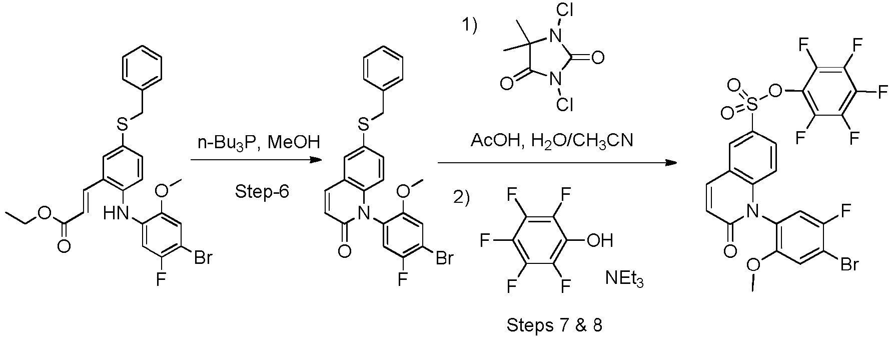

### STEP-6: 6-(BENZYLTHIO)-1-(4-BROMO-5-FLUORO-2-METHOXYPHENYL)QUINOLIN-2(1H)-ONE

To a solution of (E)-ethyl 3-(5-(benzylthio)-2-((4-bromo-5-fluoro-2-methoxyphenyl)amino)phenyl)acrylate (250.0 g, 484.0 mmol, 1.0 equiv) in methanol (2.5 L) was added tri(n-butyl)phosphine (50% solution in ethyl acetate, 48.9 mL, 96.8 mmol, 0.2 equiv, Spectrochem) and the reaction mixture was heated at 70 °C for 5 h. The reaction mixture was allowed to cool to rt, concentrated under reduced pressure to obtain the crude material which was purified by stirring with 5% ethyl acetate in hexanes (1.0 mL) and filtered to obtain 6-(benzylthio)-1-(4-bromo-5-fluoro-2-methoxyphenyl)quinolin-2(1H)-one (201.0 g, 88%) as off white solid. TLC solvent system: 30% ethyl acetate in hexanes. Product's R_{f} : 0.3. MS (ESI, positive ion) m/z; 470.0 (M+1). ¹H NMR (400 MHz, DMSO) δ 7.92 (d,*J* = 9.1 Hz, 1H), 7.79 (d, *J =* 1.7 Hz, 1H), 7.65 (d, *J =* 6.1 Hz, 1H), 7.57 (d, *J =* 8.8 Hz, 1H), 7.40 - 7.22 (m, 6H), 6.68 (d, *J =* 9.6 Hz, 1H), 6.56 (d, *J =* 8.8 Hz, 1H), 4.24 (s, 2H), 3.69 (s, 3H).

### STEPS 7 & 8: PERFLUOROPHENYL 1-(4-BROMO-5-FLUORO-2-METHOXYPHENYL)-2-OXO-1,2-DIHYDRO QUINOLINE-6-SULFONATE

To a solution of 6-(benzylthio)-1-(4-bromo-5-fluoro-2-methoxyphenyl)quinolin-2(1H)-one (250.0 g, 531.5 mmol, 1.0 equiv) in acetonitrile (2.5 L) were added acetic acid (200 mL) and water (130 mL). The resulting mixture was cooled to 0 °C and 1,3-dichloro-5,5-dimethylimidazolidine-2,4-dione (188.5 g, 956.7 mmol, 1.8 equiv, Aldrich) was added portion-wise over 20 min keeping the internal temperature below 5 °C. The resulting suspension was stirred at 0-5 °C under nitrogen for 45 min. Then a solution of pentafluorophenol (127.2 g, 690.95 mmol, 1.3 equiv, Apollo) in acetonitrile (200 mL) was added over 5 min followed by NEt₃ (307.7 mL, 2.12 mol, 4.0 equiv) over 20 min keeping the internal temperature below 5 °C. The mixture was continued to be stirred at 0-5 °C for 30 min. Water (4.0 L) was added and extracted with ethyl acetate (2 x 2.0 L). The organic layer was washed with brine (1.0 L), dried over sodium sulfate, filtered and concentrated under reduced pressure to obtain the crude which was purified by stirring with isopropyl alcohol: hexanes (1:1, 1.0 L) and filtered to obtain racemic perfluorophenyl 1-(4-bromo-5-fluoro-2-methoxyphenyl)-2-oxo-1,2-dihydroquinoline-6-sulfonate (190 g, 60%) as white solid. TLC solvent system: 30 % ethyl acetate in pet ether, Product's R_{f} : 0.4. MS (ESI, positive ion) m/z; 594.2 (M+1). ¹H-NMR (400 MHz, DMSO) δ 8.60 (d, *J =* 2.0 Hz, 1H), 8.26 (d, *J =* 9.8 Hz, 1H), 7.95 (dd, *J =* 2.2, 9.1 Hz, 1H), 7.70 (t, *J =* 8.6 Hz, 2H), 6.95 - 6.88 (m, 2H), 3.72 (s, 3H).

### STEP 9: (P)-PERFLUOROPHENYL 1-(4-BROMO-5-FLUORO-2-METHOXYPHENYL)-2-OXO-1,2-DIHYDROQUINOLINE-6-SULFONATE

Racemic perfluorophenyl 1-(4-bromo-5-fluoro-2-methoxyphenyl)-2-oxo-1,2-dihydroquinoline-6-sulfonate (See Intermediate A1 above, 76.90 g) was separated via Chiralcel OJ column (40% MeOH/60% CO₂) to give (P)-perfluorophenyl 1-(4-bromo-5-fluoro-2-methoxyphenyl)-2-oxo-1,2-dihydroquinoline-6-sulfonate and (M)-perfluorophenyl 1-(4-bromo-5-fluoro-2-methoxyphenyl)-2-oxo-1,2-dihydroquinoline-6-sulfonate as pale yellow flocculent solids. Data for peak 1: m/z (ESI) 594.0 (M+H)⁺. Data for peak 2: m/z (ESI) 594.0 (M+H)⁺.

### STEP 10: (P)-1-(4-BROMO-5-FLUORO-2-METHOXYPHENYL)-N-(ISOXAZOL-3-YL)-N-(4-METHOXYBENZYL)-2-OXO-1,2-DIHYDROQUINOLINE-6-SULFONAMIDE

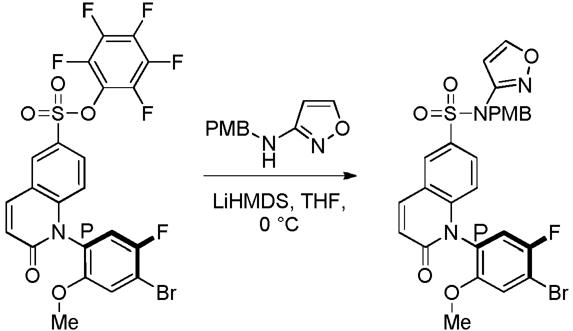

A 250-mL round-bottomed flask was charged with (P)-perfluorophenyl 1-(4-bromo-5-fluoro-2-methoxyphenyl)-2-oxo-1,2-dihydroquinoline-6-sulfonate (See Step 1 of Intermediate B1 above, 11.34 g, 19.08 mmol), and N-(4-methoxybenzyl)isoxazol-3-amine (4.09 g, 20.04 mmol), then purged with nitrogen. Tetrahydrofuran (191 mL) was introduced, and the resultant brown solution cooled to 0 °C. A solution of lithium bis(trimethylsilyl)amide in THF (1.0 M, 21.0 mL, 21.0 mmol) was added dropwise via syringe to the stirred reaction mixture over 10 min. After 15 min, 1.0 N HCl (100 mL) was introduced and the resultant reaction mixture was allowed to warm to RT. The mixture was diluted with and EtOAc (100 mL) and the layers were separated, and the aqueous layer was further extracted with EtOAc (2 × 100 mL). The combined organic layers were then washed with brine (100 mL), dried over anhydrous sodium sulfate, filtered, and concentrated under reduced pressure. The residue was then purified by flash column chromatography (100-g Biotage column, eluent: gradient, 0 to 100% EtOAc in heptane with 10 % CH₂Cl₂ as an additive) to afford (P)-1-(4-bromo-5-fluoro-2-methoxyphenyl)-N-(isoxazol-3-yl)-N-(4-methoxybenzyl)-2-oxo-1,2-dihydroquinoline-6-sulfonamide (9.54 g, 15.53 mmol, 81% yield) as a white amorphous solid. ¹H NMR (400MHz, DMSO-d₆) δ = 8.82 (d, J=2.0 Hz, 1H), 8.38 (d, J=2.3 Hz, 1H), 8.17 (d, J=9.4 Hz, 1H), 7.76 (t, J=5.1 Hz, 1H), 7.68 (d, J=6.1 Hz, 1H), 7.63 (d, J=8.5 Hz, 1H), 7.26 (d, J=7.9 Hz, 2H), 6.91 - 6.78 (m, 4H), 6.74 (d, J=2.0 Hz, 1H), 4.92 (s, 2H), 3.73 - 3.69 (m, 6H), 3.32 (s, 1H). m/z (ESI) 615.1 (M+H)⁺.

### INTERMEDIATE B1: (P)-1-(4-BROMO-5-FLUORO-2-METHOXYPHENYL)-N-(ISOXAZOL-3-YL)-2-OXO-1,2-DIHYDROQUINOLINE-6-SULFONAMIDE

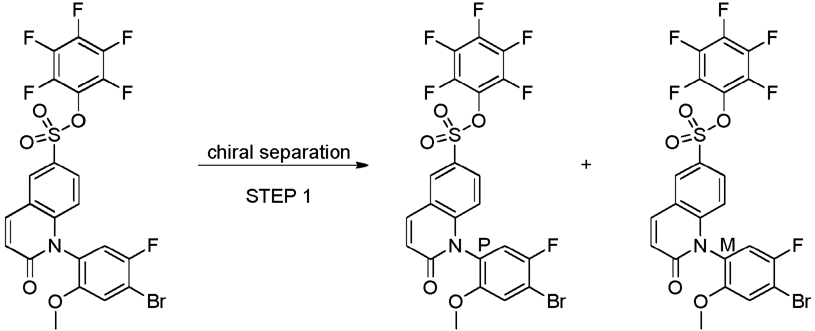

### STEP 1: (P)-PERFLUOROPHENYL 1-(4-BROMO-5-FLUORO-2-METHOXYPHENYL)-2-OXO-1,2-DIHYDROQUINOLINE-6-SULFONATE

Racemic perfluorophenyl 1-(4-bromo-5-fluoro-2-methoxyphenyl)-2-oxo-1,2-dihydroquinoline-6-sulfonate (See Intermediate A1 above, 76.90 g) was separated via Chiralcel OJ column (40% MeOH/60% CO₂) to give (P)-perfluorophenyl 1-(4-bromo-5-fluoro-2-methoxyphenyl)-2-oxo-1,2-dihydroquinoline-6-sulfonate and (M)-perfluorophenyl 1-(4-bromo-5-fluoro-2-methoxyphenyl)-2-oxo-1,2-dihydroquinoline-6-sulfonate as pale yellow flocculent solids. Data for peak 1: m/z (ESI) 594.0 (M+H)⁺. Data for peak 2: m/z (ESI) 594.0 (M+H)⁺.

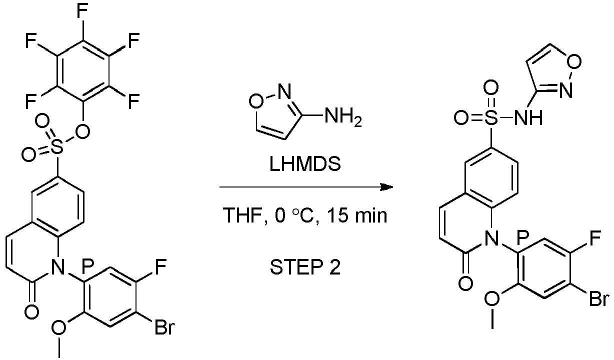

### STEP 2: (P)-1-(4-BROMO-5-FLUORO-2-METHOXYPHENYL)-N-(ISOXAZOL-3-YL)-2-OXO-1,2-DIHYDROQUINOLINE-6-SULFONAMIDE

A THF (200 mL) solution of (P)-perfluorophenyl 1-(4-bromo-5-fluoro-2-methoxyphenyl)-2-oxo-1,2-dihydroquinoline-6-sulfonate (6.00 g, 10.10 mmol) and 3-aminoisoxazole (0.821 ml, 11.11 mmol) in a 250-mL round-bottom flask was cooled to 0 °C, and lithium bis(trimethylsilyl)amide, 1.0 M solution in THF (21.20 ml, 21.20 mmol) was added dropwise. After stirring the yellow solution at 0 °C for 15 min, it was quenched at 0 °C with 1 N HCl and extracted thrice with EtOAc. The organic extracts were combined, dried over MgSO₄, filtered, and concentrated to a light tan residue. Et₂O was added, and the slurry was titurated and sonicated. Filtration afforded an off-white solid, which was washed twice with Et₂O and dried in vacuo to afford 3.88 g of product as an off-white solid. The filtrate was concentrated in vacuo and purified via column chromatography (12 g silica gel, 35% to 100% EtOAc/hept gradient) to afford an additional 1.36 g of product as a pale yellow flocculent solid. A total of 5.24 g of (P)-1-(4-bromo-5-fluoro-2-methoxyphenyl)-N-(isoxazol-3-yl)-2-oxo-1,2-dihydroquinoline-6-sulfonamide was afforded. m/z (ESI) 494.1 (M+H)⁺.

### INTERMEDIATE C1: (P)- 1-(4-BROMO-5-CHLORO-2-METHOXYPHENYL)-N-(ISOXAZOL-3-YL)-2-OXO-1,2-DIHYDROQUINOLINE-6-SULFONAMIDE

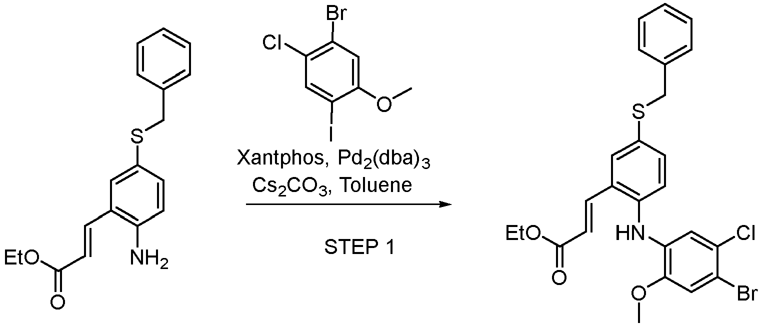

### STEP 1: ETHYL (E)-3-(5-(BENZYLTHIO)-2-((4-BROMO-5-CHLORO-2-METHOXYPHENYL)AMINO)PHENYL) ACRYLATE

To a solution of ethyl (E)-3-(2-amino-5-(benzylthio)phenyl)acrylate (175 g, 555.0 mmol, 1.0 equiv) and 1-bromo-2-chloro-4-iodo-5-methoxybenzene (231.3 g, 666.2 mmol, 1.1 equiv) in toluene (1.5 L) was added cesium carbonate (357.5 g, 1100 mmol, 2.0 equiv) and the mixture was degassed with nitrogen for 20 mins. Pd2(dba)3 (12.5 g, 13.0 mmol, 0.025 equiv,) and xantphos (15.8 g, 27.2 mmol, 0.05 equiv) were added to the reaction mixture and the mixture was heated at 110 °C for 5h. The reaction mixture was allowed to cool to room temperature, diluted with dichloromethane (1.0 L) and filtered through celite. The filtrate was concentrated under reduced pressure to obtain the crude material which was purified by stirring with 5% ethyl acetate in hexane (1.5 L) for 30 min and filtered to obtain ethyl (E)-3-(5-(benzylthio)-2-((4-bromo-5-chloro-2-methoxyphenyl)amino)phenyl)acrylate (290 g, 85 %) as yellow solid. m/z (ESI) 532.2 (M+H)⁺.

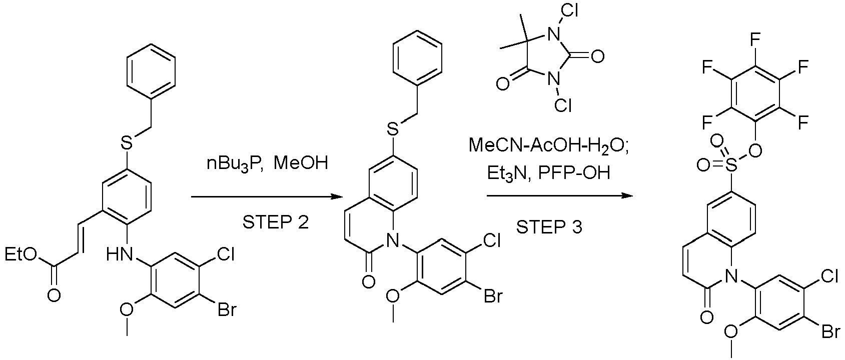

### STEP 2: 6-(BENZYLTHIO)-1-(4-BROMO-5-CHLORO-2-METHOXYPHENYL)QUINOLIN-2(1H)-ONE

To a solution of ethyl (E)-3-(5-(benzylthio)-2-((4-bromo-5-chloro-2-methoxyphenyl)amino)phenyl)acrylate (300.0 g, 5630.0 mmol, 1.0 equiv) in methanol (3.0 L) was added tri(n-butyl)phosphine (50% solution in ethyl acetate, 56.2 mL, 1126 mmol, 0.2 equiv) and the reaction mixture was heated at 70 °C for 5 h. The reaction mixture was allowed to cool to room temperature, concentrated under reduced pressure to obtain the crude material which was purified by stirring with 5% ethyl acetate in hexane (1.0 mL) and filtered to obtain 6-(benzylthio)-1-(4-bromo-5-chloro-2-methoxyphenyl)quinolin-2(1H)-one (210.0 g, 76.6 %) as an off white solid. m/z (ESI) 486.0 (M+H)⁺.

### STEP 3: PERFLUOROPHENYL 1-(4-BROMO-5-FLUORO-2-METHOXYPHENYL)-2-OXO-1,2-DIHYDRO QUINOLINE-6-SULFONATE

To a solution of 6-(benzylthio)-1-(4-bromo-5-chloro-2-methoxyphenyl)quinolin-2(1H)-one (400.0 g, 824.9 mmol, 1.0 equiv) in acetonitrile (2.5 L) and THF (2.5 L) were added acetic acid (1.0 L) and water (700 mL). The resulting mixture was cooled to 0 °C and 1,3-dichloro-5,5-dimethylimidazolidine-2,4-dione (292 g, 1484.8 mmol, 1.8 equiv) was added portion-wise over 30 min keeping the internal temperature below 5 °C. The resulting suspension was stirred at 0 °C under nitrogen for 45 min. Then a solution of pentafluorophenol (197.4 g, 1072.3 mmol, 1.3 equiv) in acetonitrile (500 mL) was added over 5 min followed by triethylamine (477 mL, 3299 mmol, 4.0 equiv) over 30 min keeping the internal temperature below 5 °C. The mixture was continued to be stirred at 0 °C for 50 min. Water (4.0 L) was added and extracted with ethyl acetate (3 x 2.0 L). The organic layer was washed with brine (2.0 L), dried over sodium sulfate, filtered and concentrated under reduced pressure to obtain the crude which was purified by stirring with isopropyl alcohol:hexane (1:1, 2.0 L) and filtered to obtain perfluorophenyl 1-(4-bromo-5-fluoro-2-methoxyphenyl)-2-oxo-1,2-dihydro quinoline-6-sulfonate (360 g, 72%) as a white solid. m/z (ESI) 610.6 (M+H)⁺.

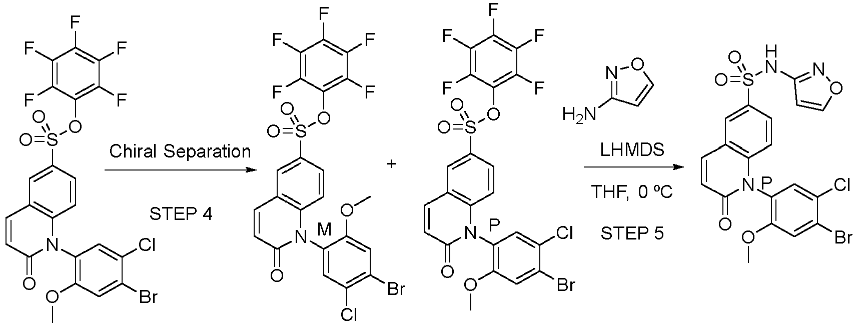

### STEP 4: (P)- PERFLUOROPHENYL 1-(4-BROMO-5-FLUORO-2-METHOXYPHENYL)-2-OXO-1,2-DIHYDRO QUINOLINE-6-SULFONATE & (M)- PERFLUOROPHENYL 1-(4-BROMO-5-FLUORO-2-METHOXYPHENYL)-2-OXO-1,2-DIHYDRO QUINOLINE-6-SULFONATE

1-(4-bromo-5-fluoro-2-methoxyphenyl)-2-oxo-1,2-dihydro quinoline-6-sulfonate (156 g, 255 mmol) was purified via chiral SFC chromatography ((S,S) Whelk-O, 45% isopropanol) to afford (P)- perfluorophenyl 1-(4-bromo-5-chloro-2-methoxyphenyl)-2-oxo-1,2-dihydroquinoline-6-sulfonate (72.66 g, 93% yield) and (M)- perfluorophenyl 1-(4-bromo-5-chloro-2-methoxyphenyl)-2-oxo-1,2-dihydroquinoline-6-sulfonate (76.13 g, 98% yield) as white solids. m/z (ESI) 610.6 (M+H)⁺.

### STEP 5: (P)- 1-(4-BROMO-5-CHLORO-2-METHOXYPHENYL)-N-(ISOXAZOL-3-YL)-2-OXO-1,2-DIHYDROQUINOLINE-6-SULFONAMIDE

To a 100 mL RB flask was added (P)-perfluorophenyl 1-(4-bromo-5-chloro-2-methoxyphenyl)-2-oxo-1,2-dihydroquinoline-6-sulfonate (2.56 g, 4.19 mmol). The flask was placed under nitrogen, then THF (41.9 ml) and isoxazol-3-amine (0.423 g, 5.03 mmol) were added. The mixture was cooled to 0 °C for 10 min, then added LHMDS 1.0 M in THF (8.80 ml, 8.80 mmol) dropwise over 5 min. The reaction was stirred for two hours. While still cold, added 1N HCl (50 mL) and EtOAc (50 mL). The layers were separated, and the organic layer was washed again with 1N HCl. Combined aqueous layers and extracted with EtOAc (2x50 mL). All combined organics were dried with sodium sulfate, filtered and concentrated. The material was purified by chromatography on silica gel (40-100% EtOAc/heptane) to afford (P)-1-(4-bromo-5-chloro-2-methoxyphenyl)-N-(isoxazol-3-yl)-2-oxo-1,2-dihydroquinoline-6-sulfonamide (2.03 g, 3.97 mmol, 95 % yield) as an off-white solid. ¹H NMR (400 MHz, DMSO-*d*₆) δ = 11.66 (br. s., 1 H), 8.69 (d, *J*=1.47 Hz, 1 H), 8.35 (d, *J=*2.15 Hz, 1 H), 8.22 (d, *J*=9.59 Hz, 1 H), 7.83 (dd, *J*=8.95, 2.20 Hz, 1 H), 7.77 (s, 1 H) 7.70 - 7.74 (m, 1 H), 6.85 (d, *J*=8.90 Hz, 1 H), 6.79 (d, *J*=9.59 Hz, 1 H), 6.42 (d, *J*=1.76 Hz, 1 H), 3.72 (s, 3 H). m/z (ESI) 511.0 (M+H)⁺.

### INTERMEDIATE D1: (P)-PERFLUOROPHENYL 1-(4-BROMO-5-FLUORO-2-METHOXYPHENYL)-7-FLUORO-2-OXO-1,2-DIHYDROQUINOLINE-6-SULFONATE.

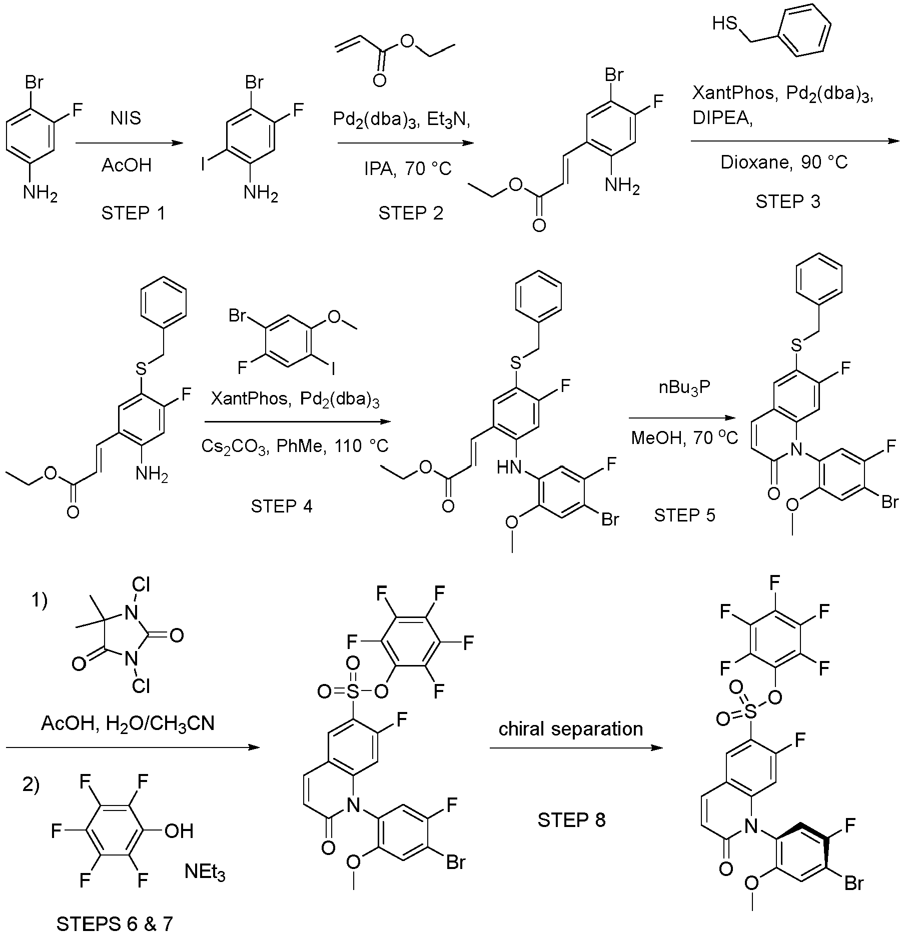

### STEP 1: 4-BROMO-5-FLUORO-2-IODOANILINE

N-iodosuccinimide (710 g, 3158 mmol) was added portion-wise to a solution of 4-bromo-3-fluoroaniline (500 g, 2631 mmol) in acetic acid (4000 mL) at 10-15°C. The reaction was stirred at rt for 1 hour. The reaction was then quenched with ice water (7 L) and the precipitated solid was filtered. The solid was washed with 5 % sodium thiosulphate soution (6 L) and water (4 L), and dried to afford 4-bromo-5-fluoro-2-iodoaniline as brown solid (750 g, 2374 mmol, 90% yield). ¹H NMR(400 MHz, DMSO-*d*6): δ ppm 7.76 (d, *J*=7.8 Hz, 1 H), 6.68 (d, *J*=11.5 Hz, 1 H), 5.68 (s, 2 H).

### STEP 2: ETHYL (E)-3-(2-AMINO-5-BROMO-4-FLUOROPHENYL)ACRYLATE

To a stirred solution of 4-bromo-5-fluoro-2-iodoaniline (500 g, 1583 mmol) in isopropanol (2550 mL) was added triethylamine (331 mL, 2374 mmol) at room temperature. The reaction mixture was degassed with nitrogen for 20 minutes. Tris(dibenzylideneacetone)dipalladium (0) (36.2 g, 39.6 mmol) was added, followed by slow addition of ethyl acrylate (162 g, 1614 mmol) under nitrogen atmosphere. Then the reaction mixture was heated to 70 °C and stirred for 6 hours. After completion, the reaction mixture was filtered through Celite and washed with dichloromethane (2 L). The filtrate was concentrated under reduced pressure to give the crude residue. The crude residue was stirred in 3% ethyl aceatate in petether (6 L) and filtered. The solid obtained was washed with 3% ethyl aceatate in petether (2 L) and dried to give ethyl (E)-3-(2-amino-5-bromo-4-fluorophenyl)acrylate (433 g, 1505 mmol, 95% yield) as a yellow solid. MS (ESI, positive ion) m/z: 288.0 (M+1). ¹H NMR(400 MHz, DMSO-*d*6): δ ppm 7.69 - 7.98 (m, 2 H), 6.61 (d, *J*=11.4 Hz, 1 H), 6.45 (d, *J*=15.6 Hz, 1 H), 6.12 (s, 2 H), 4.17 (q, *J*=7.1 Hz, 2 H), 1.26 (t, *J*=7.1 Hz, 3 H).

### STEP 3: ETHYL (E)-3-(2-AMINO-5-(BENZYLTHIO)-4-FLUOROPHENYL)ACRYLATE

To a solution of ethyl (E)-3-(2-amino-5-bromo-4-fluorophenyl)acrylate (500.0 g, 1735 mmol) in 1,4-dioxane (2500 mL) was added N-ethyl-N-isopropylpropan-2-amine (449 g, 3471 mmol) and degassed with nitrogen for 20 minutes. (9,9-dimethyl-9H-xanthene-4,5-diyl)bis(diphenylphosphane) (50.2 g, 87 mmol)
and tris(dibenzylideneacetone)dipalladium (0) (39.7 g, 43.4 mmol) were added to the reaction mixture. The mixture was purged with nitrogen and heated to 80 °C for 10 minutes. The reaction was cooled to room temperature and phenylmethanethiol (237 g,
1909 mmol) was added. The reaction was heated at 90 °C for 12 hour. The reaction was cooled to room temperature and diluted with ethyl acetate (1000 mL). The mixture was filtered through Celite and the Celite bed was washed with ethyl acetate (2500 mL). The filterate was concentrated under reduced pressure to obtain the crude material. The crude material was purified by column chromatography (silica gel; mesh size 60-
120, gradient elution 0-15% ethyl acetate and petroleum ether) to obtain ethyl (E)-3-(2-amino-5-(benzylthio)-4-fluorophenyl)acrylate (300.0 g, 905 mmol, 52% yield) as yellow solid. MS (ESI, positive ion) m/z: 332.1 (M+1). ¹H NMR(400 MHz, DMSO-*d*6): δ ppm 7.72 (d, *J*=15.7 Hz, 1 H), 7.41 (d, *J*=8.5 Hz, 1 H), 7.01 - 7.32 (m, 5 H), 6.38 - 6.55 (m, 1 H), 6.24 (d, *J*=15.7 Hz, 1 H), 6.11 (s, 2 H), 4.17 (q, *J*=7.1 Hz, 2 H), 3.89 - 4.07 (m, 2 H), 1.26 (t, *J*=7.1 Hz, 3 H).

### STEP 4: ETHYL (E)-3-(5-(BENZYLTHIO)-2-((4-BROMO-5-FLUORO-2-METHOXYPHENYL)AMINO)-4-FLUOROPHENYL)ACRYLATE

To a 250 mL 3-neck round-bottomed flask charged with ethyl (E)-3-(2-amino-5-(benzylthio)-4-fluorophenyl)acrylate (10 g, 30.2 mmol) and 1-bromo-2-fluoro-4-iodo-5-methoxybenzene (10.48 g, 31.7 mmol) in toluene (100 mL) was added cesium carbonate (39.3 g, 121 mmol). The mixture was degassed with nitrogen for 15 minutes. Tris(dibenzylideneacetone)dipalladium (0) (1.105 g, 1.207 mmol) and (9,9-dimethyl-9H-xanthene-4,5-diyl)bis(diphenylphosphane) (1.397 g, 2.414 mmol) were added to the reaction mixture and the mixture was heated at 110 °C for 16 hours. The reaction mixture was allowed to cool to room temperature, diluted with dichloromethane (200 mL) and filtered through Celite. The filtrate was concentrated under reduced pressure to obtain the crude material which was purified by stirring with methanol (250 mL) for 1 hour and filtered. The cake was washed with methanol (100 mL) and dried to obtaine ethyl (E)-3-(5-(benzylthio)-2-((4-bromo-5-fluoro-2-methoxyphenyl)amino)-4-fluorophenyl)acrylate (13.5 g, 25.3 mmol, 84 % yield) as yellow solid. MS (ESI, positive ion) m/z: 534.0 (M+1). ¹H NMR(400 MHz, DMSO-*d*6): δ ppm 7.97 (s, 1 H), 7.75 (d, *J*=8.4 Hz, 1 H), 7.66 (d, J=15.9 Hz, 1 H), 7.05 - 7.43 (m, 6 H), 6.77 (d, *J*=11.1 Hz, 1 H), 6.63 (d, *J*=10.2 Hz, 1 H), 6.52 (d, *J*=15.9 Hz, 1 H), 4.25 (s, 2 H), 4.16 (q, *J*=7.1 Hz, 2 H), 3.82 (s, 3 H), 1.23 (t, *J*=7.1 Hz, 3 H).

### STEP 5: 6-(BENZYLTHIO)-1-(4-BROMO-5-FLUORO-2-METHOXYPHENYL)-7-FLUOROQUINOLIN-2(1H)-ONE

To 500 mL 3-necked round-bottom flask was charged with ethyl (E)-3-(5-(benzylthio)-2-((4-bromo-5-fluoro-2-methoxyphenyl)amino)-4-fluorophenyl)acrylate (13.5 g, 25.3 mmol) in methanol (140 mL) was added tributylphosphane (50% solution in ethylacetate) (3.74 mL, 7.58 mmol). The reaction mixture was heated at 70 °C for 5 hours. The reaction mixture was allowed to cool 15 °C, filtered and washed with cold methanol (100 mL) and dried to give 6-(benzylthio)-1-(4-bromo-5-fluoro-2-methoxyphenyl)-7-fluoroquinolin-2(1H)-one (9.5 g, 19.45 mmol, 77 % yield) as yellow solid. MS
(ESI, positive ion) m/z: 488.0 (M+1). ¹H NMR(400 MHz, DMSO-*d*6): δ ppm 7.88 - 8.02 (m, 2 H), 7.64 (d, *J*=6.3 Hz, 1 H), 7.56 (d, *J*=8.6 Hz, 1 H), 7.20 - 7.38 (m, 5 H), 6.64 (d, *J*=9.6 Hz, 1 H), 6.48 (d, *J*=11.3 Hz, 1 H), 4.23 (s, 2 H), 3.71 (s, 3 H).

### STEP 6 & 7: PERFLUOROPHENYL 1-(4-BROMO-5-FLUORO-2-METHOXYPHENYL)-7-FLUORO-2-OXO-1,2-DIHYDROQUINOLINE-6-SULFONATE.

To a 250 mL 3-necked round-bottom flask charged with 6-(benzylthio)-1-(4-bromo-5-fluoro-2-methoxyphenyl)-7-fluoroquinolin-2(1H)-one (9.5 g, 19.45 mmol) in acetonitrile (95 mL) were added acetic acid (6.4 mL) and water (4.13 mL). The resulting mixture was cooled to 0-5 °C and 1,3-dichloro-5,5-dimethylimidazolidine-2,4-dione (6.13 g, 31.1 mmol) was added portion-wise over 10-20 min keeping the internal temperature below 5-10 °C. The resulting suspension was stirred at 5-10 °C under nitrogen for 45 minutes. Then a solution of 2,3,4,5,6-pentafluorophenol (7.16 g, 38.9 mmol) in acetonitrile (10 mL) was added over 10-15 min, followed by triethylamine (13.54 mL, 97 mmol) over 20 min keeping the internal temperature below 5-10 °C. The mixture was continued to be stirred at 5-10°C for 30 min. Ice water (200 mL) was added and the precipitated solid was filtered and washed with water (100 mL). The crude was purified by stirring with methanol (50 mL), filtered, washed with MeOH (50 mL) and dried to obtain perfluorophenyl 1-(4-bromo-5-fluoro-2-methoxyphenyl)-7-fluoro-2-oxo-1,2-dihydroquinoline-6-sulfonate (9.5 g, 15.52 mmol, 80 % yield) as an off white solid. MS (ESI, positive ion) m/z; 612.0 (M+1). ¹H NMR(400 MHz, DMSO-*d*6): δ ppm 8.53 (d, *J*=7.4 Hz, 1 H), 8.20 (d, *J*=9.8 Hz, 1 H), 7.67 (dd, *J*=16.2, 7.4 Hz, 2H), 6.99 (d, *J*=12.1 Hz, 1 H), 6.83 (d, *J*=9.8 Hz, 1 H), 3.74 (s, 3 H).

### STEP 8: (P)-PERFLUOROPHENYL 1-(4-BROMO-5-FLUORO-2-METHOXYPHENYL)-7-FLUORO-2-OXO-1,2-DIHYDROQUINOLINE-6-SULFONATE.

Perfluorophenyl 1-(4-bromo-5-fluoro-2-methoxyphenyl)-7-fluoro-2-oxo-1,2-dihydroquinoline-6-sulfonate (135 g, 220 mmol) was purified by SFC via an Regis Whelk-O s,s 5x15 cm, 5 µm column; a mobile phase of 50% dichloromethane using a flowrate of 350 mL/min to generate (P)-perfluorophenyl 1-(4-bromo-5-fluoro-2-methoxyphenyl)-7-fluoro-2-oxo-1,2-dihydroquinoline-6-sulfonate (49.2 g, 80.4 mmol, 36% yield). MS (ESI, positive ion) m/z: 612.7 (M+1).

### INTERMEDIATE E1: (P)-1-(4-BROMO-5-FLUORO-2-METHOXYPHENYL)-7-FLUORO-N-(ISOXAZOL-3-YL)-N-(4-METHOXYBENZYL)-2-OXO-1,2-DIHYDROQUINOLINE-6-SULFONAMIDE

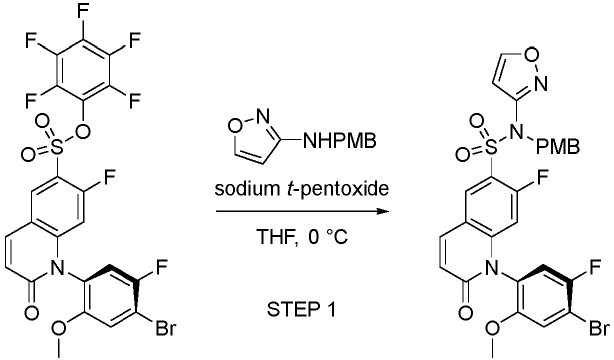

A 100 mL round-bottom flask was charged with (P)-perfluorophenyl 1-(4-bromo-5-fluoro-2-methoxyphenyl)-7-fluoro-2-oxo-1,2-dihydroquinoline-6-sulfonate (6.01 g, 9.82 mmol) and N-(4-methoxybenzyl)isoxazol-3-amine (2.48 g, 12.14 mmol). The flask was sparged with nitrogen for 5 minutes prior to the addition of tetrahydrofuran (20 mL). The mixture was cooled to -78 °C, following which sodium tert-pentoxide, 30% solution in THF (6 mL, 15.00 mmol) was added dropwise. After 15 minutes, the reaction was quenched with 5 M aqueous ammonium chloride and then warmed to ambient temperature. The mixture was extracted with EtOAc (2X). the organic layer was separated and concentrated under reduce pressure. The residue was purified by column chromatography (Biotage Isolera One, Biotage Sfar silica HC D 20 um 50g, 0-80% ethyl acetate in heptane with 10% dichloromethane as additive) to provide (P)-1-(4-bromo-5-fluoro-2-methoxyphenyl)-7-fluoro-N-(isoxazol-3-yl)-N-(4-methoxybenzyl)-2-oxo-1,2-dihydroquinoline-6-sulfonamide (5.0 g, 7.91 mmol, 81% yield) as a white solid. m/z (ESI, positive ion) 634.0 (M+H)⁺.

### Example 1

### (P)-1-(5-CHLORO-2-METHOXY-4-((TRIFLUOROMETHOXY)METHYL)PHENYL)-N-(ISOXAZOL-3-YL)-2-OXO-1,2-DIHYDROQUINOLINE-6-SULFONAMIDE

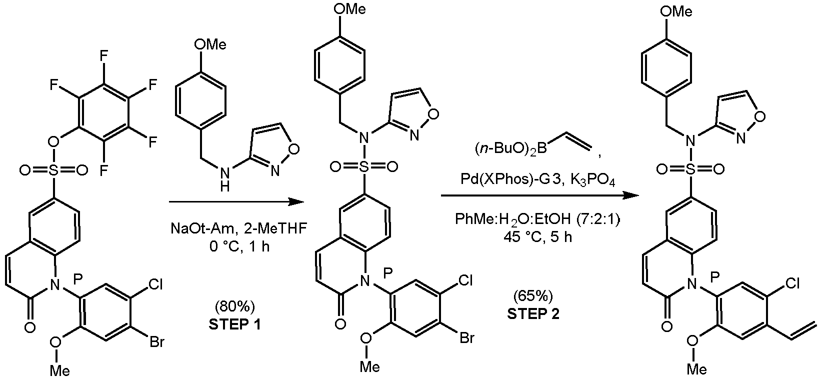

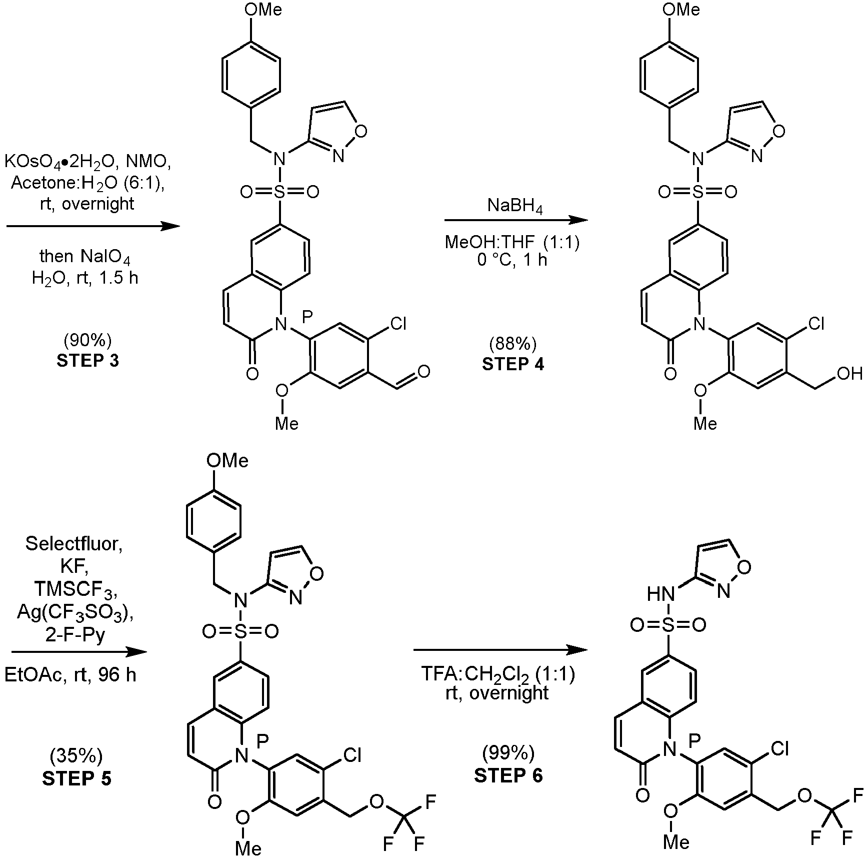

Step 1: To a solution of perfluorophenyl (P)-1-(4-bromo-5-chloro-2-methoxyphenyl)-2-oxo-1,2-dihydroquinoline-6-sulfonate (20 g, 32.7 mmol) and N-(4-methoxybenzyl)isoxazol-3-amine (7.36 g, 36.0 mmol) in 2-methyltetrahydrofuran (99 mL), cooled to -0.8 °C in a brined ice-bath, was added a solution of sodium tert-pentoxide (1.4M in THF, 28.1 mL, 39.3 mmol) dropwise at a rate such that the internal reaction temperature did not exceed 3 °C. After stirring for 1 hour, the solution was transferred into another chilled vessel containing 100 mL 2N aqueous HCl and 100 mL EtOAc. The layers were separated, and the aqueous layer was extracted with EtOAc. The combined organic layers were dried over magnesium sulfate, filtered, and concentrated. The residue was diluted with 2-propanol (500 mL), causing a white solid to precipitate. The mixture stirred overnight at ambient temperature, and the solids were isolated by filtration and washed with 2-propanol to provide (P)-1-(4-bromo-5-chloro-2-methoxyphenyl)-N-(isoxazol-3-yl)-N-(4-methoxybenzyl)-2-oxo-1,2-dihydroquinoline-6-sulfonamide (16.5 g, 26.2 mmol, 80% yield) as an off-white solid. m/z (ESI) 631.4 (M+H)⁺.

Step 2: A round-bottom flask was charged with (P)-1-(4-bromo-5-chloro-2-methoxyphenyl)-N-(isoxazol-3-yl)-N-(4-methoxybenzyl)-2-oxo-1,2-dihydroquinoline-6-sulfonamide (6.39 g, 10.1 mmol), (2-dicyclohexylphosphino-2',4',6'-triisopropyl-1,1'-biphenyl)[2-(2'-amino-1,1'-biphenyl)]palladium(ii) methanesulfonate (1.29 g, 1.52 mmol), potassium phosphate (6.45 g, 30.4 mmol), and dibutyl vinylboronate (3.73 g, 4.47 mL, 20.3 mmol). Ethanol (10.1 mL), water (20.3 mL), and toluene (70.9 mL) were then introduced and the resultant reaction mixture was sparged with nitrogen for 10 minutes before being warmed to 45 °C. After ~5 hours, the reaction mixture was cooled to ambient temperature and the reaction mixture was diluted with water and extracted with ethyl acetate. The organic layer was washed with brine, dried over anhydrous magnesium sulfate, filtered, and concentrated under reduced pressure. The residue was purified by silica gel column chromatography using a gradient of 0-50% ethyl acetate/EtOH (3:1) in heptane to afford (P)-1-(5-chloro-2-methoxy-4-vinylphenyl)-N-(isoxazol-3-yl)-N-(4-methoxybenzyl)-2-oxo-1,2-dihydroquinoline-6-sulfonamide (3.78 g, 6.54 mmol, 65 % yield). m/z (ESI) 579.0 (M+H)⁺.

Step 3: To a solution of (P)-1-(5-chloro-2-methoxy-4-vinylphenyl)-N-(isoxazol-3-yl)-N-(4-methoxybenzyl)-2-oxo-1,2-dihydroquinoline-6-sulfonamide (3.78 g, 6.54 mmol) in water (9.34 mL) and acetone (56.1 mL) was added 4-methylmorpholine 4-oxide (1.19 g, 9.81 mmol) and potassium osmate dihydrate (0.022 g, 0.065 mmol) at ambient temperature. The resultant reaction mixture was stirred for 16 h before a solution of sodium periodate (2.80 g, 13.1 mmol) in water (8.9 mL) was introduced. The resultant reaction mixture was stirred at ambient temperature for 1.5 hours. The reaction mixture was then diluted with dichloromethane, the layers were separated, and the aqueous layer was extracted with dichloromethane. The combined organic layers was dried over anhydrous magnesium sulfate, filtered, and concentrated under reduced pressure. The residue was purified by silica gel column chromatography eluting with a gradient of 0-60% EtOAc/EtOH in heptanes to afford (P)-1-(5-chloro-4-formyl-2-methoxyphenyl)-N-(isoxazol-3-yl)-N-(4-methoxybenzyl)-2-oxo-1,2-dihydroquinoline-6-sulfonamide (3.40 g, 5.86 mmol, 90% yield). m/z (ESI) 581.0 (M+H)⁺.

Step 4: A 250 mL round-bottom flask was charged with (P)-1-(5-chloro-4-formyl-2-methoxyphenyl)-N-(isoxazol-3-yl)-N-(4-methoxybenzyl)-2-oxo-1,2-dihydroquinoline-6-sulfonamide (3.78 g, 6.52 mmol), methanol (21.7 mL) and tetrahydrofuran (21.7 mL). Sodium borohydride (0.264 g, 6.84 mmol) was added to the reaction in a portionwise fasion at 0 °C. After stirring for 1 hour at 0 °C the reaction mixture was quenched with water and a saturated aqueous solution of ammonium chloride and extracted with ethyl acetate. The organic layer was washed with brine, dried over anhydrous sodium sulfate, filtered, and concentrated in vacuo to afford (P)-1-(5-chloro-4-(hydroxymethyl)-2-methoxyphenyl)-N-(isoxazol-3-yl)-N-(4-methoxybenzyl)-2-oxo-1,2-dihydroquinoline-6-sulfonamide (3.34 g, 5.74 mmol, 88 % yield). m/z (ESI) 582.0 (M+H)+.

Step 5: A 40 mL vial was charged with (P)-7-fluoro-1-(5-fluoro-4-(hydroxymethyl)-2-methoxyphenyl)-N-(isoxazol-3-yl)-N-(4-methoxybenzyl)-2-oxo-1,2-dihydroquinoline-6-sulfonamide (3.33 g, 5.72 mmol) and 1-(chloromethyl)-4-fluoro-1,4-diazabicyclo[2.2.2]octane-1,4-diium tetrafluoroborate (3.04 g, 8.58 mmol). The vial was transferred into a glovebox before potassium fluoride (997 mg, 17.2 mmol) and silver(I) trifluoromethanesulfonate (2.94 g, 11.44 mmol) were added. The vial was then capped and removed from the glovebox before ethyl acetate (14.3 mL), 2-fluoropyridine (1.11 g, 0.985 mL, 11.4 mmol), and trimethyl(trifluoromethyl)silane (1.63 g, 1.69 mL, 11.4 mmol) were introduced.

The resultant reaction mixture stirred at ambient temperature for 96 hours. After this time, the reaction mixture was filtered through a plug of silica gel and eluted with ethyl acetate. The filtrate was concentrated under reduced pressure and the residue purified by silica gel column chromatography eluting with a gradient of 0-30% EtOAc-EtOH (3:1) in heptanes to give (P)-1-(5-chloro-2-methoxy-4-((trifluoromethoxy)methyl)phenyl)-N-(isoxazol-3-yl)-N-(4-methoxybenzyl)-2-oxo-1,2-dihydroquinoline-6-sulfonamide (1.30 g, 2.00 mmol, 35.0 % yield). m/z (ESI) 650.0 (M+H)⁺.

Step 6: A round-bottom flask was charged with (P)-1-(5-chloro-2-methoxy-4-((trifluoromethoxy)methyl)phenyl)-N-(isoxazol-3-yl)-N-(4-methoxybenzyl)-2-oxo-1,2-dihydroquinoline-6-sulfonamide (1.30 g, 2.00 mmol), dichloromethane (8.0 mL),
and trifluoroacetic acid (13.1 g, 8.82 mL, 114 mmol). The reaction mixture was stirred at ambient temperature. After 16 h, the reaction mixture was concentrated in vacuo and the residue was purifed via silica gel column chromatography eluting with a gradient of 0-75% EtOAc-EtOH (3:1) in heptanes to give (P)-1-(5-chloro-2-methoxy-4-((trifluoromethoxy)methyl)phenyl)-N-(isoxazol-3-yl)-2-oxo-1,2-dihydroquinoline-6-sulfonamide (1.05 g, 1.98 mmol, 99 % yield). 1H NMR (500 MHz, DMSO-d6) δ 11.64 (br s, 1 H), 8.72 (d, J=1.82 Hz, 1 H), 8.37 (d, J=2.21 Hz, 1 H), 8.22 (d, J=9.60 Hz, 1 H), 7.85 (dd, J=8.95, 2.21 Hz, 1 H), 7.68 (s, 1 H), 7.50 - 7.64 (m, 1 H), 6.79 (dd, J=9.28, 4.87 Hz, 2 H), 6.44 (d, J=1.82 Hz, 1H), 5.28 - 5.35 (m, 2 H), 3.67 - 3.76 (m, 3 H). m/z (ESI) 530.0 (M+H)⁺.

### Example 2

### (P)-1-(5-FLUORO-2-METHOXY-4-((TRIFLUOROMETHOXY)METHYL)PHENYL)-N-(ISOXAZOL-3-YL)-2-OXO-1,2-DIHYDROQUINOLINE-6-SULFONAMIDE

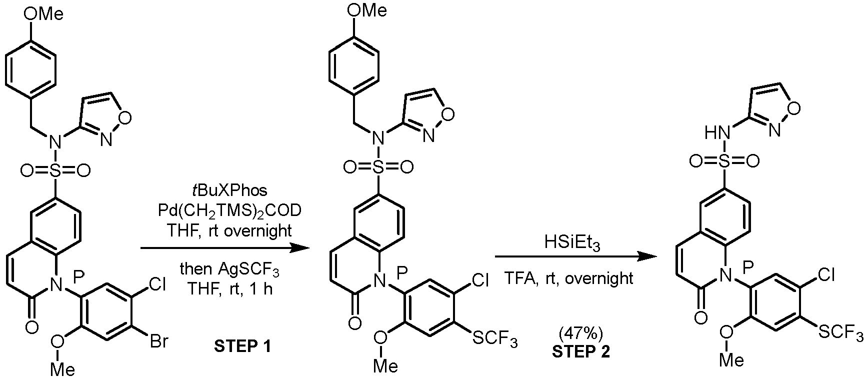

Step 1: A 40 mL vial was charged with (P)-1-(4-bromo-5-chloro-2-methoxyphenyl)-N-(isoxazol-3-yl)-N-(4-methoxybenzyl)-2-oxo-1,2-dihydroquinoline-6-sulfonamide (824 mg, 1.31 mmol), 2-di-t-butylphosphino-2,4,6-tri-i-propyl-1,1-biphenyl (555 mg, 1.31 mmol), and (1,5-cyclooctadiene)bis(trimethylsilylmethyl)palladium(ii) (508 mg, 1.31 mmol). The vial was purged with nitrogen for 10 minutes prior to the addition of tetrahydrofuran (6.53 mL). The vial was capped and the reaction was stirred at ambient temperature. After 16 h, (trifluoromethylthio) silver (355 mg, 1.70 mmol) was added in one portion to the reaction mixture. The resultant reaction mixture was stirred at ambient temperature. After 1 h, Silicycle SiliaMetS^{®} DMT metal scavenger (1.0 g) was introduced and the mixture was diluted with dichloromethane, filtered over Celite^{®}, and the Celite^{®} was washed with ethyl acetate. The filtrate was concentrated under reduced pressure to afford (P)-1-(5-chloro-2-methoxy-4-((trifluoromethyl)thio)phenyl)-N-(isoxazol-3-yl)-N-(4-methoxybenzyl)-2-oxo-1,2-dihydroquinoline-6-sulfonamide which was used in the next step without further purification. m/z (ESI) 653.6 (M+H)⁺.

Step 2: A 40 mL vial was charged with (P)-1-(5-chloro-2-methoxy-4-((trifluoromethyl)thio)phenyl)-N-(isoxazol-3-yl)-N-(4-methoxybenzyl)-2-oxo-1,2-dihydroquinoline-6-sulfonamide, triethylsilane (759 mg, 1.06 mL, 6.53 mmol) and trifluoroacetic acid (6.53 mL). The reaction mixture was stirred at ambient temperature. After 16 h, the solvent was removed under reduced pressure. The residue was purified via reverse phase HPLC to give (P)-1-(5-chloro-2-methoxy-4-((trifluoromethyl)thio)phenyl)-N-(isoxazol-3-yl)-2-oxo-1,2-dihydroquinoline-6-sulfonamide as an orange solid (323 mg, 47% overall yield). ¹H NMR (500 MHz, DMSO-*d*₆) δ 8.70 (s, 1 H), 8.37 (d, *J*=2.1 Hz, 1 H), 8.23 (d, *J*=9.7 Hz, 1 H), 7.92 (s, 1 H), 7.86 (dd, *J*=9.0, 2.2 Hz, 1 H), 7.78 (s, 1 H), 6.80 (dd, *J*=9.2, 7.4 Hz, 2 H), 6.43 (d, *J*=1.7 Hz, 1 H), 3.75 (s, 3 H). m/z (ESI) 532.8 (M+H)⁺.

### Example 3

### (P)-1-(5-CHLORO-2-METHOXY-4-(TRIFLUOROMETHOXY)PHENYL)-N-(ISOXAZOL-3-YL)-2-OXO-1,2-DIHYDROQUINOLINE-6-SULFONAMIDE

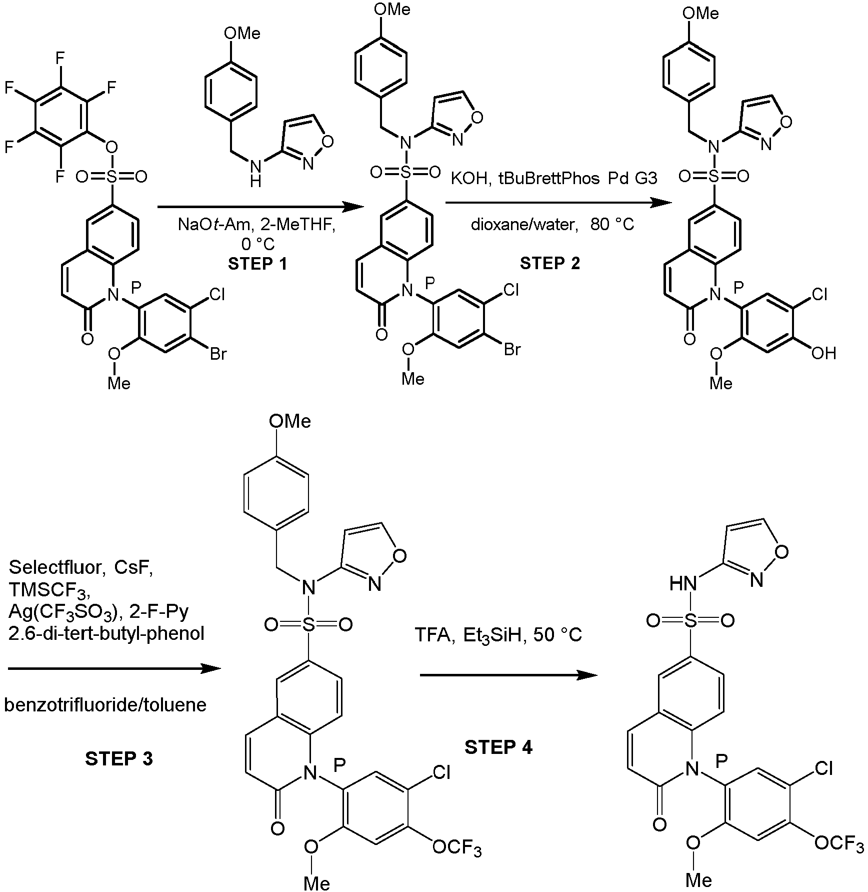

Step 1: A 250 mL three-neck round-bottom flask equipped with a Claisen adapter, nitrogen inlet, thermocouple, overhead stirrer and addition funnel was charged with (P)perfluorophenyl-1-(4-bromo-5-chloro-2-methoxyphenyl)-2-oxo-1,2-dihydroquinoline-6-sulfonate (20.0 g, 32.7 mmol) and N-(4-methoxybenzyl)isoxazol-3-amine (7.36 g, 36.0 mmol). The flask was evacuated and back-filled with nitrogen three times, before 2-methyltetrahydrofuran (99.0 mL) was introduced and the resultant solution was cooled to -0.8 °C. A 1.4-M solution of sodium tert-pentoxide in THF (28.1 ml, 39.3 mmol) was transferred to the addition funnel and then added dropwise at such a rate that the internal reaction temperature did not exceed 3 °C. After the addition was complete, the solution was siphoned into another chilled vessel containing 100 mL 2.0 N aqueous HCl and 100 mL EtOAc. The layers were separated and the aqueous layer was extracted with EtOAc (2x). The combined organic layers were dried over anhydrous magnesium sulfate, filtered, and concentrated under reduced pressure. The residue was diluted with IPA (500 mL), causing the formation of a white solid precipitate. The mixture was stirred overnight and the solids were isolated by filtration. The isolated product was washed once with IPA and dried to afford (P)-1-(4-bromo-5-chloro-2-methoxyphenyl)-N-(isoxazol-3-yl)-N-(4-methoxybenzyl)-2-oxo-1,2-dihydroquinoline-6-sulfonamide (16.5 g, 26.2 mmol, 80 % yield) as an off-white solid. m/z (ESI) 631.4 (M+H)⁺.

Step 2: To a vial containing [(2-di-tert-butylphosphino-3,6-dimethoxy-2',4',6'-triisopropyl-1,1'-biphenyl)-2-(2'-amino-1,1'-biphenyl)]palladium(ii) methanesulfonate (278 mg, 0.33 mmol) and 1-(4-bromo-5-chloro-2-methoxyphenyl)-N-(isoxazol-3-yl)-N-(4-methoxybenzyl)-2-oxo-1,2-dihydroquinoline-6-sulfonamide (2.05 g, 3.25 mmol) was added potassium hydroxide (548 mg, 9.76 mmol), 1,4-dioxane (6.00 mL), and water (2.00 mL). The reaction mixture was stirred at 80 °C. After 2 hours, the reaction mixture was partitioned between DCM and water. The layers were separated and the aqueous layer was extracted with DCM (2x). The combined organic layers were dried over anhydrous magnesium sulfate, filtered, and concentrated under reduced pressure. The residue was purified by flash chromatography (silica gel, eluent: 0-60% ethyl acetate in heptane) to provide (P)-1-(5-chloro-4-hydroxy-2-methoxyphenyl)-N-(isoxazol-3-yl)-N-(4-methoxybenzyl)-2-oxo-1,2-dihydroquinoline-6-sulfonamide (1.52 g, 2.67 mmol, 82 % yield). m/z (ESI) 568.0 (M+H)⁺.

Step 3: A 40 mL vial was charged with silver(I) trifluoromethanesulfonate (1.81 g, 7.04 mmol), 1-(chloromethyl)-4-fluoro-1,4-diazabicyclo[2.2.2]octane-1,4-diium tetrafluoroborate (998 mg, 2.82 mmol), N-fluoro-N-(phenylsulfonyl)benzenesulfonamide (888 mg, 2.82 mmol), cesium fluoride (1.28 g, 8.45 mmol), 2,6-di-tert-butylphenol (581 mg, 2.82 mmol), 1-(5-chloro-4-hydroxy-2-methoxyphenyl)-N-(isoxazol-3-yl)-N-(4-methoxybenzyl)-2-oxo-1,2-dihydroquinoline-6-sulfonamide (800 mg, 1.41 mmol) in a nitrogen-filled glovebox. To the vial was removed from the glovebox before toluene (7.1 mL), benzotrifluoride (14.2 mL), 2-fluoropyridine (684 mg, 606 µL, 7.04 mmol), and trimethyl(trifluoromethyl)silane (1.00 g, 1.04 mL, 7.04 mmol) successively introduced under an argon atmosphere. The resultant reaction mixture was stirred at ambient temperature for 36 hours. After this time, the reaction mixture was filtered through a plug of silica gel and eluted with ethyl acetate. The filtrate was concentrated under reduced pressure and the residue was purified by column chromatography (silica gel, eluent: 0-40% EtOAc/EtOH (3:1) in heptane with 10% dichloromethane as an additive) to afford (P)-1-(5-chloro-2-methoxy-4-(trifluoromethoxy)phenyl)-N-(isoxazol-3-yl)-N-(4-methoxybenzyl)-2-oxo-1,2-dihydroquinoline-6-sulfonamide (254 mg, 0.40 mmol, 28.4 % yield). m/z (ESI) 635.8 (M+H)⁺.

Step 4: A solution of (P)-1-(5-chloro-2-methoxy-4-(trifluoromethoxy)phenyl)-N-(isoxazol-3-yl)-N-(4-methoxybenzyl)-2-oxo-1,2-dihydroquinoline-6-sulfonamide (254 mg, 0.40 mmol) and triethylsilane (203 mg, 280 µL, 1.75 mmol) in TFA (1.18 mL) was stirred at 50 °C for 2 hours. The reaction mixture was then concentrated under reduced pressure and the residue was purified by column chromatography (silica gel, eluent: 0-40% EtOAc/EtOH (3:1) in heptane with 10% dichloromethane as an additive) to afford (P)-1-(5-chloro-2-methoxy-4-(trifluoromethoxy)phenyl)-N-(isoxazol-3-yl)-2-oxo-1,2-dihydroquinoline-6-sulfonamide (122 mg, 0.237 mmol, 16.8 % yield). ¹H NMR (500 MHz, methanol-*d*₄) δ 8.44 (d, *J*=1.69 Hz, 1 H), 8.32 (d, *J*=2.08 Hz, 1 H), 8.12 (d, *J*=9.60 Hz, 1 H), 7.92 (dd, *J*=8.95, 2.21 Hz, 1 H), 7.62 (s, 1 H), 7.35 - 7.38 (m, 1 H), 6.86 (d, *J*=8.95 Hz, 1 H), 6.83 (d, *J*=9.73 Hz, 1 H), 6.48 (d, *J*=1.82 Hz, 1 H), 3.77 (s, 3 H). m/z (ESI) 515.8 (M+H)⁺.

### Example 4

### (P)-1-(5-FLUORO-2-METHOXY-4-((TRIFLUOROMETHOXY) METHYL)PHENYL)-N-(ISOXAZOL-3-YL)-2-OXO-1,2-DIHYDROQUINOLINE-6-SULFONAMIDE

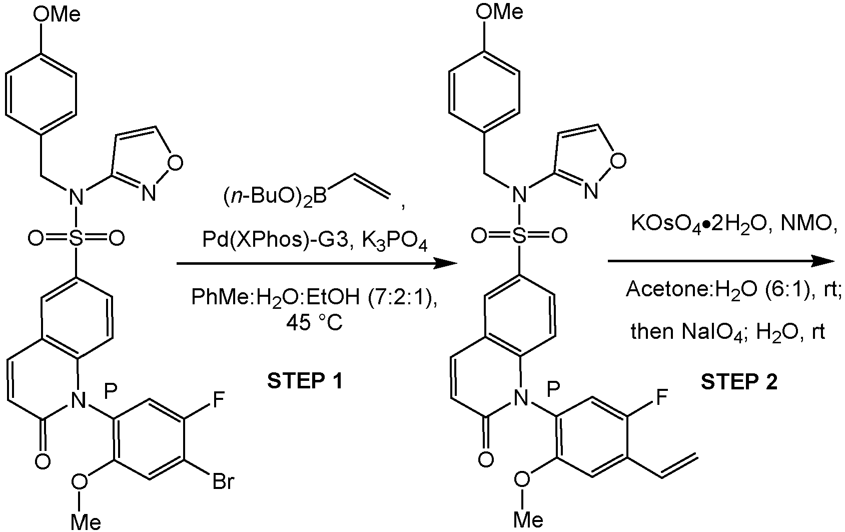

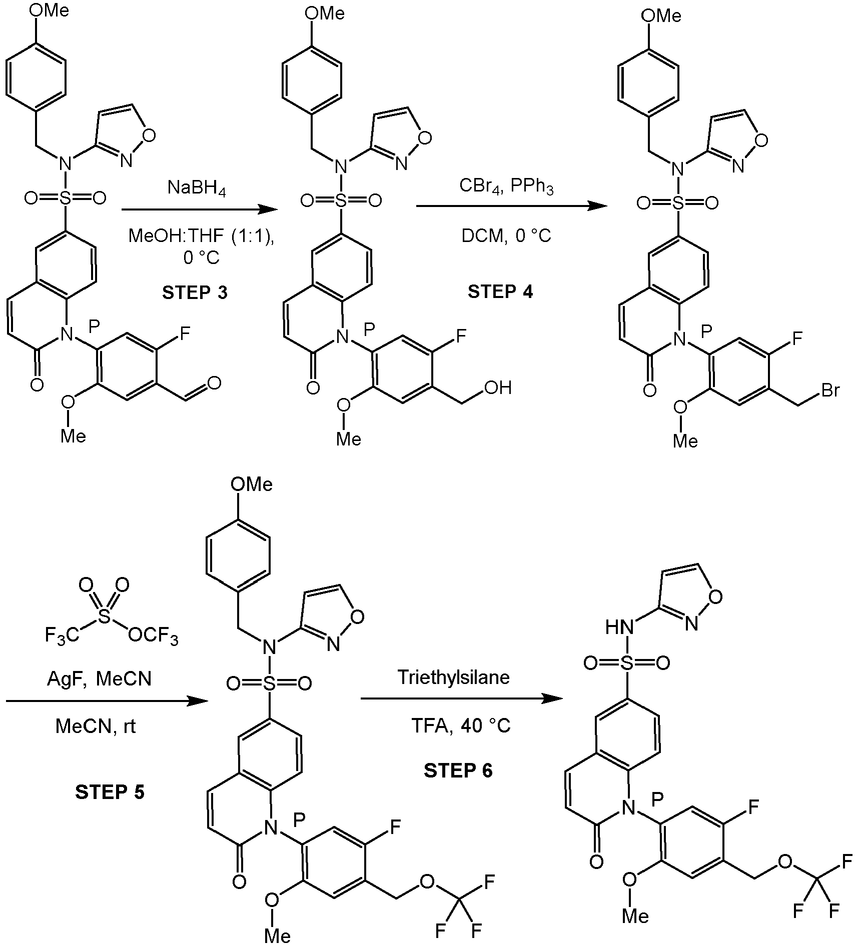

Step 1: A 1 L round-bottom flask was charged with (P)-1-(4-bromo-5-fluoro-2-methoxyphenyl)-N-(isoxazol-3-yl)-N-(4-methoxybenzyl)-2-oxo-1,2-dihydroquinoline-6-sulfonamide (40.0 g, 65.1 mmol), methanesulfonato(2-dicyclohexylphosphino-2',4',6'-tri-i-propyl-1,1'-biphenyl)(2'-amino-1,1'-biphenyl-2-yl)palladium(II) (5.51 g, 6.51 mmol), and potassium phosphate tribasic (41.5 g, 195 mmol). Vinylboronic acid di-n-butyl ester (24.0 g, 28.7 mL, 130 mmol) was added, followed by ethanol (65.1 mL), water (130 mL), and toluene (456 mL). The flasked purged with nitrogen for 10 minutes and the reaction mixture was warmed to 45 °C. After 20 hours, the reaction mixture was cooled to room temperature and was diluted with water. The layers were separated and the aqueous layer was extracted EtOAc (3x). The combined organic layers were washed with brine, dried over sodium sulfate, filtered, and concentrated under reduced pressure. The residue was purified by flash column chromatography (Biotage, 340 g Silica Cartridge, eluent: 0-65% ethyl acetate in heptane with 10% DCM additive) to afford (P)-1-(5-fluoro-2-methoxy-4-vinylphenyl)-N-(isoxazol-3-yl)-N-(4-methoxybenzyl)-2-oxo-1,2-dihydroquinoline-6-sulfonamide (35.4 g, 63.0 mmol, 97 % yield) as a tan solid. ¹H NMR (500 MHz, CDCl₃) δ: 8.23 (d, 1 H, *J*=1.82 Hz), 7.83 (d, 1 H, *J*=2.08 Hz), 7.69 (d, 1 H, *J*=9.73 Hz), 7.56 (dd, 1 H, *J*=9.02, 2.14 Hz), 7.36 (d, 2 H, *J*=8.69 Hz), 7.19 (d, 1 H, *J*=6.36 Hz), 6.87 - 7.00 (m, 2 H), 6.83 (d, 1 H, *J*=9.60 Hz), 6.75 - 6.81 (m, 2 H), 6.68 - 6.75 (m, 2 H), 5.92 (d, 1 H, *J=* 17.65 Hz), 5.52 (d, 1 H, *J*=11.16 Hz), 4.95 (s, 2 H), 3.75 (d, 6 H, *J*=5.32 Hz). m/z (ESI) 562.2 (M+H)⁺.

Step 2: A 1 L round-bottom flask was charged with (P)-1-(5-fluoro-2-methoxy-4-vinylphenyl)-N-(isoxazol-3-yl)-N-(4-methoxybenzyl)-2-oxo-1,2-dihydroquinoline-6-sulfonamide (14.0 g, 24.9 mmol), water (35.6 mL), and acetone (214 mL). 4-Methylmorpholine 4-oxide (4.38 g, 37.4 mmol) and potassium dioxidodioxoosmium dihydrate (0.092 g, 0.249 mmol) were introduced and the resultant reaction mixture stirred at room temperature. After 21 h, a solution of sodium periodate (10.7 g, 49.8 mmol) in water (250 mL) was added to the reaction mixture. After 20 minutes, additional water was added (~500 mL) and the solid precipitate was collected by filtration. The solid was washed with water and dried under vacuum to afford (P)-1-(5-fluoro-4-formyl-2-methoxyphenyl)-N-(isoxazol-3-yl)-N-(4-methoxybenzyl)-2-oxo-1,2-dihydroquinoline-6-sulfonamide (13.8 g, 24.4 mmol, 98 % yield) as an off-white solid, which was used without further purification. ¹H NMR (500 MHz, CDCl₃) δ: 10.44 (s, 1H), 8.24 (d, 1H, *J*=1.82 Hz), 7.84 (d, 1H, *J*=2.08 Hz), 7.72 (d, 1H, *J*=9.73 Hz), 7.52-7.61 (m, 2H), 7.32-7.39 (m, 2H), 7.16 (d, 1H, *J*=9.21 Hz), 6.83 (d, 1H, *J*=9.60 Hz), 6.74-6.81 (m, 2H), 6.71 (d, 1H, *J*=1.69 Hz), 6.63 (d, 1H, *J*=8.95 Hz), 4.95 (s, 2H), 3.79 (s, 3H), 3.77 (s, 3H). m/z (ESI) 564.2 (M+H)⁺.

Step 3: A 1 L round-bottom flask was charged with (P)-1-(5-fluoro-4-formyl-2-methoxyphenyl)-N-(isoxazol-3-yl)-N-(4-methoxybenzyl)-2-oxo-1,2-dihydroquinoline-6-sulfonamide (24.4 g, 43.2 mmol), methanol (216 mL), and tetrahydrofuran (216 mL) then cooled to 0 °C before sodium borohydride was added portionwise over 5 minutes (1.72 g, 45.4 mmol). Following addition, the reaction mixture stirred for 10 minutes before an aqueous solution of ammonium chloride (5 M) was introduced. The resultant mixture was extracted with EtOAc (3x). The combined organic layers were washed with an aqueous solution of sodium chloride (5 M), dried over anhydrous sodium sulfate, filtered, and concentrated under reduced pressure to afford (P)-1-(5-fluoro-4-(hydroxymethyl)-2-methoxyphenyl)-N-(isoxazol-3-yl)-N-(4-methoxybenzyl)-2-oxo-1,2-dihydroquinoline-6-sulfonamide (23.3 g, 41.2 mmol, 95 % yield) as a white solid, which was used without further purification. ¹H NMR (500 MHz, DMSO-*d*₆) δ: 8.79 (s, 1 H), 8.35 (s, 1 H), 8.14 (d, 1 H, *J*=9.7 Hz), 7.78 (br d, 1 H, *J*=9.1 Hz), 7.37 (br d, 1 H, *J*=6.2 Hz), 7.21 - 7.33 (m, 3 H), 6.79 - 6.88 (m, 3 H), 6.72 (s, 1 H), 6.72 (d, 1 H, *J*=6.3 Hz), 5.47 (t, 1 H, *J*=5.4 Hz), 4.92 (s, 2 H), 4.60 - 4.69 (m, 2 H), 3.72 (s, 3 H), 3.70 (s, 3 H), 3.30 (s, 1 H). m/z (ESI) 566.2 (M+H)⁺.

Step 4: A 250 mL round-bottom flask was charged with (P)-1-(5-fluoro-4-(hydroxymethyl)-2-methoxyphenyl)-N-(isoxazol-3-yl)-N-(4-methoxybenzyl)-2-oxo-1,2-dihydroquinoline-6-sulfonamide (8.05 g, 14.2 mmol) and carbon tetrabromide (5.66 g, 17.1 mmol). Dichloromethane (71.2 mL) was then added and the reaction mixture was cooled to 0 °C before triphenylphosphine (4.85 g, 18.5 mmol) was introduced in one portion. After 20 minutes, water was introduced and the mixture was extracted with DCM. The organic layer was dried over anhydrous magnesium sulfate, filtered, concentrated under reduced pressure, and purified by flash column chromatography (Biotage, 100 g Silica Cartridge, 100% heptane for 1 column volume, then 0-45% ethyl acetate in heptane with 10% dichloromethane additive for 1 column volume, then 45-65% ethyl acetate in heptane with 10% dichloromethane additive for 1 column volume) to afford (P)-1-(4-(bromomethyl)-5-fluoro-2-methoxyphenyl)-N-(isoxazol-3-yl)-N-(4-methoxybenzyl)-2-oxo-1,2-dihydroquinoline-6-sulfonamide as a white solid (7.20 g, 11.5 mmol, 80% yield). ¹H NMR (500 MHz, CDCl₃) δ: 8.25 (d, 1 H, *J*=1.8 Hz), 7.84 (d, 1 H, *J*=2.2 Hz), 7.71 (d, 1 H, *J*=9.6 Hz), 7.58 (dd, 1 H, *J*=9.0, 2.2 Hz), 7.35 - 7.40 (m, 2 H), 7.16 (d, 1 H, *J*=6.4 Hz), 7.01 (d, 1 H, *J*=8.8 Hz), 6.77 - 6.86 (m, 3 H), 6.68 - 6.75 (m, 2 H), 4.96 (s, 2 H), 4.65 (d, 1 H, *J*=10.5 Hz), 4.53 (d, 1 H, *J*=10.6 Hz), 3.78 (s, 3 H), 3.77 (s, 3 H). m/z (ESI) 629.8 (M+H)⁺.

Step 5: Trifluoromethyl triflate (10.2 g, 10.2 mL, 46.8 mmol) was rapidly transferred to a 250 mL three-neck round-bottom flask and the reaction vessel was immediately cooled in a dry ice-acetone bath. The flask was then connected via teflon tubing to an oil bubbler, the outlet of which was connected to an aqueous solution of potassium hydroxide (6 M). A solid addition funnel charged with silver(I) fluoride (6.05 g, 47.7 mmol) was then attached to the reaction vessel. The reaction vessel was then purged with nitrogen for 5 minutes. Acetonitrile (90 mL) was introduced and the resultant biphasic mixture warmed to 0 °C in ice-water bath. Silver(I) fluoride (6.05 g, 47.7 mmol) was then added portionwise to the reaction mixture at a rate that controlled gas evolution. Following addition, the reaction mixture stirred for 30 minutes to afford a solution of AgOCF₃ that was used without further manipulation. A separate 500 mL round-bottom flask was charged with (P)-1-(4-(bromomethyl)-5-fluoro-2-methoxyphenyl)-N-(isoxazol-3-yl)-N-(4-methoxybenzyl)-2-oxo-1,2-dihydroquinoline-6-sulfonamide (8.90 g, 14.2 mmol), purged with nitrogen for 5 min, and was charged with acetonitrile (70.8 mL). The previously prepared solution of AgOCF₃ was then transferred dropwise via cannula into the stirred reaction mixture. Following addition, the nitrogen inlet line was removed and the reaction stirred at ambient temperature. After 19 hours, a saturated aqueous solution of sodium bicarbonate was introduced and the resultant mixture was diluted with dichloromethane. The resulting biphasic mixture was filtered, the organic layer was separated via an Isolute^{®} phase separator, concentrated under reduced pressure, and purified by flash column chromatography (Biotage, 350 g Silica Cartridge, eluent: 100% heptane for 1 column volume, 0-40% ethyl acetate in heptane with 10% dichloromethane additive for 0.5 column volume, then 40-65% ethyl acetate in heptane with 10% dichloromethane additive for 8 column volumes) to afford (P)-1-(5-fluoro-2-methoxy-4-((trifluoromethoxy)methyl)phenyl)-N-(isoxazol-3-yl)-N-(4-methoxybenzyl)-2-oxo-1,2-dihydroquinoline-6-sulfonamide (7.83 g, 12.4 mmol, 87 % yield) as a white solid. ¹H NMR (500 MHz, CDCl₃) δ: 8.24 (d, 1 H, *J=*1.8 Hz), 7.84 (d, 1 H, *J*=2.2 Hz), 7.71 (d, 1 H, *J=*9.6 Hz), 7.57 (dd, 1 H, *J*=9.0, 2.1 Hz), 7.36 (d, 2 H, *J*=7.4 Hz), 7.17 (d, 1 H, *J*=6.1 Hz), 7.04 (d, 1 H, *J*=9.0 Hz), 6.76 - 6.85 (m, 3 H), 6.72 (d, 1 H, *J*=1.8 Hz), 6.67 (d, 1 H, *J*=9.0 Hz), 5.22 (d, 1 H, *J*=12.2 Hz), 5.10 (d, 1 H, *J*=12.2 Hz), 4.95 (s, 2 H), 3.77 (s, 6 H). m/z (ESI) 634.0 (M+H)⁺.

Step 6: A 100 mL round-bottom flask was charged with (P)-1-(5-fluoro-2-methoxy-4-((trifluoromethoxy)methyl)phenyl)-N-(isoxazol-3-yl)-N-(4-methoxybenzyl)-2-oxo-1,2-dihydroquinoline-6-sulfonamide (6.0 g, 9.47 mmol) and triethylsilane (5.51 g, 7.56 mL, 47.4 mmol). 1,1,1-Trifluoroacetic acid (32.4 g, 21.9 mL, 284 mmol) was added dropwise to the reaction mixture at ambient temperature. The resultant reaction mixture was warmed to 40 °C. After 8 h, the reaction mixture was cooled to ambient temperature and concentrated under reduced pressure. The residue was purified by flash column chromatography (Biotage, 100 g Silica Cartridge, elutant: 0-20% EtOAc in DCM) to afford (P)-1-(5-fluoro-2-methoxy-4-((trifluoromethoxy)methyl)phenyl)-N-(isoxazol-3-yl)-2-oxo-1,2-dihydroquinoline-6-sulfonamide (4.00 g, 7.79 mmol, 82% yield) as off white solid. ¹H NMR (500 MHz, DMSO-d₆) δ:11.63 (s, 1 H), 8.73 (d, 1 H, *J*=1.8 Hz), 8.37 (d, 1 H, *J=*2.2 Hz), 8.22 (d, 1 H, *J*=9.6 Hz), 7.85 (dd, 1 H, *J*=9.0, 2.2 Hz), 7.54 (d, 1 H, *J*=6.4 Hz), 7.50 (d, 1 H, *J*=9.5 Hz), 6.79 (dd, 2H, J=9.3, 4.7 Hz), 6.44 (d, 1 H, *J*=1.8 Hz), 5.27 - 5.34 (m, 2 H), 3.69 (s, 3 H). m/z (ESI) 514.0 (M+H)⁺.

### Example 5

### (P)-1-(5-FLUORO-2-METHOXY-4-((2,2,2-TRIFLUOROETHYL)THIO)PHENYL)-N-(ISOXAZOL-3-YL)-2-OXO-1,2-DIHYDROQUINOLINE-6-SULFONAMIDE

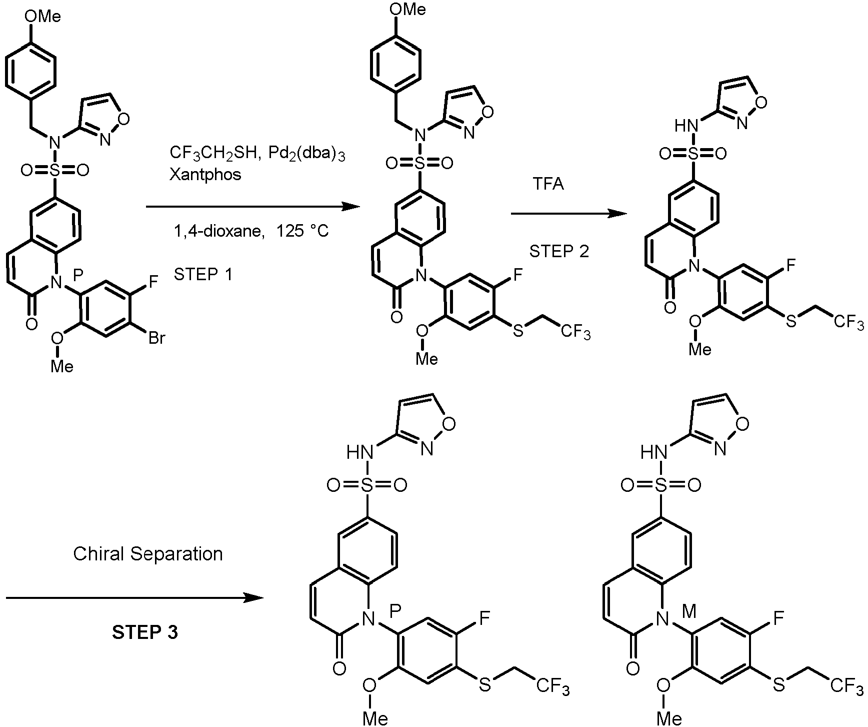

Step 1: A microwave vial was charged with 1-(4-bromo-5-fluoro-2-methoxyphenyl)-N-(isoxazol-3-yl)-N-(4-methoxybenzyl)-2-oxo-1,2-dihydroquinoline-6-sulfonamide (1.00 g, 1.63 mmol), tris(dibenzylideneacetone)dipalladium(0) (59.6 mg, 0.07 mmol), 4,5-bis(diphenylphosphino)-9,9-dimethylxanthene (75.4 mg, 0.13 mmol), and 1,4-dioxane (4.00 mL). The reaction mixture was sparged with nitrogen before diisopropylethylamine (420 mg, 566 µL, 3.25 mmol) and 2,2,2-trifluoroethane-1-thiol (226 mg, 174 µL, 1.95 mmol) were introduced. The reaction mixture was subsequently sparged with nitrogen, capped, and irradiated for 1 h at 125 °C. The reaction mixture was then filtered through a pad of Celite^{®} and the filter cake was rinsed with ethyl acetate. The filtrate was partitioned between ethyl acetate and water; the aqueous layer was extracted twice with ethyl acetate. The combined organic layers were washed with brine, dried over anhydrous sodium sulfate, filtered, and concentrated under reduced pressure. The residue was purified by flash column chromatography (silica gel, eluent: 0-30% ethyl acetate/EtOH (3:1) in heptane) to afford racemic 1-(5-fluoro-2-methoxy-4-((2,2,2-trifluoroethyl)thio)phenyl)-N-(isoxazol-3-yl)-N-(4-methoxybenzyl)-2-oxo-1,2-dihydroquinoline-6-sulfonamide (625 mg, 0.96 mmol, 59% yield) as a pale yellow solid. m/z (ESI) 649.8 (M+H)⁺.

Step 2: A solution of 1-(5-fluoro-2-methoxy-4-((2,2,2-trifluoroethyl)thio)phenyl)-N-(isoxazol-3-yl)-N-(4-methoxybenzyl)-2-oxo-1,2-dihydroquinoline-6-sulfonamide (625 mg, 0.96 mmol) and trifluoroacetic acid (2.0 mL) was stirred at ambient temperature. After 16 hours, the reaction mixture was concentrated under reduced pressure to provide 1-(5-fluoro-2-methoxy-4-((2,2,2-trifluoroethyl)thio)phenyl)-N-(isoxazol-3-yl)-2-oxo-1,2-dihydroquinoline-6-sulfonamide as a mixture of atropisomers.

Step 3: Racemic 1-(5-fluoro-2-methoxy-4-((2,2,2-trifluoroethyl)thio)phenyl)-N-(isoxazol-3-yl)-2-oxo-1,2-dihydroquinoline-6-sulfonamide was separated by chiral SFC chromatography, via a Regis Whelk-O S,S, 3 x 15 cm, 5 µm column, a mobile phase of 35% 1:1 methanol/DCM, and a flowrate of 160 mL/min. The first-eluting peak was concentrated to afford (P)-1-(5-fluoro-2-methoxy-4-((2,2,2-trifluoroethyl)thio)phenyl)-N-(isoxazol-3-yl)-2-oxo-1,2-dihydroquinoline-6-sulfonamide (248
mg, 0.47 mmol, 48.6% yield over two steps) as a white solid; the second-eluting peak was separately concentrated to afford the (M) atropisomer. ¹H NMR (400 MHz, DMSO-*d*₆) δ: 11.64 (s, 1 H), 8.72 (d, *J*=1.76 Hz, 1 H), 8.36 (d, *J*=2.18 Hz, 1 H), 8.21 (d, *J*=9.54 Hz, 1 H), 7.84 (dd, *J*=8.91, 2.28 Hz, 1 H), 7.44 - 7.51 (m, 2 H), 6.79 (dd, *J*=9.28, 2.02 Hz, 2 H), 6.44 (d, *J*=1.76 Hz, 1 H), 4.15 - 4.27 (m, 2 H), 3.70 (s, 3 H). m/z (ESI) 559.8 (M+H)+.

### Example 6

### (P)-7-FLUORO-1-(5-FLUORO-2-METHOXY-4-((TRIFLUOROMETHOXY)METHYL)PHENYL)-N-(ISOXAZOL-3-YL)-2-OXO-1,2-DIHYDROQUINOLINE-6-SULFONAMIDE

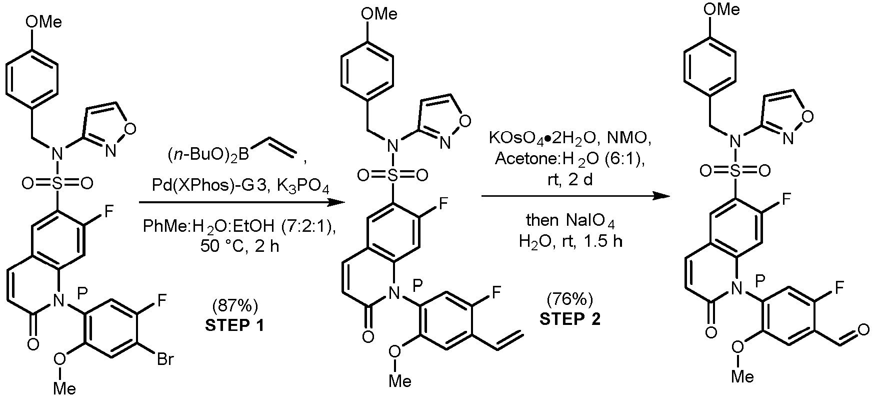

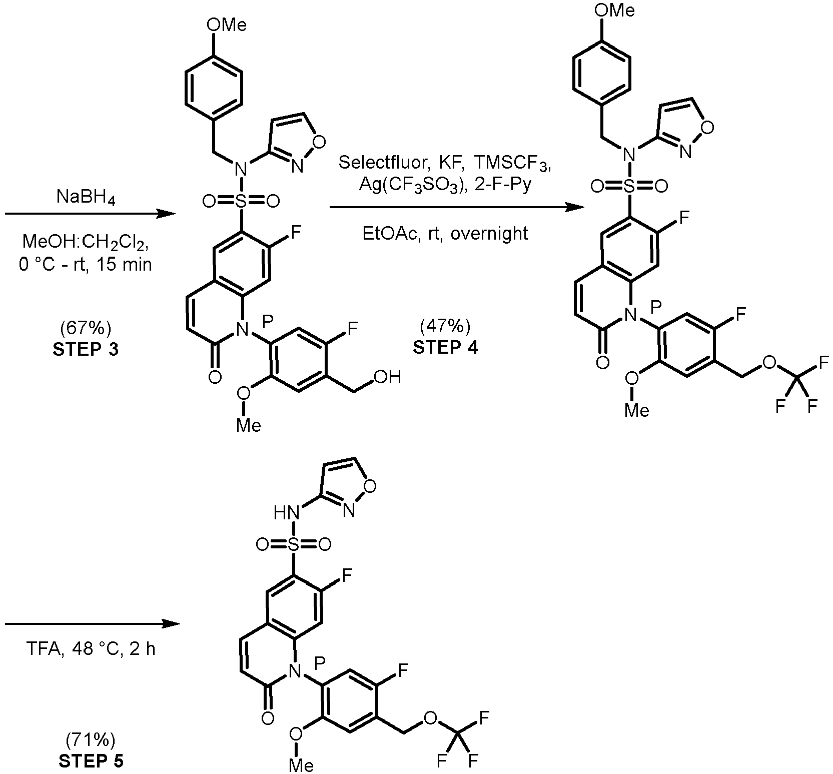

Step 1: A 100 mL round-bottom flask was charged with (P)-1-(4-bromo-5-fluoro-2-methoxyphenyl)-7-fluoro-N-(isoxazol-3-yl)-N-(4-methoxybenzyl)-2-oxo-1,2-dihydroquinoline-6-sulfonamide (2.00 g, 3.16 mmol), (2-dicyclohexylphosphino-2',4',6'-triisopropyl-1,1'-biphenyl)[2-(2'-amino-1,1'-biphenyl)]palladium(ii) methanesulfonate (0.402 g, 0.474 mmol), potassium phosphate (2.01 g, 9.49 mmol), dibutyl vinylboronate (1.164 g, 1.39 mL, 6.32 mmol), ethanol (3.16 mL), water (6.32 mL), and toluene (22.1 mL). The reaction mixture was stirred at 50 °C for 2 hours. After this time, the reaction mixture was diluted with water and extracted with EtOAc (3x). The combined organic layers were washed with brine and dried over anhydrous sodium sulfate, filtered, and concentrated under reduced pressure. The residue was purified by flash chromatography using a gradient of 0-40% ethyl acetate/EtOH (3:1) in heptane to afford (P)-7-fluoro-1-(5-fluoro-2-methoxy-4-vinylphenyl)-N-(isoxazol-3-yl)-N-(4-methoxybenzyl)-2-oxo-1,2-dihydroquinoline-6-sulfonamide (1.60 g, 2.76 mmol, 87 % yield). m/z (ESI) 580.8 (M+H)+.

Step 2: A 40 mL vial was charged with (P)-7-fluoro-1-(5-fluoro-2-methoxy-4-vinylphenyl)-N-(isoxazol-3-yl)-N-(4-methoxybenzyl)-2-oxo-1,2-dihydroquinoline-6-sulfonamide (570 mg, 0.98 mmol), N-methylmorpholine N-oxide (NMO) (173 mg, 1.48 mmol), and potassium osmate dihydrate (1.62 mg, 4.92 µmol). The resultant reaction mixture was stirred at room temperature for 48 hours. After this time, the solution was then treated with a solution of sodium periodate (421 mg, 1.97 mmol) in water (15.8 mL). The resultant reaction mixture was stirred at room temperature for 1.5 hours. The reaction mixture was then diluted with dichloromethane, the layers were separated, and the aqueous layer was further extracted with dichloromethane. The combined organic layers were dried over anhydrous sodium sulfate, filtered, and concentrated under reduced pressure. The residue was purified by column chromatography (RediSep 40 g, 3-50% EtOAc/EtOH (3:1) in heptane with 10% dichloromethane as an additive) to afford (P)-7-fluoro-1-(5-fluoro-4-formyl-2-methoxyphenyl)-N-(isoxazol-3-yl)-N-(4-methoxybenzyl)-2-oxo-1,2-dihydroquinoline-6-sulfonamide (0.435 g, 0.748 mmol, 76 % yield) as a yellow solid. m/z (ESI) 582.8 (M+H)+.

Step 3: A vial containing (P)-7-fluoro-1-(5-fluoro-4-formyl-2-methoxyphenyl)-N-(isoxazol-3-yl)-N-(4-methoxybenzyl)-2-oxo-1,2-dihydroquinoline-6-sulfonamide (785 mg, 1.35 mmol), methanol (6.75 mL), and dichloromethane (5 mL) was cooled to 0 °C before sodium tetrahydroborate (51.1 mg, 1.35 mmol) was added in a portionwise fasion to the stirred reaction mixture. The resultant reaction mixture was stirred for 5 min a 0 °C and then 10 min at ambient temperature. The reaction mixture was quenched with water, extracted with dichloromethane, washed with brine, dried over sodium sulfate and concentrated under reduced pressure. The residue was purified by column chromatography (RediSep 24 g, 0-50% EtOAc/EtOH (3:1) in heptane with 10% dichloromethane as an additive) to afford (P)-7-fluoro-1-(5-fluoro-4-(hydroxymethyl)-2-methoxyphenyl)-N-(isoxazol-3-yl)-N-(4-methoxybenzyl)-2-oxo-1,2-dihydroquinoline-6-sulfonamide (524 mg, 0.897 mmol, 67 % yield) as a white solid. m/z (ESI) 584.8 (M+H)+.

Step 4: A 40 mL vial was charged with (P)-7-fluoro-1-(5-fluoro-4-(hydroxymethyl)-2-methoxyphenyl)-N-(isoxazol-3-yl)-N-(4-methoxybenzyl)-2-oxo-1,2-dihydroquinoline-6-sulfonamide (524 mg, 0.897 mmol) and 1-(chloromethyl)-4-fluoro-1,4-diazabicyclo[2.2.2]octane-1,4-diium tetrafluoroborate (477 mg, 1.35 mmol). The vial was transferred into a glovebox before potassium fluoride (209 mg, 3.59 mmol) and silver(I) trifluoromethanesulfonate (692 mg, 2.69 mmol) was subsequently added. The vial was then capped and removed from the glovebox before ethyl acetate (4.49 mL), 2-fluoropyridine (261 mg, 232 µL, 2.69 mmol) and trimethyl(trifluoromethyl)silane (383 mg, 398 µL, 2.69 mmol) were introduced. The resultant reaction mixture was stirred at room temperature for 16 h. After this time, ethyl acetate (10 mL) was added and the mixture was passed through a plug of silica gel. The silica gel plug was washed with ethyl acetate (20 mL). The filtrate was washed with brine, dried over anhydrous sodium sulfate, filtered, and concentrated under reduced pressure. The residue was purified via column chromatography (RediSep 24 g, 2-40% EtOAc/EtOH (3:1) in heptane with 10% dichloromethane as an additive) to afford (P)-7-fluoro-1-(5-fluoro-2-methoxy-4-((trifluoromethoxy)methyl)phenyl)-N-(isoxazol-3-yl)-N-(4-methoxybenzyl)-2-oxo-1,2-dihydroquinoline-6-sulfonamide (274 mg, 0.421 mmol, 47 % yield) as a white solid. m/z (ESI) 652.8 (M+H)+.

Step 5: A 20-mL vial was charged with (P)-7-fluoro-1-(5-fluoro-2-methoxy-4-((trifluoromethoxy)methyl)phenyl)-N-(isoxazol-3-yl)-N-(4-methoxybenzyl)-2-oxo-1,2-dihydroquinoline-6-sulfonamide (274 mg, 0.421 mmol) and trifluoroacetic acid (4.0 mL). The reaction was stirred at 48 °C for 2 hours. The mixture was concentrated under reduced pressure. The residue was purified via column chromatography (RediSep 12 g, 2-45% EtOAc/EtOH (3:1) in heptane with 10% dichloromethane as an additive) to afford (P)- 7-fluoro-1-(5-fluoro-2-methoxy-4-((trifluoromethoxy)methyl)phenyl)-N-(isoxazol-3-yl)-2-oxo-1,2-dihydroquinoline-6-sulfonamide (158 mg, 0.297 mmol, 71 % yield) as an off-white solid. ¹H NMR (CDCl₃, 500 MHz) δ 8.75 (br s, 1H), 8.24 (d, *J*=1.4 Hz, 1H), 8.1-8.2 (m, 1H), 7.79 (d, *J*=9.7 Hz, 1H), 7.18 (d, *J*=6.1 Hz, 1H), 7.03 (d, *J*=8.8 Hz, 1H), 6.81 (d, *J*=9.7 Hz, 1H), 6.55 (d, *J*=1.6 Hz, 1H), 6.4-6.5 (m, 1H), 5.1-5.3 (m, 2H), 3.7-3.8 (m, 3H). m/z (ESI) 532.8 (M+H)+

### Example 7

### (P)-7-FLUORO-1-(5-FLUORO-2-METHOXY-4-((TRIFLUOROMETHYL)THIO)PHENYL)-N-(ISOXAZOL-3-YL)-2-OXO-1,2-DIHYDROQUINOLINE-6-SULFONAMIDE

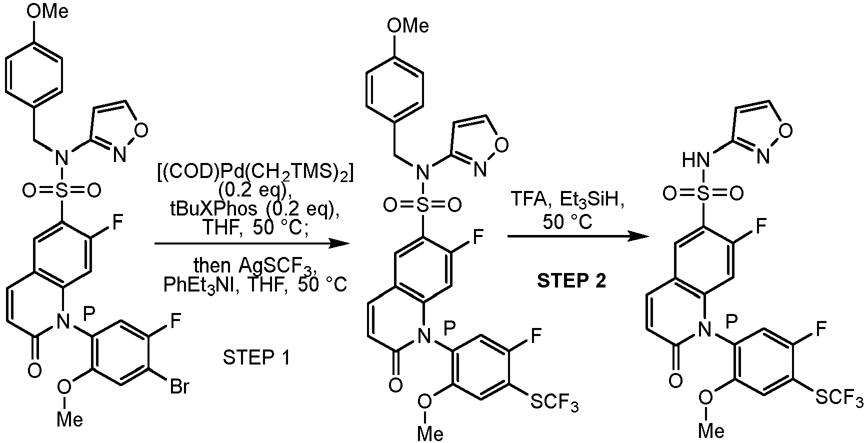

Step 1: A 40 mL vial was charged with (1,5-cyclooctadiene)bis(trimethylsilylmethyl)palladium(II) (0.246 g, 0.632 mmol), di-tert-butyl(2',4',6'-triisopropyl-[1,1'-biphenyl]-2-yl)phosphane (0.295 g, 0.696 mmol), (P)-1-(4-bromo-5-fluoro-2-methoxyphenyl)-7-fluoro-N-(isoxazol-3-yl)-N-(4-methoxybenzyl)-2-oxo-1,2-dihydroquinoline-6-sulfonamide (2.00 g, 3.16 mmol), and toluene (16 mL). The reaction mixture was sparged with nitrogen for 15 min then warmed at 50 °C for 45 min to afford a homogeneous solution. A separate 40 mL vial was charged with phenyltriethylammonium iodide (1.25 g, 4.11 mmol) and silver(I) trifluoromethylthiolate (859 mg, 4.11 mmol). The vial was purged with nitrogen for 15 min before the solution from the first vial was cannulated into second vial and the resultant mixture was vigorously stirred at 50 °C. After 45 min, the mixture was cooled to room temperature, filtered through the pad of Celite, and the filter cake was washed with toluene (10 mL). The filtrate was concentrated in vacuo and the residue was purified by flash column chromatography (ISCO CombiFlash, 24 g Silica Cartridge, eluent: 0 to 50 % EtOAc in heptane) to afford (P)-7-fluoro-1-(5-fluoro-2-methoxy-4-((trifluoromethyl)thio)phenyl)-N-(isoxazol-3-yl)-N-(4-methoxybenzyl)-2-oxo-1,2-dihydroquinoline-6-sulfonamide (1.74 g, 2.66 mmol, 84% yield) as white solid. m/z (ESI) 654.0 (M+H)+.

Step 2: A 250 mL round-bottom flask equipped with a reflux condenser was charged with (P)-7-fluoro-1-(5-fluoro-2-methoxy-4-((trifluoromethyl)thio)phenyl)-N-(isoxazol-3-yl)-N-(4-methoxybenzyl)-2-oxo-1,2-dihydroquinoline-6-sulfonamide (23.2 g, 35.5 mmol), triethylsilane (26.3 mL, 177 mmol), and trifluoroacetic acid (81 mL, 1.05 mol). The resultant mixture was warmed to 50 °C for 6 hours. The reaction mixture was then allowed to cool to room temperature and concentrated under reduced pressure. Saturated aqueous sodium bicarbonate solution (200 mL) was introduced. The resultant mixture was extracted with EtOAc, the organic layer was washed with brine, dried over anhydrous magnesium sulfate, filtered, and concentrated under reduced pressure. The residue was azeotroped from heptane (3 x 70 mL) and the residue was purified by flash column chromatography (Biotage, 200 g Silica Cartridge, eluent: 0-60% ethyl acetate/ethanol (3:1 mix) gradient in heptane/DCM (9:1 mix)). Fractions containing the product were combined and concentrated under reduced pressure. The resulting solid was purified by SFC via a Regis Whelk-O s,s 3 x 15 cm, 5 µm column, a mobile phase of 35 % methanol, and a flowrate of 180 mL/min to afford (P)-7-fluoro-1-(5-fluoro-2-methoxy-4-((trifluoromethyl)thio)phenyl)-N-(isoxazol-3-yl)-2-oxo-1,2-dihydroquinoline-6-sulfonamide (13.7 g, 25.6 mmol, 72 % yield) as white solid. ¹H NMR (500 MHz, DMSO-d6) δ: 12.02 (br s, 1 H), 8.73 (d, *J*=1.6 Hz, 1 H), 8.49 (d, *J*=7.8 Hz, 1 H), 8.26 (d, *J*=9.7 Hz, 1 H), 7.73 (d, *J*=8.4 Hz, 1 H), 7.68 (d, *J=*6.2 Hz, 1 H), 6.77 (d, *J*=9.7 Hz, 1 H), 6.63 (d, *J=*11.8 Hz, 1 H), 6.38 (d, *J*=1.7 Hz, 1 H), 3.74 (s, 3 H). m/z (ESI) 534.0 (M+H)⁺.

### Example 8

### (P)-1-(3-FLUORO-2-METHOXY-4-((TRIFLUOROMETHYL)THIO)PHENYL)-N-(ISOXAZOL-3-YL)-2-OXO-1,2-DIHYDROQUINOLINE-6-SULFONAMIDE

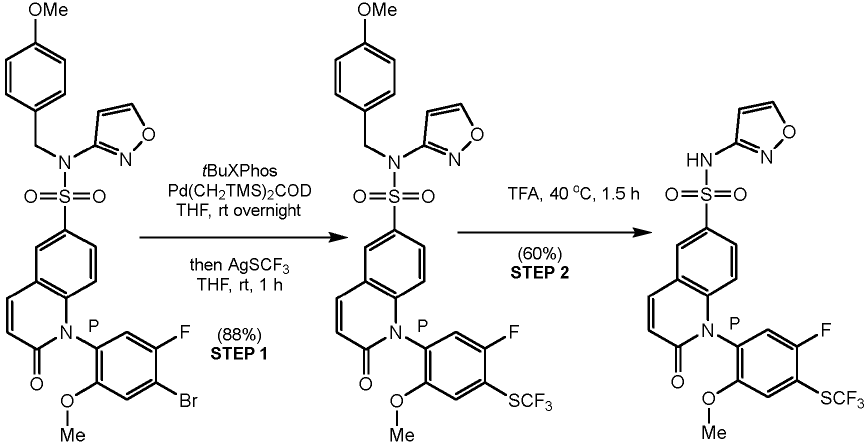

Step 1: A 500 mL round-bottom flask was charged with (P)-1-(4-bromo-5-fluoro-2-methoxyphenyl)-N-(isoxazol-3-yl)-N-(4-methoxybenzyl)-2-oxo-1,2-dihydroquinoline-6-sulfonamide (1.30 g, 2.12 mmol), 2-di-t-butylphosphino-2,4,6-tri-i-propyl-1,1-biphenyl (898 mg, 2.12 mmol) and (1,5-cyclooctadiene)bis(trimethylsilylmethyl)palladium(ii) (823 mg, 2.12 mmol). The flask was purged with nitrogen for 30 minutes before tetrahydrofuran (21.2 mL) was introduced and the reaction mixture was stirred at ambient temperature. After 21 hours, ((trifluoromethyl)thio)silver (575 mg, 2.75 mmol) was introduced in one portion to the stirred reaction mixture. After 1.5 hours, Silicycle SiliaMetS^{®} DMT metal scavenger (9.0 g) was introduced and the mixture was diluted with dichloromethane. The mixture was then filtered through a plug of Celite^{®} and eluted with 10% MeOH in dichloromethane. The filtrate was concentrated under reduced pressure and purified by silica gel chromatography (220 g silica gel column and eluted with 0 to 50% EtOAc/EtOH (3:1) in heptane (with 10% dichloromethane)) to provide (P)-1-(5-fluoro-2-methoxy-4-((trifluoromethyl)thio)phenyl)-N-(isoxazol-3-yl)-N-(4-methoxybenzyl)-2-oxo-1,2-dihydroquinoline-6-sulfonamide (1.19 g, 1.87 mmol, 88 % yield) as a brown foam. m/z (ESI) 636.0 (M+H)+.

Step 2: A 20 mL vial was charged with (P)-1-(5-fluoro-2-methoxy-4-((trifluoromethyl)thio)phenyl)-N-(isoxazol-3-yl)-N-(4-methoxybenzyl)-2-oxo-1,2-dihydroquinoline-6-sulfonamide (1.19 g, 1.87 mmol) and trifluoroacetic acid (19.2 g, 12.9 mL, 169 mmol). The reaction mixture was stirred at 40 °C for 1.5 hours. The reaction mixture was concentrated under reduced pressure before a saturated aqueous solution of sodium bicarbonate was added and the mixture was extracted with dichloromethane. The combined organic layer was concentrated and subjected to reverse phase MPLC purification, eluting with 30 to 75% acetonitrile in water (0.1% formic acid) to provide (P)-1-(5-fluoro-2-methoxy-4-((trifluoromethyl)thio)phenyl)-N-(isoxazol-3-yl)-2-oxo-1,2-dihydroquinoline-6-sulfonamide (0.58 g, 1.13 mmol, 60 % yield) as a beige solid. ¹H NMR (CDCl₃, 500 MHz) δ 8.28 (d, *J*=1.8 Hz, 1H), 8.21 (br s, 1H), 8.1-8.2 (m, 1H), 7.81 (t, *J=*10.5 Hz, 2H), 7.38 (d, *J*=5.8 Hz, 1H), 7.16 (d, *J=*7.7 Hz, 1H), 6.87 (d, *J*=9.6 Hz, 1H), 6.72 (d, *J*=9.0 Hz, 1H), 6.62 (d, *J*=1.8 Hz, 1H), 3.77 (s, 3H). m/z (ESI) 516.7 (M+H)+.

### Example 9

### (P)-1-(5-FLUORO-2-METHOXY-4-(TRIFLUOROMETHOXY)PHENYL)-N-(ISOXAZOL-3-YL)-2-OXO-1,2-DIHYDROQUINOLINE-6-SULFONAMIDE

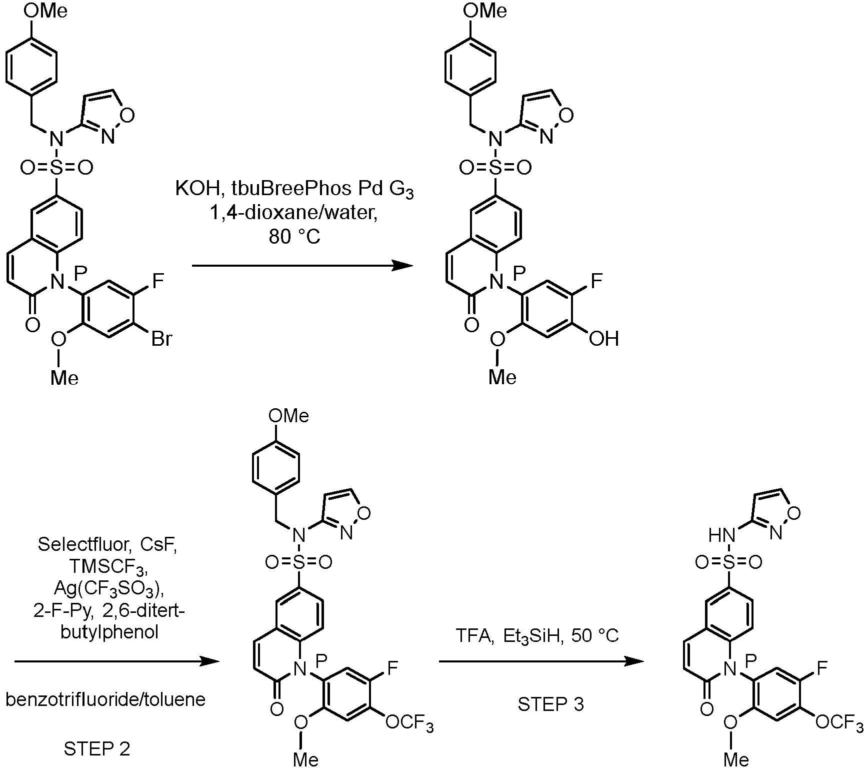

Step 1: To a vial containing [(2-di-tert-butylphosphino-3,6-dimethoxy-2',4',6'-triisopropyl-1,1'-biphenyl)-2-(2'-amino-1,1'-biphenyl)]palladium(ii) methanesulfonate (278 mg, 0.33 mmol) and (P)-1-(4-bromo-5-fluoro-2-methoxyphenyl)-N-(isoxazol-3-yl)-N-(4-methoxybenzyl)-2-oxo-1,2-dihydroquinoline-6-sulfonamide (2.00 g, 3.25 mmol) was added a solution of potassium hydroxide (548 mg, 9.76 mmol) in water (2.00 mL) and 1,4-dioxane (6.00 mL). The resultant reaction mixture was stirred at 80 °C for 2 hours. The reaction mixture was then cooled to ambient temperature and partitioned between DCM and water. The pH of the aqueous phase was adjusted to ~7 with an aqueous solution of HCl (1.0 N) and extracted with DCM (2x). The combined organic layers were dried over anhydrous magnesium sulfate, filtered and concentrated under reduced pressure. The residue was purified by flash column chromatography (silica gel, eluent: 0-50% ethyl acetate/EtOH (3:1) in heptane with 10% DCM) to afford (P)-1-(5-fluoro-4-hydroxy-2-methoxyphenyl)-N-(isoxazol-3-yl)-N-(4-methoxybenzyl)-2-oxo-1,2-dihydroquinoline-6-sulfonamide (1.67 g, 3.03 mmol, 93 % yield). m/z (ESI) 552.0 (M+H)+.

Step 2: A 40 mL vial was charged with silver(I) trifluoromethanesulfonate (1.17 g, 4.53 mmol), 1-(chloromethyl)-4-fluoro-1,4-diazabicyclo[2.2.2]octane-1,4-diium tetrafluoroborate (0.64 g, 1.81 mmol), N-fluoro-N-(phenylsulfonyl)benzenesulfonamide (0.57 g, 1.81 mmol), cesium fluoride (0.83 g, 5.44 mmol), 2,6-di-*tert*-butylphenol (0.37 g, 1.81 mmol), and 1-(5-fluoro-4-hydroxy-2-methoxyphenyl)-N-(isoxazol-3-yl)-N-(4-methoxybenzyl)-2-oxo-1,2-dihydroquinoline-6-sulfonamide (0.50 g, 0.91 mmol) in a nitrogen-filled glovebox. The vial was removed from the glovebox and toluene (4.58 mL), benzotrifluoride (9.16 mL), 2-fluoropyridine (0.44 g, 0.39 mL, 4.53 mmol), and trimethyl(trifluoromethyl)silane (0.64 g, 0.67 mL, 4.53 mmol) were introduced under an argon atmosphere. The resultant reaction mixture was stirred at ambient temperature. After 16 hours, the reaction mixture was filtered through a plug of silica gel and washed with ethyl acetate. The filtrate was concentrated under reduced pressure, and the residue was purified by column chromatography (silica gel eluent: 0-100% ethyl acetate/EtOH (3:1) in heptane with 10% DCM) to afford (P)-1-(5-fluoro-2-methoxy-4-(trifluoromethoxy)phenyl)-N-(isoxazol-3-yl)-N-(4-methoxybenzyl)-2-oxo-1,2-dihydroquinoline-6-sulfonamide (126 mg, 0.20 mmol, 22.4 % yield). m/z (ESI) 620.0 (M+H)+.

Step 3: A mixture of (P)-1-(5-fluoro-2-methoxy-4-(trifluoromethoxy)phenyl)-N-(isoxazol-3-yl)-N-(4-methoxybenzyl)-2-oxo-1,2-dihydroquinoline-6-sulfonamide (126 mg, 0.20 mmol), triethylsilane (0.212 g, 0.29 mL, 1.82 mmol), and 2,2,2-trifluoroacetic acid (1.76 g, 1.15 mL, 15.4 mmol) was stirred at 50 °C. After 2 hours, the reaction mixture was concentrated under reduced pressure, and the residue was purified by column chromatography (silica gel, eluent: 0-60% ethyl acetate/EtOH (3:1) in heptane with 10% DCM) to afford (P)-1-(5-fluoro-2-methoxy-4-(trifluoromethoxy)phenyl)-N-(isoxazol-3-yl)-2-oxo-1,2-dihydroquinoline-6-sulfonamide (22.2 mg, 0.044 mmol, 22.2% yield). ¹H NMR (500 MHz, DMSO-*d*₆) δ: 11.64 (bs, 1 H), 8.72 (d, *J*=1.69 Hz, 1 H), 8.37 (d, *J*=2.08 Hz, 1 H), 8.23 (d, *J*=9.73 Hz, 1 H), 7.84 (dd, *J*=8.95, 2.21 Hz, 1 H), 7.77 (d, *J*=10.38 Hz, 1 H), 7.54 (d, *J*=7.14 Hz, 1 H), 6.85 (d, *J*=8.95 Hz, 1 H), 6.80 (d, *J*=9.60 Hz, 1 H), 6.44 (d, *J*=1.82 Hz, 1 H), 3.71 (s, 3 H). m/z (ESI) 500.0 (M+H)+.

### Example 10

### (P)- 1-(5-FLUORO-2-METHOXY-4-(2,2,2-TRIFLUOROETHOXY) PHENYL)-N-(ISOXAZOL-3-YL)-2-OXO-1,2-DIHYDROQUINOLINE-6-SULFONAMIDE

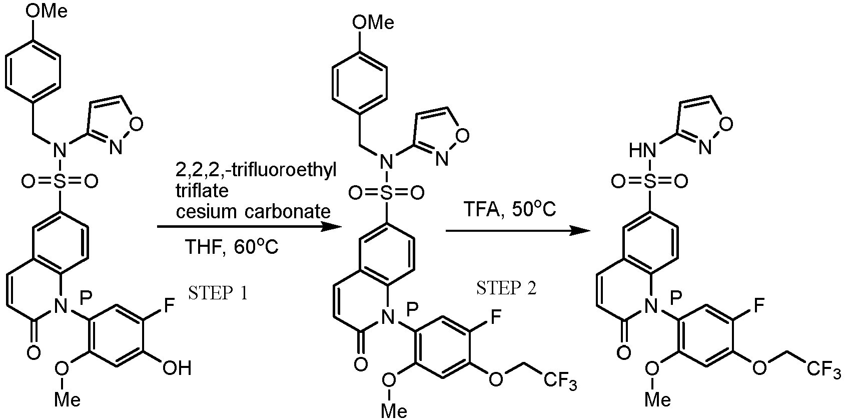

Step 1: To a solution of (P)-1-(5-fluoro-4-hydroxy-2-methoxyphenyl)-N-(isoxazol-3-yl)-N-(4-methoxybenzyl)-2-oxo-1,2-dihydroquinoline-6-sulfonamide (300 mg, 0.54 mmol) and cesium carbonate (532 mg, 1.63 mmol) in tetrahydrofuran (1.00 mL) was added 2,2,2-trifluoroethyl triflate (252 mg, 1.09 mmol). The resultant reaction mixture was purged with nitrogen and warmed to 50 °C. After 6 hours, the reaction mixture was concentrated under reduced pressure and the residue was partitioned between DCM and water. The organic layer was concentrated to afford 1-(5-fluoro-2-methoxy-4-(2,2,2-trifluoroethoxy)phenyl)-N-(isoxazol-3-yl)-N-(4-methoxybenzyl)-2-oxo-1,2-dihydroquinoline-6-sulfonamide. m/z (ESI) 634.1 (M+H)+.

Step 2: A solution of 1-(5-fluoro-2-methoxy-4-(2,2,2-trifluoroethoxy)phenyl)-N-(isoxazol-3-yl)-N-(4-methoxybenzyl)-2-oxo-1,2-dihydroquinoline-6-sulfonamide in TFA (0.5 mL) was stirred for 1 hour at 50 °C. The reaction mixture was concentrated under reduced pressure and the residue was purified by chiral SFC chromatography, via a Regis Whelk-O1 *S,S,* 21 x 250 mm, 5 µm column, a mobile phase of 30 % methanol, and a flowrate of 80 mL/min, to afford (P)-1-(5-fluoro-2-methoxy-4-(2,2,2-trifluoroethoxy)phenyl)-N-(isoxazol-3-yl)-2-oxo-1,2-dihydroquinoline-6-sulfonamide (81.3 mg, 0.16 mmol, 29.1 % yield over two steps) as a white solid. ¹H NMR (600 MHz, DMSO-*d*₆) δ 11.62 (br s, 1 H), 8.72 (d, *J*=1.73 Hz, 1 H), 8.35 (d, *J*=2.09 Hz, 1 H), 8.19 (d, *J*=9.63 Hz, 1 H), 7.84 (dd, *J*=8.99, 2.18 Hz, 1 H), 7.45 (d, *J*=11.08 Hz, 1 H), 7.24 (d, *J*=7.72 Hz, 1 H), 6.81 (d, *J*=8.99 Hz, 1 H), 6.78 (d, *J*=9.63 Hz, 1 H), 6.44 (d, *J*=1.73 Hz, 1 H), 4.97 - 5.06 (m, 2 H), 3.69 (s, 3 H). m/z (ESI) 514.0 (M+H)+.

EXAMPLES 11-26: The compounds below were prepared analogous to the preparations described in the above Examples 1-10.

| Ex | Structure | Chemical Name | m/z (ESI) |
|---|---|---|---|
| 11 | 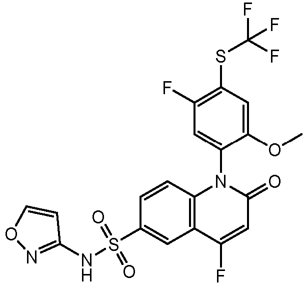 | P-4-fluoro-1-(5-fluoro-2-methoxy-4-((trifluoromethyl)thio) phenyl)-N-(isoxazol-3-yl)-2-oxo-1,2-dihydroquinoline-6-sulfonamide | 533.8 |
| 12 | 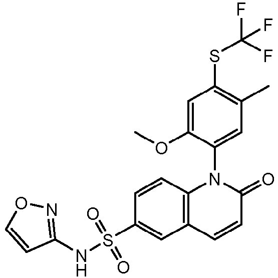 | P-N-(isoxazol-3-yl)-1-(2-methoxy-5-methyl-4-((trifluoromethyl)thio) phenyl)-2-oxo-1,2-dihydroquinoline-6-sulfonamide | 512.2 |
| 13 | 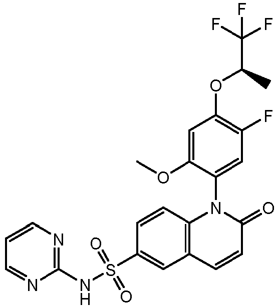 | P-(R)-1-(5-fluoro-2-methoxy-4-((1,1,1-trifluoropropan-2-yl)oxy)phenyl)-2-oxoN-(pyrimidin-2-yl)-1,2-dihydroquinoline-6-sulfonamide | 539.0 |
| 14 | 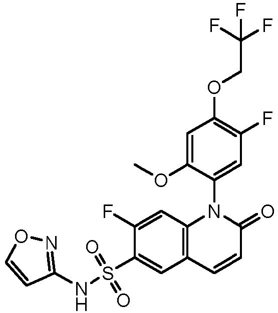 | P-7-fluoro-1-(5-fluoro-2-methoxy-4-(2,2,2-trifluoroethoxy)phenyl )-N-(isoxazol-3-yl)-2-oxo-1,2-dihydroquinoline-6-sulfonamide | 532.0 |
| 15 | 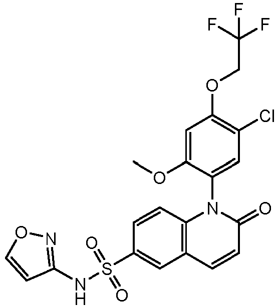 | (P)-1-(5-chloro-2-methoxy-4-(2,2,2-trifluoroethoxy)phenyl )-N-(isoxazol-3-yl)-2-oxo-1,2-dihydroquinoline-6-sulfonamide | 529.8 |
| 16 | 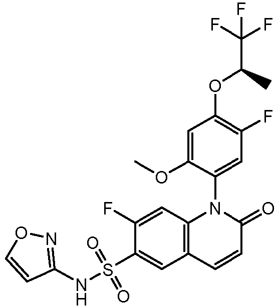 | (P)-(R)-7-fluoro-1-(5-fluoro-2-methoxy-4-((1,1,1-trifluoropropan-2-yl)oxy)phenyl)-N-(isoxazol-3-yl)-2-oxo-1,2-dihydroquinoline-6-sulfonamide | No data |
| 17 | 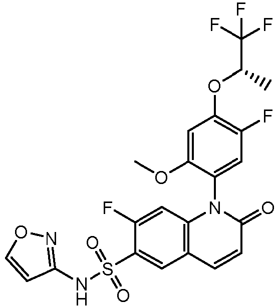 | (P)-(S)-7-fluoro-1-(5-fluoro-2-methoxy-4-((1,1,1-trifluoropropan-2-yl)oxy)phenyl)-N-(isoxazol-3-yl)-2-oxo-1,2-dihydroquinoline-6-sulfonamide | 545.9 |
| 18 | 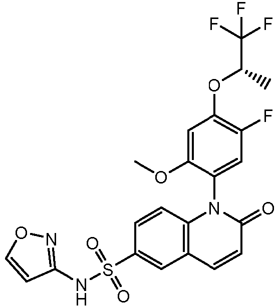 | (P)-(S)-1-(5-fluoro-2-methoxy-4-((1,1,1-trifluoropropan-2-yl)oxy)phenyl)-N-(isoxazol-3-yl)-2-oxo-1,2-dihydroquinoline-6-sulfonamide | 527.8 |
| 19 | 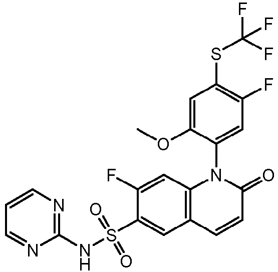 | (P)-7-fluoro-1-(5-fluoro-2-methoxy-4-((trifluoromethyl)thio) phenyl)-2-oxo-N-(pyrimidin-2-yl)-1,2-dihydroquinoline-6-sulfonamide | 545.0 |
| 20 | 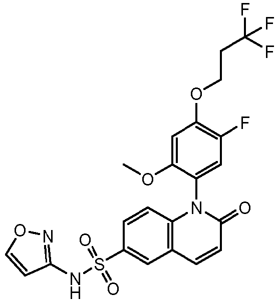 | (P)-1-(5-fluoro-2-methoxy-4-(3,3,3-trifluoropropoxy)phen yl)-N-(isoxazol-3-yl)-2-oxo-1,2-dihydroquinoline-6-sulfonamide | 528.0 |
| 21 | 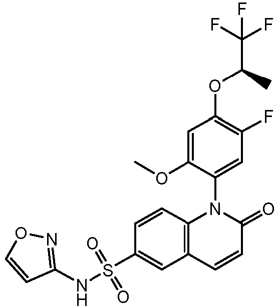 | (P)-(R)-1-(5-fluoro-2-methoxy-4-((1,1,1-trifluoropropan-2-yl)oxy)phenyl)-N-(isoxazol-3-yl)-2-oxo-1,2-dihydroquinoline-6-sulfonamide | 527.8 |
| 22 | 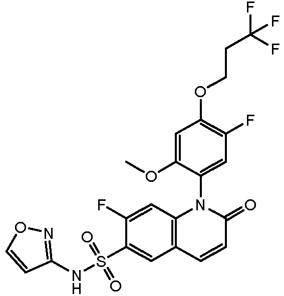 | (P)-7-fluoro-1-(5-fluoro-2-methoxy-4-(3,3,3-trifluoropropoxy)phen yl)-N-(isoxazol-3-yl)-2-oxo-1,2-dihydroquinoline-6-sulfonamide | 546.0 |
| 23 | 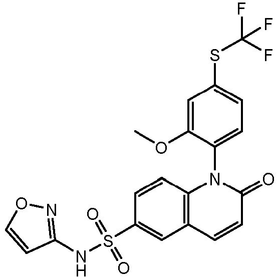 | (P)-N-(isoxazol-3-yl)-1-(2-methoxy-4-((trifluoromethyl)thio) phenyl)-2-oxo-1,2-dihydroquinoline-6-sulfonamide | 497.8 |
| 24 | 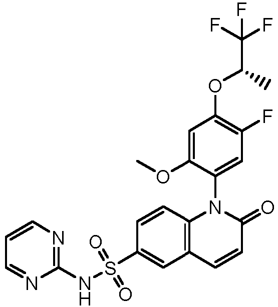 | (P)-(S)-1-(5-fluoro-2-methoxy-4-((1,1,1-trifluoropropan-2-yl)oxy)phenyl)-2-oxoN-(pyrimidin-2-yl)-1,2-dihydroquinoline-6-sulfonamide | 539.0 |
| 25 | 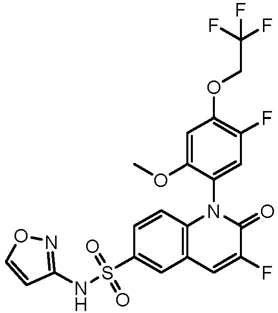 | (P)-3-fluoro-1-(5-fluoro-2-methoxy-4-(2,2,2-trifluoroethoxy)phenyl )-N-(isoxazol-3-yl)-2-oxo-1,2-dihydroquinoline-6-sulfonamide | 532.0 |
| 26 | 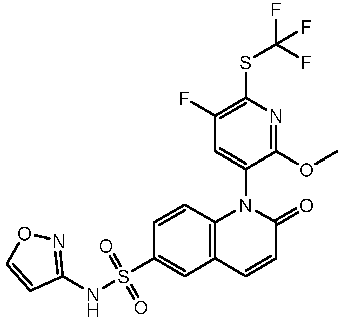 | (P)-1-(5-fluoro-2-methoxy-6-((trifluoromethyl)thio) pyridin-3-yl)-N-(isoxazol-3-yl)-2-oxo-1,2-dihydroquinoline-6-sulfonamide | No data |

### BIOLOGICAL EXAMPLES

The following assays were used in testing the exemplary compounds of the invention. Data for those examples tested in accordance with the procedures described below are presented in Table 1 below.

### IONWORKS BARRACUDA AUTOMATED PATCH CLAMP ASSAY (SAME PROTOCOL FOR BOTH HUMAN AND MOUSE)

Human Nav1.7 currents were recorded in population patch-clamp mode with the IWB automated electrophysiology system (Molecular Devices, LLC, Sunnyvale, CA). Spiking HEK cells (without Kir2.1 transfection) were cultured and prepared for recordings as previously described for IonWorks Quattro testing¹. The external solution consisted of the following (in mM): NaCl 140, KCl 5, CaCl₂ 2, MgCl₂ 1, HEPES 10, and glucose 11, pH 7.4, with N-methyl-D-glucamine at 320 mOsmol. The internal solution consisted of the following (in mM): KCl 70, KF 70, MgCl₂ 0.25, HEDTA 5, and HEPES 10, pH 7.25, with Nmethyl-D-glucamine, 300 mOsmol. From a holding potential of -110 mV, currents were elicited by a train of 26 depolarizations of 150 ms duration to -20 mV at a frequency of 5 Hz. Cells were then clamped to -20 mV for a period of 4 minutes in the presence of a single concentration of test compound. Following this compound incubation period, cells were clamped to -110 mV for three seconds to recover unbound channels and put through the same 26 pulse voltage protocol as above. Peak inward current during the 26th pulse to -20 mV in the presence of compound was divided by the peak inward current evoked by the 26th pulse to -20 mV in the absence of compound to determine percent inhibition. Concentration-response curves of percent inhibition as a function of concentration were generated to calculate IC₅₀ values as described in Kornecook, T. J.; Yin, R.; Altmann, S.; et al. Pharmacologic Characterization of AMG8379, a Potent and Selective Small Molecule Sulfonamide Antagonist of the Voltage-Gated Sodium Channel NaV1. 7. J. Pharmacol. Exp. Ther. 2017, 362, 146-160.

### MICROSOMAL INTRINSIC CLEARANCE ASSAY

The purpose of this assay is to determine the intrinsic clearance of test compound in microsomes from preclinical species and human by monitoring the disappearance of test article over time in hepatic microsomes. 20 mg/mL stock, stored at -80°C microsome was used. List of chemical used: (1) Test article, 10 mM stock (DMSO) or powder from sample bank; (2) Verapamil, 10 mM stock; (3) NADPH, powder (Sigma); (4) Potassium phosphate buffer, 100 mM, pH 7.4; and (5) Tolbutamide (or equivalent). Final incubation concentrations were 0.25 mg/mL microsomal protein and 0.5 µM test article, and incubations are performed in triplicate. The typical time points for the assay were 1, 5, 10, 20, 30, and 40. The assay was carried out in 96-well format, and serially sampled from 400 µL incubation. At the appropriate timepoints, the incubations were quenched with acetonitrile containing internal standard (tolbutamide). Tolbutamide was the default internal standard because it has a signal by positive or negative ion mass spectrometry. The positive control for microsomal intrinsic clearance assay was verapamil. Samples were subjected to LC-MS/MS analysis, and relative amount of compound was calculated by peak area of compound normalized to peak area of internal standard (A/IS). Calculations of intrinsic clearance were performed with Galileo.

### Procedure:

Remove microsomes from -80 °C freezer. Thaw at room temperature or in 37 °C water bath. Store on ice once thawed. Add microsomes (0.53 mg/) to 0.1 M phosphate buffer and aliquot 250 µL per reaction. Prepare 10 mM stock of test article in DMSO. Dilute 1/100 into acetonitrile:water 50:50 to make 100 µM stock. Add about 2.5 µL of the 100 µM test article stock to each reaction to a final concentration of 1.05 µM substrate. (NB: At this stage, concentrations are ~2X higher than the final incubation conditions, to account for ~1:1 dilution with NADPH).

### Prepare 1.9 mM NADPH solution in 0.1 mM phosphate buffer.

Prepare 4 x 250 µL replicate wells of substrate + microsomes containing 1.05 µM substrate and 0.53 mg/mL protein. Prepare 3 replicate wells containing 210 µL 1.90 mM NADPH + 1 well of buffer (-NADPH). Preincubate microsomes + 0.1 M phosphate buffer + test article for 5 minutes at 37°C. To initiate reaction, add 190 µL of substrate + to the wells containing NADPH, yielding a final concentration of 0.25 mg/mL microsomes, 0.5 µM test article, and 1 mM NADPH. Remove 35 µL at 1, 5, 10, 20, 30, and 40 minutes. Quench 1:1 with acetonitrile containing internal standard. Vortex and centrifuge. Transfer for bioanalysis by LC-MS/MS.

### OPEN-FIELD LOCOMOTOR ACTIVITY IN MICE.

On the day of testing, C57Bl/6 male mice were orally administered either Nav1.7 compound or a vehicle control formulation at a dose volume of 10 ml/kg. The vehicle used was 2% HPMC/1% Tween 80 pH 10 with NaOH; DI water at pH 10 w/NaOH; or 2% HPMC/1% Tween 80 pH 2.2.

Two to three hours following test article treatment, depending on the cmax of the each Nav1.7 test compound of the invention, animals were placed into open-field chamber and the animal behavior was monitored over a 30-minute period. For the Thousand Oaks Site Experiments, 16" x 16" open-field chamber, KINDER SCIENTIFIC^{®}, San Diego, CA, was used. For the Cambridge Massachusetts Site Experiments, 16" x 16" open-field chamber, SAN DIEGO INSTRUMENTS^{®}, San Diego, CA, was used. Locomotor activity (horizontal movement and rearing activity) parameters were measured in an automated manner via infrared photo-beam breaks.

### HUMAN CYP 3A4 INDUCTION ASSAY

Cryopreserved human hepatocytes were seeded in 96-well collagen coated plates at 70,000 cells per well in hepatocyte plating media (HPM, final concentrations: 1x Dulbecco's Modified Eagle's Medium, 0.1 µM dexamethasone, 10% fetal bovine serum, 1x ITS, 1x PSG) followed by incubation at 37 °C under 5% CO2 and 90% relative humidity for 2 days to allow hepatocytes to form a confluent layer. On Day 3, hepatocytes were treated with either test compound or rifampin (20 µM, positive control for CYP3A induction) prepared in hepatocyte incubation media ((HIM, final concentrations: 1X William's Medium E, 0.1 µM dexamethasone, 1x ITS, 1x PSG). Treatment was performed for 72 hours with either 2 concentrations (2 µM or 10 µM) or a range of concentrations (0.001 µM to 100 µM) of the test compound to obtain full dose-response curve. Fresh media containing the relevant concentrations of the test compound was replaced every day until the samples were processed. After 72 hours of incubation, samples were processed for mRNA analysis using bDNA technology using manufacturer's instructions (Affymetrix, Fremont, CA). Cell viability was tested at the end of the experiment using MTT assay kit (Roche Diagnostics, Basel, Switzerland). Data was analyzed and presented as percent of control (POC) and Eₘₐₓ and EC₅₀ obtained when appropriate according to guidance from Center for Drug Evaluation and Research (CDER), 2006, Guidance for Industry, Drug Interaction Studies - Study Design, Data Analysis, and Implications for Dosing and Labeling.

Cryopreserved human hepatocytes were seeded in 96-well collagen coated plates at 70,000 cells per well in hepatocyte plating media (HPM, final concentrations: 1x Dulbecco's Modified Eagle's Medium, 0.1 µM dexamethasone, 10% fetal bovine serum, 1x ITS, 1x PSG) followed by incubation at 37 °C under 5% CO2 and 90% relative humidity for 2 days to allow hepatocytes to form a confluent layer. On Day 3, hepatocytes were treated with either test compound or rifampin (20 µM, positive control for CYP3A induction) prepared in hepatocyte incubation media ((HIM, final concentrations: 1X William's Medium E, 0.1 µM dexamethasone, 1x ITS, 1x PSG). Treatment was performed for 72 hours with either 2 concentrations (2 µM or 10 µM) or a range of concentrations (0.001 µM to 100 µM) of the test compound to obtain full dose-response curve. Fresh media containing the relevant concentrations of the test compound was replaced every day until the samples were processed. After 72 hours of incubation, samples were processed for mRNA analysis using bDNA technology using manufacturer's instructions (Affymetrix, Fremont, CA). Cell viability was tested at the end of the experiment using MTT assay kit (Roche Diagnostics, Basel, Switzerland). Data was analyzed and presented as percent of control (POC) and Eₘₐₓ and EC₅₀ obtained when appropriate, as described in Halladay, J. et al, 2012, An "all-inclusive" 96-well cytochrome P450 induction method: Measuring enzyme activity, mRNA levels, protein levels, and cytotoxicity from one well using cryopreserved human hepatocytes, Pharmacological and Toxicological Methods, 66:270-275.

The compounds of the present invention may also be tested in the following in vivo assays.

### RAT FORMALIN MODEL OF PERSISTENT PAIN

On the test day, animals (Naive, male Sprague Dawley rats) weighing between 260-300g at the start of testing can be obtained from Harlan (Indianapolis, IN). All animals may be housed under a 12/12h light/dark cycle with lights on at 0600. Rodents can be housed two to a cage on solid bottom cages with com cob bedding and can have access to food and water *ad libitum.* Animals should be allowed to habituate to the vivarium for at least five days before testing is begun and should be brought into the testing room at least 30 minutes prior to dosing. Animals are pretreated with the appropriate test compound either by oral gavage or intraperitoneal injection at the desired pretreatment time (typically two hours before test onset) and then returned to their home cages. After dosing and at least 30 minutes prior to test onset, animals can be acclimated to the individual testing chambers. At test time, each animal can be gently wrapped in a towel with the left hind paw exposed. A dilute solution of formalin (2.5%) in phosphate buffered saline can be injected subcutaneously into the dorsal surface of the left hind paw in a volume to 50 µL with a 30 g needle. Immediately following injection, a small metal band can be affixed to the plantar side of the left hind paw with a drop of LOCTITE (adhesive). Animals may be then placed into the testing chambers and the number of flinches can be recorded between 10 to 40 minutes after formalin injection. A flinch is defined as a quick and spontaneous movement of the injected hind paw not associated with ambulation. Flinches can be quantified with the aid of the Automated Nociception Analyzer built by the University of California, San Diego Department of Anesthesiology. Individual data can be expressed as a % maximal potential effect (%MPE) calculated with the following formula:(-(Individual score- Vehicle average score)/ Vehicle average score)) * 100 = %MPE

Statistical analysis can be performed by analysis of variance (ANOVA), with post-hoc analysis using Bonferroni compared to the vehicle group for a significant main effect. Data can be represented as mean %MPE +/- standard error for each group.

### RAT OPEN FIELD ASSAY

On the test day, animals (Naive, male Sprague Dawley rats) weighing between 260-300g at the start of testing may be obtained from Harlan (Indianapolis, IN). All animals can be housed under a 12/12h light/dark cycle with lights on at 0600. Rodents can be housed two to a cage on solid bottom cages with com cob bedding and can have access to food and water *ad libitum.* Animals should be allowed to habituate to the vivarium for at least five days before testing is begun and should be brought into the testing room at least 30 minutes prior to dosing. In a room separate from the testing room, animals can be pretreated with the appropriate test compound either by oral gavage or intraperitoneal injection at the desired pretreatment time (typically two hours before test onset) and then can be returned to their home cages until the pretreatment has elapsed. At test time, animal can be transferred to the open field testing room in their home cages. Each animal may be placed in a separate testing chamber and the motion tracking system is started. The house lights in the testing room should be turned off and the animals can be allowed to explore the novel open field for 30 minutes. An automated motion tracker, made by San Diego Instruments, San Diego, CA, can be used to capture animal exploration with the aid of infrared photo beams to detect animal movement. These behaviors include basic movement and vertical rearing, which can be used as the primary endpoints for this assay. At the end of the test, house lights can be turned on and the animals should be removed from the testing apparatus. Data can be expressed as a percent change from the vehicle control using the following equation.

### (1-(Test mean/ Vehicle mean))*100= %Change.

Statistical analysis can be performed by analysis of variance (ANOVA), with post-hoc analysis using Dunnett to follow up significant main effects.

### MOUSE FORMALIN MODEL OF PERSISTENT PAIN

Mice (Naive, male C57Bl/6) weighing between 22-30 g at the start of testing were obtained from Harlan (Indianapolis, IN). All animals were housed under a 12/12h light/dark cycle with lights on at 0630. Rodents were singly housed on solid bottom cages with corn cob bedding and had access to food and water *ad libitum.* Animals were allowed to habituate to the vivarium for at least five days before testing was begun and were brought into the testing room at least 30 minutes prior to dosing. Animals were pretreated with the appropriate test compound either by oral gavage or intraperitoneal injection at the desired pretreatment time (typically two hours before test onset) and then returned to their home cages. After dosing and at least 5 minutes prior to test onset, animals were acclimated to the individual testing chambers. At test time, each animal was gently wrapped in a cloth glove with the left hind paw exposed. A dilute solution of formalin (2%) in phosphate buffered saline was injected subcutaneously into the dorsal surface of the left hind paw in a volume to 20 µL with a 30 g needle. Animals were then placed into the observation chambers and the behaviors were recorded for 60 minutes following the formalin injection. A pain-like behavior was defined as licking and/or non-weight bearing of the injected hind paw not associated with ambulation.

Statistical analysis was performed by analysis of variance (ANOVA), with post-hoc analysis using the Dunnett post-hoc test compared to the vehicle group for any significant main effect. Data were represented as mean +/- standard error for each group.

Table 1 provides data for compounds exemplified in the present application and priority document thereof, as representative compounds of the present invention, as follows: compound name (as named using ChemDraw Ultra version 15.1); and biological data including *in-vitro* Nav 1.7 IWQ data (IC₅₀ in uM), and CYP3A4 mRNA Human Induction data in vitro @ 2 uM and 10 uM Percent of Control (POC) (%), where available. Ex. # refers to Example No. ND means no data was available. Representative compounds of the present invention show lower CYP3A4 mRNA Human Induction data compared to the comparison compound A. Preferably, representative compounds of the present invention show lower CYP3A4 mRNA Human Induction data as well as favorable *in-vitro* human Nav 1.7 IWQ data activities compared to the comparison compound A. Compound A is named (P)-1-(5-fluoro-2-methoxy-4-(3,3,3-trifluoropropyl)phenyl)-N-(1,2-oxazol-3-yl)-2-oxo-1,2-dihydro-6-quinolinesulfonamide, having the structure below:

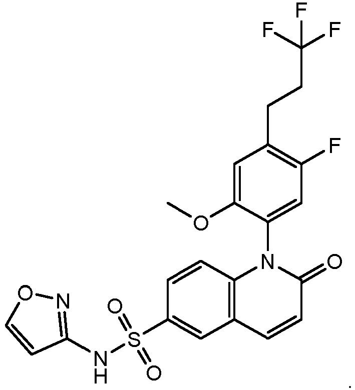

Compound A was exemplified in International Patent Publication No. WO/2017/106871.

**TABLE 1: BIOLOGICAL DATA**

| Ex.# | hNaV1.7 IWB-U IC50 | CYP3A4 mRNA Induct Hu 2 uM POC | CYP3A4 mRNA Induct Hu 10 uM POC |
|---|---|---|---|
| Compound A | 0.014 | 41.9 | 76.1 |
| 1 | 0.007 | 1.7 | ND |
| 2 | 0.008 | ND | ND |
| 3 | 0.009 | 10.1 | 35.4 |
| 4 | 0.011 | 0.2 | 0.2 |
| 5 | 0.011 | 3.4 | 7.5 |
| 6 | 0.011 | 18.3 | 30.1 |
| 7 | 0.011 | 20.9 | 54.2 |
| 8 | 0.015 | 6.1 | 24.5 |
| 9 | 0.021 | 7.8 | 22.2 |
| 10 | 0.021 | 10.8 | 35.7 |
| 11 | 0.010 | 7.3 | 48.1 |
| 12 | 0.014 | 20.2 | 61.2 |
| 13 | 0.021 | 7.0 | 35.3 |
| 14 | 0.023 | 3.6 | 21.4 |
| 15 | 0.023 | 7.7 | 20.2 |
| 16 | 0.024 | 3.9 | 7.2 |
| 17 | 0.027 | 16.3 | 39.8 |
| 18 | 0.027 | 2.6 | 9.5 |
| 19 | 0.029 | 17.4 | 49.2 |
| 20 | 0.030 | 10.8 | 29.6 |
| 21 | 0.032 | 0.4 | 1.8 |
| 22 | 0.034 | 14.8 | 41.5 |
| 23 | 0.039 | 7.4 | 25.1 |
| 24 | 0.068 | 0.8 | 1.2 |
| 25 | 0.102 | 11.5 | 38.0 |
| 26 | 0.185 | 5.7 | 25.6 |

The foregoing invention has been described in some detail by way of illustration and example, for purposes of clarity and understanding. Those skilled in the art understand that changes and modifications may be practiced within the scope of the appended claims. Therefore, it is to be understood that the above description is intended to be illustrative and not restrictive. The scope of the invention should, therefore, be determined not with reference to the above description, but should instead be determined with reference to the following appended claims.

## Claims

1. A compound of Formula I, or an enantiomer, diastereoisomer, atropisomer, or a mixture
thereof, or a pharmaceutically acceptable salt thereof, 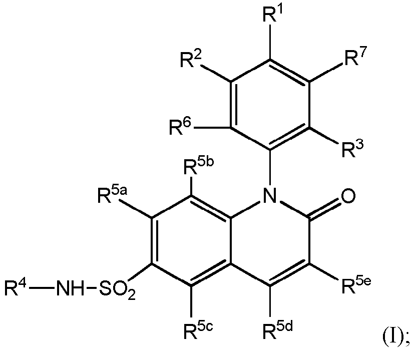 wherein:
R¹ is -C₀₋₄alk-O-C₁₋₈alk, -C₀₋₄alk-S-C₁₋₈alk, or -C₀₋₄alk-NH(C₁₋₈alk); wherein said C₁₋₈alk is substituted by 0, 1, 2, 3, 4, 5, or 6 halo;
R² is H, halo, CN, C₁₋₆alk, or C₁₋₆haloalk;
R³ is C₁₋₆alk, C₁₋₆haloalk, -O-C₁₋₆alk, -O-cyclopropyl, or -O-cyclobutyl;
R⁴ is a 5- to 6-membered heteroaryl;
each of R⁶ and R⁷ is hydrogen; and
each of R^{5a}; R^{5b};R^{5c}; R^{5d;} and R^{5e} is independently hydrogen or halo.

2. The compound according to claim 1, or an enantiomer, diastereoisomer, atropisomer, or a mixture thereof, or a pharmaceutically acceptable salt thereof, wherein R¹ is -O-C₁₋₈alk, -CH₂-O-C₁₋₈alk, -S-C₁₋₈alk, -CH₂-S-C₁₋₈alk, or -CH(CH₃)-S-C₁₋₈alk, wherein said C₁₋₈alk is substituted by 1, 2, 3, or 4 halo.

3. The compound according to any one of claims 1-2, or an enantiomer, diastereoisomer, atropisomer, or a mixture thereof, or a pharmaceutically acceptable salt thereof, wherein R¹ is selected from -O-CF₃, -O-CH₂-CF₃, -O-CH₂-CH₂-CF₃, -O-CH(CH₃)-CF₃, -CH₂-O-CF₃, -S-CF₃, or -S-CH₂-CF₃.

4. The compound according to any one of claims 1-3, or an enantiomer, diastereoisomer, atropisomer, or a mixture thereof, or a pharmaceutically acceptable salt thereof, wherein R² is H, fluoro, chloro, methyl, CN, CF₃, CHF₂, or CH₂F.

5. The compound according to any one of claims 1-4, or an enantiomer, diastereoisomer, atropisomer, or a mixture thereof, or a pharmaceutically acceptable salt thereof, wherein R³ is methoxy.

6. The compound according to any one of claims 1-5, or an enantiomer, diastereoisomer, atropisomer, or a mixture thereof, or a pharmaceutically acceptable salt thereof, wherein R⁴ is isoxazolyl, pyridazinyl, thiazolyl, thiadiazolyl, oxazolyl, or pyrimidinyl.

7. The compound according to any one of claims 1-6, or an enantiomer, diastereoisomer, atropisomer, or a mixture thereof, or a pharmaceutically acceptable salt thereof, wherein:
each of R^{5a}; R^{5b};R^{5c}; R^{5d;} and R^{5e} is hydrogen;
R^{5a} is F and each of R^{5b};R^{5c}; R^{5a;} and R^{5e} is hydrogen; or
R^{5c} is F; and each of R^{5a}; R^{5b};R^{5d}; and R^{5e} is hydrogen.

8. The compound according to claim 1, or a pharmaceutically acceptable salt thereof, which is selected from the group consisting of:
a) (P)-1-(5-chloro-2-methoxy-4-((trifluoromethoxy)methyl)phenyl)-N-(isoxazol-3-yl)-2-oxo-1,2-dihydroquinoline-6-sulfonamide;
b) (P)-1-(5-chloro-2-methoxy-4-((trifluoromethyl)thio)phenyl)-N-(isoxazol-3-yl)-2-oxo-1,2-dihydroquinoline-6-sulfonamide;
c) (P)-1-(5-chloro-2-methoxy-4-(triBuoromethoxy)phenyl)-N-(isoxazol-3-yl)-2-oxo-1,2-dihydroquinoline-6-sulfonamide;
d) (P)-1-(5-fluoro-2-methoxy-4-((trifluoromethoxy)methyl)phenyl)-N-(isoxazol-3-yl)-2-oxo-1,2-dihydroquinoline-6-sulfonamide;
e) (P)-1-(5-fluoro-2-methoxy-4-((2,2,2-trifluoroethyl)thio)phenyl)-N-(isoxazol-3-yl)-2-oxo-1,2-dihydroquinoline-6-sulfonamide;
f) (P)-7-fluoro-1-(5-fluoro-2-methoxy-4-((trifluoromethoxy)methyl)phenyl)-N-(isoxazol -3-yl)-2-oxo-1,2-dihydroquinoline-6-sulfonamide;
g) (P)-7-fluoro-1-(5-fluoro-2-methoxy-4-((trifluoromethyl)thio)phenyl)-N-(isoxazol-3-y1)-2-oxo-1,2-dihydroquinoline-6-sulfonamide;
h) (P)-4-fluoro-1-(5-fluoro-2-methoxy-4-((trifluoromethyl)thio)phenyl)-N-(isoxazol-3-y1)-2-oxo-1,2-dihydroquinoline-6-sulfonamide; or
i) (P)-N-(isoxazol-3-yl)-1-(2-methoxy-5-methyl-4-((trifluoromethyl)thio)phenyl)-2-oxo-1,2-dihydroquinoline-6-sulfonamide.

9. The compound according to claim 1, or a pharmaceutically acceptable salt thereof, which is selected from the group consisting of:
a) (P)-1-(5-chloro-2-methoxy-4-((trifluoromethoxy)methyl)phenyl)-N-(isoxazol-3-yl)-2-oxo-1,2-dihydroquinoline-6-sulfonamide;
b) (P)-1-(5-chloro-2-methoxy-4-(trifluoromethoxy)phenyl)-N-(isoxazol-3-y1)-2-oxo-1,2-dihydroquinoline-6-sulfonamide;
c) (P)-1-(5-fluoro-2-methoxy-4-((trifluoromethoxy)methyl)phenyl)-N-(isoxazol-3-yl)-2-oxo-1,2-dihydroquinoline-6-sulfonamide; or
d) (P)-7-fluoro-1-(5-fluoro-2-methoxy-4-((trifluoromethoxy)methyl)phenyl)-N-(isoxazol-3-yl)-2-oxo-1,2-dihydroquinoline-6-sulfonamide.

10. The compound according to claim 1, or a pharmaceutically acceptable salt thereof, which is selected from the group consisting of:
a) (P)-1-(5-chloro-2-methoxy-4-((trifluoromethyl)thio)phenyl)-N-(isoxazol-3-yl)-2-oxo-1,2-dihydroquinoline-6-sulfonamide;
b) (P)-1-(5-fluoro-2-methoxy-4-((2,2,2-trifluoroethyl)thio)phenyl)-N-(isoxazol-3-yl)-2-oxo-1,2-dihydroquinoline-6-sulfonamide;
c) (P)-7-fluoro-1-(5-fluoro-2-methoxy-4-((trifluoromethyl)thio)phenyl)-N-(isoxazol-3-yl)-2-oxo-1,2-dihydroquinoline-6-sulfonamide;
d) (P)-4-fluoro-1-(5-fluoro-2-methoxy-4-((trifluoromethyl)thio)phenyl)-N-(isoxazol-3-yl)-2-oxo-1,2-dihydroquinoline-6-sulfonamide; or
e) (P)-N-(isoxazol-3-yl)-1-(2-methoxy-5-methyl-4-((trifluoromethyl)thio)phenyl)-2-oxo-1,2-dihydroquinoline-6-sulfonamide.

11. The compound according to any one of claims 1-7, or an enantiomer, diastereoisomer, atropisomer, or a mixture thereof, or a pharmaceutically acceptable salt thereof, wherein said atropisomer, when present, is a P atropisomer.

12. A pharmaceutical composition comprising a compound according to any one of claims 1-11, or a pharmaceutically acceptable salt thereof, and a pharmaceutically acceptable excipient.

13. The compound according to any one of claims 1-11, or a pharmaceutically acceptable salt thereof, or the pharmaceutical composition according to claim 12, for use as a medicament.

14. The compound according to any one of claims 1-11, or a pharmaceutically acceptable salt thereof, or the pharmaceutical composition according to claim 12, for use in treating pain, cough, or itch.

15. The compound, or a pharmaceutically acceptable salt thereof, or the pharmaceutical composition for use according to claim 14, wherein the pain is selected from chronic pain, acute pain, neuropathic pain, pain associated with rheumatoid arthritis, pain associated with osteoarthritis, pain associated with cancer, peripheral diabetic neuropathy, and neuropathic low back pain.

16. The compound, or a pharmaceutically acceptable salt thereof, or the pharmaceutical composition for use according to claim 14, wherein the cough is selected from post viral cough, viral cough, or acute viral cough.

## Patentansprüche

1. Eine Verbindung der Formel I oder ein Enantiomer, Diastereomer, Atropisomer oder eine Mischung davon oder ein pharmazeutisch akzeptables Salz davon, 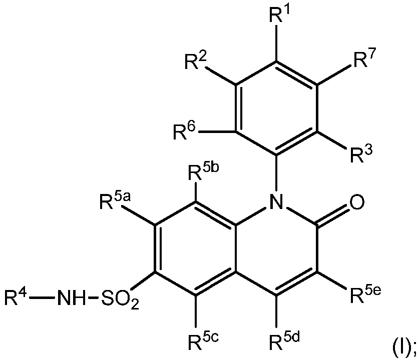 wobei:
R¹ -C₀₋₄Alk-O-C₁₋₈Alk, -C₀₋₄Alk-S-C₁₋₈Alk oder -C₀₋₄Alk-NH(C₁₋₈Alk) ist; wobei das C₁₋₈Alk mit 0, 1, 2, 3, 4, 5 oder 6 Halo substituiert ist;
R² H, Halo, CN, C₁₋₆Alk oder C₁₋₆Haloalk ist;
R³ C₁₋₆Alk, C₁₋₆Haloalk, -O-C₁₋₆Alk, -O-Cyclopropyl oder -O-Cyclobutyl ist;
R⁴ ein 5- bis 6-gliedriges Heteroaryl ist;
jedes von R⁵ und R⁷ Wasserstoff ist; und
jedes von R^{5a}, R^{5b}, R^{5c}, R^{5d} und R^{5e} unabhängig Wasserstoff oder Halo ist.

2. Verbindung gemäß Anspruch 1 oder ein Enantiomer, Diastereomer, Atropisomer oder eine Mischung davon oder ein pharmazeutisch akzeptables Salz davon, wobei R¹ -O-C₁₋₈Alk, -CH₂-O-C₁₋₈Alk, -S-C₁₋₈Alk, -CH₂-S-C₁₋₈Alk oder -CH(CH₃)-S-C₁₋₈Alk ist, wobei das C₁₋₈Alk mit 1, 2, 3 oder 4 Halo substituiert ist.

3. Verbindung gemäß einem der Ansprüche 1-2 oder ein Enantiomer, Diastereomer, Atropisomer oder eine Mischung davon oder ein pharmazeutisch akzeptables Salz davon, wobei R¹ aus -O-CF₃, -O-CH₂-CF₃, -O-CH₂-CH₂-CF₃, -O-CH(CH₃)-CF₃, -CH₂-O-CF₃, -S-CF₃ oder -S-CH₂-CF₃ ausgewählt ist.

4. Verbindung gemäß einem der Ansprüche 1-3 oder ein Enantiomer, Diastereomer, Atropisomer oder eine Mischung davon oder ein pharmazeutisch akzeptables Salz davon, wobei R² H, Fluor, Chlor, Methyl, CN, CF₃, CHF₂ oder CH₂F ist.

5. Verbindung gemäß einem der Ansprüche 1-4 oder ein Enantiomer, Diastereomer, Atropisomer oder eine Mischung davon oder ein pharmazeutisch akzeptables Salz davon, wobei R³ Methoxy ist.

6. Verbindung gemäß einem der Ansprüche 1-5 oder ein Enantiomer, Diastereomer, Atropisomer oder eine Mischung davon oder ein pharmazeutisch akzeptables Salz davon, wobei R⁴ Isoxazolyl, Pyridazinyl, Thiazolyl, Thiadiazolyl, Oxazolyl oder Pyrimidinyl ist.

7. Verbindung gemäß einem der Ansprüche 1-6 oder ein Enantiomer, Diastereomer, Atropisomer oder eine Mischung davon oder ein pharmazeutisch akzeptables Salz davon, wobei:
jedes von R^{5a}, R^{5b}, R^{5c}, R^{5d} und R^{5e} Wasserstoff ist;
R^{5a} F ist und jedes von R^{5b}, R^{5c}, R^{5d} und R^{5e} Wasserstoff ist; oder
R^{5c} F ist, und jedes von R^{5a}, R^{5b}, R^{5d} und R^{5e} Wasserstoff ist.

8. Verbindung gemäß Anspruch 1 oder ein pharmazeutisch akzeptables Salz davon, ausgewählt aus der Gruppe, bestehend aus:
a) (P)-1-(5-Chlor-2-methoxy-4-((trifluormethoxy)methyl)phenyl)-N-(isoxazol-3-yl)-2-oxo-1,2-dihydrochinolin-6-sulfonamid;
b) (P)-1-(5-Chlor-2-methoxy-4-((trifluormethyl)thio)phenyl)-N-(isoxazol-3-yl)-2-oxo-1,2-dihydrochinolin-6-sulfonamid;
c) (P)-1-(5-Chlor-2-methoxy-4-(trifluormethoxy)phenyl)-N-(isoxazol-3-yl)-2-oxo-1,2-dihydrochinolin-6-sulfonamid;
d) (P)-1-(5-Fluor-2-methoxy-4-((trifluormethoxy)methyl)phenyl)-N-(isoxazol-3-yl)-2-oxo-1,2-dihydrochinolin-6-sulfonamid;
e) (P)-1-(5-Fluor-2-methoxy-4-((2,2,2-trifluorethyl)thio)phenyl)-N-(isoxazol-3-yl)-2-oxo-1,2-dihydrochinolin-6-sulfonamid;
f) (P)-7-Fluor-1-(5-fluor-2-methoxy-4-((trifluormethoxy)methyl)phenyl)-N-(isoxazol-3-yl)-2-oxo-1,2-dihydrochinolin-6-sulfonamid;
g) (P)-7-Fluor-1-(5-fluor-2-methoxy-4-((trifluormethyl)thio)phenyl)-N-(isoxazol-3-yl)-2-oxo-1,2-dihydrochinolin-6-sulfonamid;
h) (P)-4-Fluor-1-(5-fluor-2-methoxy-4-((trifluormethyl)thio)phenyl)-N-(isoxazol-3-yl)-2-oxo-1,2-dihydrochinolin-6-sulfonamid; oder
i) (P)-N-(Isoxazol-3-yl)-1-(2-methoxy-5-methyl-4-((trifluormethyl)thio)phenyl)-2-oxo-1,2-dihydrochinolin-6-sulfonamid.

9. Verbindung gemäß Anspruch 1 oder ein pharmazeutisch akzeptables Salz davon, ausgewählt aus der Gruppe, bestehend aus:
a) (P)-1-(5-Chlor-2-methoxy-4-((trifluormethoxy)methyl)phenyl)-N-(isoxazol-3-yl)-2-oxo-1,2-dihydrochinolin-6-sulfonamid;
b) (P)-1-(5-Chlor-2-methoxy-4-(trifluormethoxy)phenyl)-N-(isoxazol-3-yl)-2-oxo-1,2-dihydrochinolin-6-sulfonamid;
c) (P)-1-(5-Fluor-2-methoxy-4-((trifluormethoxy)methyl)phenyl)-N-(isoxazol-3-yl)-2-oxo-1,2-dihydrochinolin-6-sulfonamid; oder
d) (P)-7-Fluor-1-(5-fluor-2-methoxy-4-((trifluormethoxy)methyl)phenyl)-N-(isoxazol-3-yl)-2-oxo-1,2-dihydrochinolin-6-sulfonamid.

10. Verbindung gemäß Anspruch 1 oder ein pharmazeutisch akzeptables Salz davon, ausgewählt aus der Gruppe, bestehend aus:
a) (P)-1-(5-Chlor-2-methoxy-4-((trifluormethyl)thio)phenyl)-N-(isoxazol-3-yl)-2-oxo-1,2-dihydrochinolin-6-sulfonamid;
b) (P)-1-(5-Fluor-2-methoxy-4-((2,2,2-trifluorethyl)thio)phenyl)-N-(isoxazol-3-yl)-2-oxo-1,2-dihydrochinolin-6-sulfonamid;
c) (P)-7-Fluor-1-(5-fluor-2-methoxy-4-((trifluormethyl)thio)phenyl)-N-(isoxazol-3-yl)-2-oxo-1,2-dihydrochinolin-6-sulfonamid;
d) (P)-4-Fluor-1-(5-fluor-2-methoxy-4-((trifluormethyl)thio)phenyl)-N-(isoxazol-3-yl)-2-oxo-1,2-dihydrochinolin-6-sulfonamid; oder
e) (P)-N-(Isoxazol-3-yl)-1-(2-methoxy-5-methyl-4-((trifluormethyl)thio)phenyl)-2-oxo-1,2-dihydrochinolin-6-sulfonamid.

11. Verbindung gemäß einem der Ansprüche 1-7 oder ein Enantiomer, Diastereomer, Atropisomer oder eine Mischung davon oder ein pharmazeutisch akzeptables Salz davon, wobei das Atropisomer, wenn vorhanden, ein P-Atropisomer ist.

12. Eine pharmazeutische Zusammensetzung, beinhaltend eine Verbindung gemäß einem der Ansprüche 1-11 oder ein pharmazeutisch akzeptables Salz davon und einen pharmazeutisch akzeptablen Hilfsstoff.

13. Verbindung gemäß einem der Ansprüche 1-11 oder ein pharmazeutisch akzeptables Salz davon oder pharmazeutische Zusammensetzung gemäß Anspruch 12 zur Verwendung als Medikament.

14. Verbindung gemäß einem der Ansprüche 1-11 oder ein pharmazeutisch akzeptables Salz davon oder pharmazeutische Zusammensetzung gemäß Anspruch 12 zur Verwendung bei der Behandlung von Schmerzen, Husten oder Juckreiz.

15. Verbindung oder ein pharmazeutisch akzeptables Salz davon oder pharmazeutische Zusammensetzung zur Verwendung gemäß Anspruch 14, wobei die Schmerzen aus chronischen Schmerzen, akuten Schmerzen, neuropathischen Schmerzen, mit rheumatoider Arthritis verbundenen Schmerzen, mit Osteoarthrose verbundenen Schmerzen, mit Krebs verbundenen Schmerzen, peripherer diabetischer Neuropathie und neuropathischen Schmerzen im unteren Rücken ausgewählt sind.

16. Verbindung oder ein pharmazeutisch akzeptables Salz davon oder pharmazeutische Zusammensetzung zur Verwendung gemäß Anspruch 14, wobei der Husten aus postviralem Husten, viralem Husten oder akutem viralem Husten ausgewählt ist.

## Revendications

1. Un composé de Formule (I), ou un énantiomère, diastéréoisomère, atropisomère, ou un mélange de ceux-ci, ou un sel pharmaceutiquement acceptable de celui-ci, 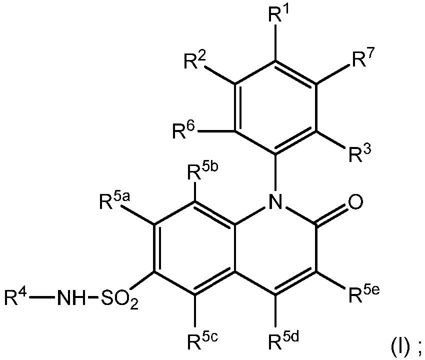 où :
R¹ est un -alk en C₀₋₄-O-alk en C₁₋₈, un -alk en C₀₋₄-S-alk en C₁₋₈, ou un -alk en C₀₋₄-NH(alk en C₁₋₈) ; où ledit alk en C₁₋₈ est substitué par 0, 1, 2, 3, 4, 5, ou 6 halo ;
R² est H, un halo, CN, un alk en C₁₋₆, ou un haloalk en C₁₋₆ ;
R³ est un alk en C₁₋₆, un haloalk en C₁₋₆, un -O-alk en C₁₋₆, un -O-cyclopropyle, ou un - O-cyclobutyle ;
R⁴ est un hétéroaryle de 5 à 6 chaînons ;
chaque R parmi R⁶ et R⁷ est l'hydrogène ; et
chaque R parmi R^{5a} ; R^{5b} ; R^{5c} ; R^{5d} ; et R^{5e} est indépendamment l'hydrogène ou un halo.

2. Le composé selon la revendication 1, ou un énantiomère, diastéréoisomère, atropisomère, ou un mélange de ceux-ci, ou un sel pharmaceutiquement acceptable de celui-ci, où R¹ est un -O-alk en C₁₋₈, un -CH₂-O-alk en C₁₋₈, un -S-alk en C₁₋₈, un -CH₂-S-alk en C₁₋₈, ou un -CH(CH₃)-S-alk en C₁₋₈, où ledit alk en C₁₋₈ est substitué par 1, 2, 3, ou 4 halo.

3. Le composé selon l'une quelconque des revendications 1 à 2, ou un énantiomère, diastéréoisomère, atropisomère, ou un mélange de ceux-ci, ou un sel pharmaceutiquement acceptable de celui-ci, où R¹ est sélectionné parmi -O-CF₃, -O-CH₂-CF₃, -O-CH₂-CH₂-CF₃, -O-CH(CH₃)-CF₃, -CH₂-O-CF₃, -S-CF₃, ou -S-CH₂-CF₃.

4. Le composé selon l'une quelconque des revendications 1 à 3, ou un énantiomère, diastéréoisomère, atropisomère, ou un mélange de ceux-ci, ou un sel pharmaceutiquement acceptable de celui-ci, où R² est H, un fluoro, un chloro, un méthyle, CN, CF₃, CHF₂, ou CH₂F.

5. Le composé selon l'une quelconque des revendications 1 à 4, ou un énantiomère, diastéréoisomère, atropisomère, ou un mélange de ceux-ci, ou un sel pharmaceutiquement acceptable de celui-ci, où R³ est un méthoxy.

6. Le composé selon l'une quelconque des revendications 1 à 5, ou un énantiomère, diastéréoisomère, atropisomère, ou un mélange de ceux-ci, ou un sel pharmaceutiquement acceptable de celui-ci, où R⁴ est un isoxazolyle, un pyridazinyle, un thiazolyle, un thiadiazolyle, un oxazolyle, ou un pyrimidinyle.

7. Le composé selon l'une quelconque des revendications 1 à 6, ou un énantiomère, diastéréoisomère, atropisomère, ou un mélange de ceux-ci, ou un sel pharmaceutiquement acceptable de celui-ci, où :
chaque R parmi R^{5a} ; R^{5b} ; R^{5c} ; R^{5d} ; et R^{5c} est l'hydrogène ;
R^{5a} est F et chaque R parmi R^{5b} ; R^{5c} ; R^{5d} ; et R^{5e} est l'hydrogène ; ou
R^{5c} est F ; et chaque R parmi R^{5a} ; R^{5b} ; R^{5d} ; et R^{5e} est l'hydrogène.

8. Le composé selon la revendication 1, ou un sel pharmaceutiquement acceptable de celui-ci, lequel est sélectionné dans le groupe constitué :
a) du (P)-1-(5-chloro-2-méthoxy-4-((trifluorométhoxy)méthyl)phényl)-N-(isoxazol-3-yl)-2-oxo-1,2-dihydroquinoléine-6-sulfonamide ;
b) du (P)-1-(5-chloro-2-méthoxy-4-((trifluorométhyl)thio)phényl)-N-(isoxazol-3-yl)-2-oxo-1,2-dihydroquinoléine-6-sulfonamide ;
c) du (P)-1-(5-chloro-2-méthoxy-4-(trifluorométhoxy)phényl)-N-(isoxazol-3-yl)-2-oxo-1,2-dihydroquinoléine-6-sulfonamide ;
d) du (P)-1-(5-fluoro-2-méthoxy-4-((trifluorométhoxy)méthyl)phényl)-N-(isoxazol-3-yl)-2-oxo-1,2-dihydroquinoléine-6-sulfonamide ;
e) du (P)-1-(5-fluoro-2-méthoxy-4-((2,2,2-trifluoroéthyl)thio)phényl)-N-(isoxazol-3-yl)-2-oxo-1,2-dihydroquinoléine-6-sulfonamide ;
f) du (P)-7-fluoro-1-(5-fluoro-2-méthoxy-4-((trifluorométhoxy)méthyl)phényl)-N-(isoxazol-3-yl)-2-oxo-1,2-dihydroquinoléine-6-sulfonamide ;
g) du (P)-7-fluoro-1-(5-fluoro-2-méthoxy-4-((trifluorométhyl)thio)phényl)-N-(isoxazol-3-yl)-2-oxo-1,2-dihydroquinoléine-6-sulfonamide ;
h) du (P)-4-fluoro-1-(5-fluoro-2-méthoxy-4-((trifluorométhyl)thio)phényl)-N-(isoxazol-3-yl)-2-oxo-1,2-dihydroquinoléine-6-sulfonamide ; ou
i) du (P)-N-(isoxazol-3-yl)-1-(2-méthoxy-5-méthyl-4-((trifluorométhyl)thio)phényl)-2-oxo-1,2-dihydroquinoléine-6-sulfonamide.

9. Le composé selon la revendication 1, ou un sel pharmaceutiquement acceptable de celui-ci, lequel est sélectionné dans le groupe constitué :
a) du (P)-1-(5-chloro-2-méthoxy-4-((trifluorométhoxy)méthyl)phényl)-N-(isoxazol-3-yl)-2-oxo-1,2-dihydroquinoléine-6-sulfonamide ;
b) du (P)-1-(5-chloro-2-méthoxy-4-(trifluorométhoxy)phényl)-N-(isoxazol-3-yl)-2-oxo-1,2-dihydroquinoléine-6-sulfonamide ;
c) du (P)-1-(5-fluoro-2-méthoxy-4-((trifluorométhoxy)méthyl)phényl)-N-(isoxazol-3-yl)-2-oxo-1,2-dihydroquinoléine-6-sulfonamide ; ou
d) du (P)-7-fluoro-1-(5-fluoro-2-méthoxy-4-((trifluorométhoxy)méthyl)phényl)-N-(isoxazol-3-yl)-2-oxo-1,2-dihydroquinoléine-6-sulfonamide.

10. Le composé selon la revendication 1, ou un sel pharmaceutiquement acceptable de celui-ci, lequel est sélectionné dans le groupe constitué :
a) du (P)-1-(5-chloro-2-méthoxy-4-((trifluorométhyl)thio)phényl)-N-(isoxazol-3-yl)-2-oxo-1,2-dihydroquinoléine-6-sulfonamide ;
b) du (P)-1-(5-fluoro-2-méthoxy-4-((2,2,2-trifluoroéthyl)thio)phényl)-N-(isoxazol-3-yl)-2-oxo-1,2-dihydroquinoléine-6-sulfonamide ;
c) du (P)-7-fluoro-1-(5-fluoro-2-méthoxy-4-((trifluorométhyl)thio)phényl)-N-(isoxazol-3-yl)-2-oxo-1,2-dihydroquinoléine-6-sulfonamide ;
d) du (P)-4-fluoro-1-(5-fluoro-2-méthoxy-4-((trifluorométhyl)thio)phényl)-N-(isoxazol-3-yl)-2-oxo-1,2-dihydroquinoléine-6-sulfonamide ; ou
e) du (P)-N-(isoxazol-3-yl)-1-(2-méthoxy-5-méthyl-4-((trifluorométhyl)thio)phényl)-2-oxo-1,2-dihydroquinoléine-6-sulfonamide.

11. Le composé selon l'une quelconque des revendications 1 à 7, ou un énantiomère, diastéréoisomère, atropisomère, ou un mélange de ceux-ci, ou un sel pharmaceutiquement acceptable de celui-ci, où ledit atropisomère, lorsqu'il est présent, est un atropisomère P.

12. Une composition pharmaceutique comprenant un composé selon l'une quelconque des revendications 1 à 11, ou un sel pharmaceutiquement acceptable de celui-ci, et un excipient pharmaceutiquement acceptable.

13. Le composé selon l'une quelconque des revendications 1 à 11, ou un sel pharmaceutiquement acceptable de celui-ci, ou la composition pharmaceutique selon la revendication 12, pour son utilisation comme médicament.

14. Le composé selon l'une quelconque des revendications 1 à 11, ou un sel pharmaceutiquement acceptable de celui-ci, ou la composition pharmaceutique selon la revendication 12, pour son utilisation dans le traitement de la douleur, de la toux, ou de démangeaisons.

15. Le composé, ou un sel pharmaceutiquement acceptable de celui-ci, ou la composition pharmaceutique pour son utilisation selon la revendication 14, où la douleur est sélectionnée parmi la douleur chronique, la douleur aiguë, la douleur neuropathique, la douleur associée à l'arthrite rhumatoïde, la douleur associée à l'ostéoarthrite, la douleur associée au cancer, la neuropathie diabétique périphérique, et la douleur lombaire neuropathique.

16. Le composé, ou un sel pharmaceutiquement acceptable de celui-ci, ou la composition pharmaceutique pour son utilisation selon la revendication 14, où la toux est sélectionnée parmi la toux post-virale, la toux virale, ou la toux virale aiguë.
